# EUROPEAN PATENT SPECIFICATION

(11) **EP 2 465 846 B1**
(45) Date of publication and mention of the grant of the patent: **09.04.2014**
(21) Application number: 10808259.5
(22) Date of filing: 06.08.2010
(51) Int. Cl.: C07C 319/18, C07C 315/02, C07C 315/04, C07C 317/44, C07C 319/20, C07C 323/52, C07C 323/60

(54) **PROCESS FOR PREPARATION OF (FLUOROALKYLTHIO)ACETIC ACID ESTERS**
VERFAHREN ZUR HERSTELLUNG VON (FLUORALKYLTHIO-)ESSIGSÄUREESTERN
PROCÉDÉ DE FABRICATION D'ESTERS D'ACIDES (FLUOROALKYLTHIO) ACÉTIQUES

(30) Priority: 10.08.2009 JP 2009185696
(43) Date of publication of application: 20.06.2012
(60) Divisional application: 13175777.5
(73) Proprietor: Sumitomo Chemical Company, Limited, Tokyo 104-8260 (JP)
(72) Inventor: HIROTA, Masaji, Ibaraki-shi Osaka 567-0841 (JP); MIYAZAKI, Hiroyuki, Oita-shi, Oita (JP); ITOH, Tadayoshi, Takarazuka-shi Hyogo 665-0051 (JP)
(74) Representative: Winter, Brandl, Fürniss, Hübner, Röss, Kaiser, Polte - Partnerschaft
(86) International application number: PCT/JP2010/063735
(87) International publication number: WO 2011/019076

(56) References cited:
- WO-A1-2007/060839
- JP-A- 49 062 416
- JP-A- 2008 013 538
- JP-A- 2009 001 551
- AMEDURI, B. ET AL.: "Synthesis and polymerization of fluorinated monomers bearing a reactive lateral group. Part 4. Preparation of functional perfluorovinyl monomers by radical addition of functional mercaptans to 1,1,2-trifluoro 1,4-pentadiene", JOURNAL OF FLUORINE CHEMISTRY, vol. 92, no. 1, 1998, pages 77-84, XP002686759,
- AMEDURI, B. ET AL.: 'Synthesis and polymerization of fluorinated monomers bearing a reactive lateral group. Part 4. Preparation of functional perfluorovinyl monomers by radical addition of functional mercaptans to 1,1,2-trifluoro 1,4-pentadiene' JOURNAL OF FLUORINE CHEMISTRY vol. 92, no. 1, 1998, pages 77 - 84
- MCMANUS, JAMES M.: 'Preparation of 1,2,4- benzothiadiazines' JOURNAL OF HETEROCYCLIC CHEMISTRY vol. 5, no. 1, 1968, pages 137 - 139

## Description

### TECHNICAL FIELD

The present invention relates to a process for producing a (fluoroalkylthio)acetic acid ester.

### BACKGROUND ART

JP-A-2009-1551 discloses that a (fluoroalkylthio)acetic acid ester such as methyl (3,3,3-trifluoropropylthio)acetate is an intermediate for the synthesis of an organic sulfur compound, which exerts an excellent control effect against harmful arthropods, and also discloses, as a process for producing methyl (3,3,3-trifluoropropylthio)acetate, a process in which methyl thioglycolate is reacted with 1-iodo-3,3,3-trifluoropropane in the presence of potassium carbonate.

Document WO2007060839 discloses a process for the preparation of (fluoroalkylthio)acetic acid esters involving a different starting material (iodofluoroalkyls instead of fluoroalkenes) and no radical generator.

In JOURNAL OF FLUORINE CHEMISTRY, vol. 92, no. 1, 1998, pages 77-84 a process is disclosed which uses either long-chain fluoroalkenes or trifluorodienes to a thioglycolic acid instead of a thioester and a radical generator. However, the products and the starting materials are different.

### SUMMARY OF THE INVENTION

The present invention is provided as follows.
[1] A process for producing a (fluoroalkylthio)acetic acid ester represented by the formula (3): 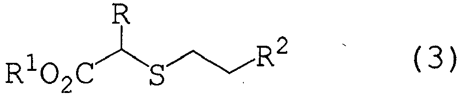
   wherein R, R¹ and R² have the same meanings as defined below, which comprises reacting a thioglycolic acid ester represented by the formula (1): 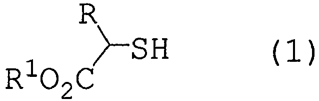
   wherein R represents a hydrogen atom or an alkyl group having 1 to 5 carbon atoms, and R¹ represents an alkyl group having 1 to 4 carbon atoms, with fluoroolefin represented by the formula (2): 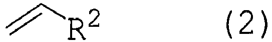
   wherein R² represents a fluoroalkyl group having 1 to 5 carbon atoms, in the presence of a radical generator.
[2] The process according to [1], wherein the radical generator is an azo compound represented by the formula (4): 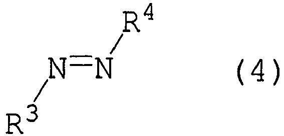
   wherein R³ and R⁴ each independently represents an alkyl group capable of having a substituent.
[3] The process according to [2], wherein the azo compound represented by the formula (4) is 2,2'-azobis(2,4-dimethyl-4-methoxyvaleronitrile).
[4] The process according to any one of [1] to [3], wherein R¹ is a methyl group.
[5] The process according to [1] to [4], wherein R² is a trifluoromethyl group.
[6] The process according to any one of [1] to [5], wherein the reaction is carried out in the presence of ethyl acetate.
[7] A process for producing an ester compound represented by the formula (5): 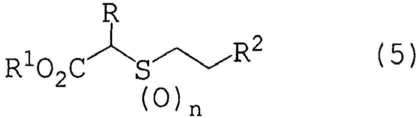
   wherein R, R¹ and R² have the same meanings as defined below, and n represents 1 or 2, which comprises obtaining a (fluoroalkylthio)acetic acid ester represented by the formula (3):

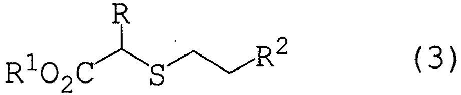

   wherein R represents a hydrogen atom or an alkyl group having 1 to 5 carbon atoms, R¹ represents an alkyl group having 1 to 4 carbon atoms, and R² represents a fluoroalkyl group having 1 to 5 carbon atoms, by the process according to any one of [1] to [6], and then oxidizing the obtained (fluoroalkylthio)acetic acid ester represented by the formula (3).
[8] A process for producing an amide compound represented by the formula (6): 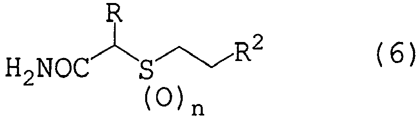
   wherein R, R² and n have the same meanings as defined below, which comprises obtaining an ester compound represented by the formula (5):

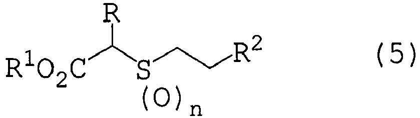

   wherein R represents a hydrogen atom or an alkyl group having 1 to 5 carbon atoms, R¹ represents an alkyl group having 1 to 4 carbon atoms, R² represents a fluoroalkyl group having 1 to 5 carbon atoms, and n represents 1 or 2, by the process according to [7], and then reacting the obtained ester compound represented by the formula (5) with ammonia.
[9] A process for producing a nitrile compound represented by the formula (7): 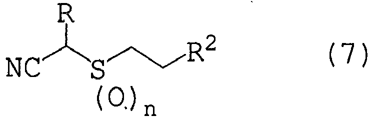
   wherein R, R² and n have the same meanings as defined below, which comprises obtaining an amide compound represented by the formula (6):

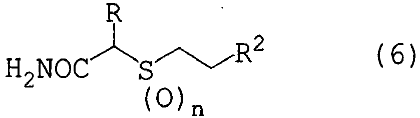

   wherein R represents a hydrogen atom or an alkyl group having 1 to 5 carbon atoms, R² represents a fluoroalkyl group having 1 to 5 carbon atoms, and n represents 1 or 2, by the process according to [8], and then converting a carbamoyl group (-CONH₂) of the obtained amide compound represented by the formula (6) into a cyano group (-CN).
[10] A process for producing a (fluoroalkylthio)acetamide represented by the formula (8): 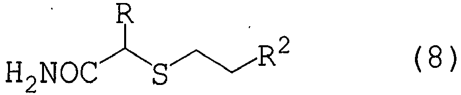
   wherein R and R² have the same meanings as defined below, which comprises obtaining a (fluoroalkylthio)acetic acid ester represented by the formula (3): 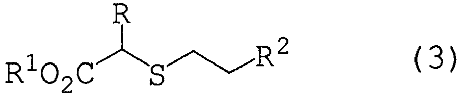
   wherein R represents a hydrogen atom or an alkyl group having 1 to 5 carbon atoms, R¹ represents an alkyl group having 1 to 4 carbon atoms, and R² represents a fluorine-containing alkyl group having 1 to 5 carbon atoms, by the process according to any one of [1] to [6], and then reacting the obtained (fluoroalkylthio)acetic acid ester represented by the formula (3) with ammonia.
[11] A process for producing an amide compound represented by the formula (6): 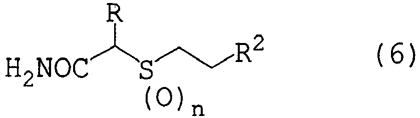
   wherein R and R² have the same meanings as defined below, and n represents 1 or 2, which comprises obtaining a (fluoroalkylthio)acetamide represented by the formula (8): 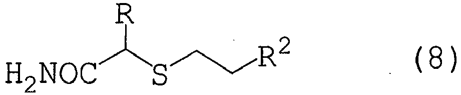
   wherein R represents a hydrogen atom or an alkyl group having 1 to 5 carbon atoms, and R² represents a fluorine-containing alkyl group having 1 to 5 carbon atoms, by the process according to [10], and then oxidizing the obtained (fluoroalkylthio)acetamide represented by the formula (8).
[12] A process for producing a nitrile compound represented by the formula (7) 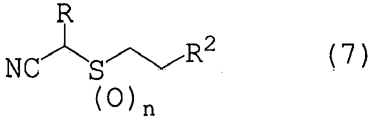
   wherein R, R² and n have the same meanings as defined below, which comprises obtaining an amide compound represented by the formula (6): 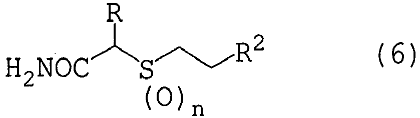
   wherein R represents a hydrogen atom or an alkyl group having 1 to 5 carbon atoms, R² represents a fluorine-containing alkyl group having 1 to 5 carbon atoms, and n represents 1 or 2, by the process according to [11], and then converting a carbamoyl group (-CONH₂) of the obtained amide compound represented by the formula (6) into a cyano group (-CN).
[13] A process for producing a (fluoroalkylthio)acetonitrile represented by the formula (9): 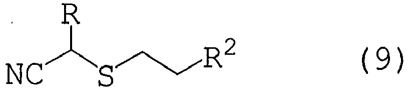
   wherein R and R² have the same meanings as defined below, which comprises obtaining a (fluoroalkylthio)acetamide represented by the formula (8): 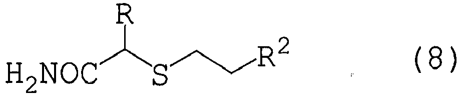
   wherein R represents a hydrogen atom or an alkyl group having 1 to 5 carbon atoms, and R² represents a fluorine-containing alkyl group having 1 to 5 carbon atoms, by the process according to [10], and then converting a carbamoyl group (-CONH₂) of the obtained (fluoroalkylthio)acetamide represented by the formula (8) into a cyano group (-CN).
[14] A process for producing a nitrile compound represented by the formula (7): 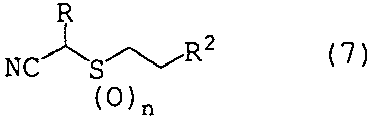
   wherein R and R² have the same meanings as defined below, and n represents 1 or 2), which comprises obtaining an (fluoroalkylthio)acetonitrile represented by the formula (9) : 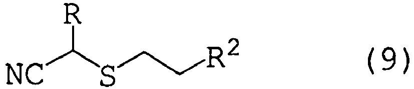
   wherein R represents a hydrogen atom or an alkyl group having 1 to 5 carbon atoms, and R² represents a fluorine-containing alkyl group having 1 to 5 carbon atoms, by the process according [13], and then oxidizing the obtained (fluoroalkylthio)acetonitrile represented by the formula (9).

### BEST MODE FOR CARRYING OUT THE INVENTION

In the formula of a thioglycolic acid ester (hereinafter abbreviated to an ester (1)) represented by the formula (1): 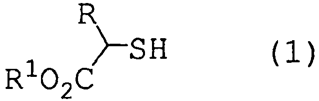
R represents a hydrogen atom or an alkyl group having 1 to 5 carbon atoms, and R¹ represents an alkyl group having 1 to 4 carbon atoms.

Examples of the alkyl group having 1 to 4 carbon atoms represented by R¹ include a methyl group, an ethyl group, a propyl group, an isopropyl group, a butyl group, an isobutyl group and a tert-butyl group, and a methyl group is preferable.

Examples of the alkyl group having 1 to 5 carbon atoms represented by R include a methyl group, an ethyl group, a propyl group, an isopropyl group, a butyl group, an isobutyl group and a pentyl group. R is preferably a hydrogen atom or a methyl group, and more preferably a hydrogen atom.

Examples of the ester (1) include methyl thioglycolate, ethyl thioglycolate, propyl thioglycolate, isopropyl thioglycolate, butyl thioglycolate, isobutyl thioglycolate, tert-butyl thioglycolate, methyl 2-mercaptopropionate, ethyl 2-mercaptopropionate, propyl 2-mercaptopropionate, isopropyl 2-mercaptopropionate, butyl 2-mercaptopropionate, isobutyl 2-mercaptopropionate, tert-butyl 2-mercaptopropionate, methyl 2-mercaptobutyrate, ethyl 2-mercaptobutyrate, propyl 2-mercaptobutyrate, isopropyl 2-mercaptobutyrate, butyl 2-mercaptobutyrate, isobutyl 2-mercaptobutyrate, tert-butyl 2-mercaptobutyrate, methyl 3-methyl-2-mercaptobutyrate, ethyl 3-methyl-2-mercaptobutyrate, propyl 3-methyl-2-mercaptobutyrate, isopropyl 3-methyl-2-mercaptobutyrate, butyl 3-methyl-2-mercaptobutyrate, isobutyl 3-methyl-2-mercaptobutyrate, tert-butyl 3-methyl-2-mercaptobutyrate, methyl 2-mercaptovalerate, ethyl 2-mercaptovalerate, propyl 2-mercaptovalerate, isopropyl 2-mercaptovalerate, butyl 2-mercaptovalerate, isobutyl 2-mercaptovalerate, tert-butyl 2-mercaptovalerate, methyl 4-methyl-2-mercaptovalerate, ethyl 4-methyl-2-mercaptovalerate, propyl 4-methyl-2-mercaptovalerate, isopropyl 4-methyl-2-mercaptovalerate, butyl 4-methyl-2-mercaptovalerate, isobutyl 4-methyl-2-mercaptovalerate, tert-butyl 4-methyl-2-mercaptovalerate, methyl 2-mercaptocaproate, ethyl 2-mercaptocaproate, propyl 2-mercaptocaproate, isopropyl 2-mercaptocaproate, butyl 2-mercaptocaproate, isobutyl 2-mercaptocaproate, tert-butyl 2-mercaptocaproate, methyl 2-mercaptoenanthate, ethyl 2-mercaptoenanthate, propyl 2-mercaptoenanthate, isopropyl 2-mercaptoenanthate, butyl 2-mercaptoenanthate, isobutyl 2-mercaptoenanthate and tert-butyl 2-mercaptoenanthate.

Among these esters, methyl thioglycolate, ethyl thioglycolate, propyl thioglycolate, isopropyl thioglycolate, butyl thioglycolate, isobutyl thioglycolate, tert-butyl thioglycolate, methyl 2-mercaptopropionate, ethyl 2-mercaptopropionate, propyl 2-mercaptopropionate, isopropyl 2-mercaptopropionate, butyl 2-mercaptopro.pionate, isobutyl 2-mercaptopropionate, tert-butyl 2-mercaptopropionate, methyl 2-mercaptobutyrate, ethyl 2-mercaptobutyrate, propyl 2-mercaptobutyrate, isopropyl 2-mercaptobutyrate, butyl 2-mercaptobutyrate, isobutyl 2-mercaptobutyrate and tert-butyl 2-mercaptobutyrate are preferable, and methyl thioglycolate , ethyl thioglycolate, propyl thioglycolate, isopropyl thioglycolate, butyl thioglycolate, isobutyl thioglycolate and tert-butyl thioglycolate are more preferable.

Commercially available esters may be used as the ester (1), and esters produced according to a conventional process may also be used.

In the formula of fluoroolefin (hereinafter abbreviated to an olefin (2)) represented by the formula (2): 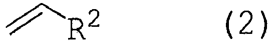
R² represents a fluoroalkyl group having 1 to 5 carbon atoms.

Herein, the "fluoroalkyl group" means a group in which at least one hydrogen atom of an alkyl group is substituted with a fluorine atom. Examples of the fluoroalkyl group having 1 to 5 carbon atoms include fluoromethyl group, a difluoromethyl group, a trifluoromethyl group, a 2-fluoroethyl group, a 2,2,2-trifluoroethyl group, a 1,1,2,2,2-pentafluoroethyl group, a 2-fluoropropyl group, a 2,2-difluoropropyl group, a 3-fluoropropyl group, a 3,3-difluoropropyl group, a 3,3,3-trifluoropropyl group, a 2,2,3,3,3-pentafluoropropyl group, a 2,2,3,3-tetrafluoropropyl group, a 2,2,2-trifluoro-(1-trifluoromethyl)ethyl group, a 2-fluorobutyl group, a 2,2-difluorobutyl group, a 3-fluorobutyl group, a 3,3-difluorobutyl group, a 4-fluorobutyl group, a 4,4-difluorobutyl group, a 4,4,4-trifluorobutyl group, a 3,3,4,4,4-pentafluorobutyl group, a 2,2,3,4,4-pentafluorobutyl group, a 2,2,3,3,4,4,4-heptafluorobutyl group, a 2-fluoropentyl group, a 2,2-difluoropentyl group, a 3-fluoropentyl group, a 3,3-difluoropentyl group, a 4-fluoropentyl group, a 4,4-difluoropentyl group, a 5-fluoropentyl group, a 5,5-difluoropentyl group, a 5,5,5-trifluoropentyl group, a 4,4,5,5,5-pentafluoropentyl group, a 3,3,4,4,5,5,5-heptafluoropentyl group, a 2,2,3,3,4,4,5,5-octafluoropentyl group and a 2,2,3,3,4,4,5,5,5-nonafluoropentyl group.

Examples of the olefin (2) include 3-fluoropropene, 3,3-difluoropropene, 3,3,3-trifluoropropene, 4-fluoro-1-butene, 4,4,4-trifluoro-1-butene, 3,3,4,4,4-pentafluoro-1-butene, 4-fluoro-1-pentene, 4,4-difluoro-1-pentene, 5-fluoro-1-pentene, 5,5-difluoro-1-pentene, 5,5,5-trifluoro-1-pentene, 4,4,5,5,5-pentafluoro-1-pentene, 4,4,5,5-tetrafluoro-1-pentene, 4,4,4-trifluoro-3-trifluoromethyl-1-butene, 5-fluoro-1-hexene, 5,5-difluoro-1-hexene, 4-fluoro-1-hexene, 4,4-fluoro-1-hexene, 6-fluoro-1-hexene, 6,6-difluoro-1-hexene, 6,6,6-trifluoro-1-hexene, 5,5,6,6,6-pentafluoro-1-hexene, 4,4,5,6,6-pentafluoro-1-hexene, 4,4,5,5,6,6,6-heptafluoro-1-hexene, 4-fluoro-1-heptene, 4,4-difluoro-1-heptene, 5-fluoro-1-heptene, 5,5-difluoro-1-heptene, 6-fluoro-1-heptene, 6,6-difluoro-1-heptene, 7-fluoro-1-heptene, 7,7-difluoro-1-heptene, 7,7,7-trifluoro-1-heptene, 6,6,7,7,7-pentafluoro-1-heptene, 5,5,6,6,7,7,7-heptafluoro-1-heptene and 4,4,5,5,6,6,7,7,7-nonafluoro-1-heptene. Among these olefins, 3-fluoropropene, 3,3-difluoropropene and 3,3,3-trifluoropropene are preferable, and 3,3,3-trifluoropropene is more preferable.

Commercially available olefins may be used as the olefin (2), and olefins produced according to a conventional process may also be used.

It is possible to produce a (fluoroalkylthio)acetic acid ester (hereinafter abbreviated to an ester (3)) represented by the formula (3): 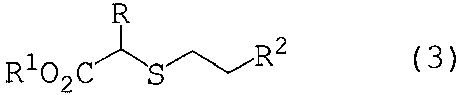
wherein R, R¹ and R² have the same meanings as defined above) by reacting an ester (1) with an olefin (2) in the presence of a radical generator.

The use amount of the olefin (2) usually from 0.8 to 10 mol, and preferably from 1 to 5 mol, based on 1 mol of the ester (1).

Examples of the radical generator include boron-containing compounds such as triethylborane; peroxides such as tert-butyl hydroperoxide and hydrogen peroxide; and azo compounds. Among these radical generators, azo compounds are preferable, and azo compounds represented by the formula (4): 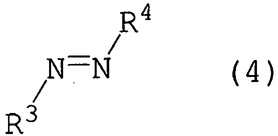
wherein R³ and R⁴ each independently represents an alkyl group capable of having a substituent, are more preferable.

Examples of the alkyl group represented by R³ and R⁴ include alkyl groups having 1 to 12 carbon atoms, such as a methyl group, an ethyl group, a propyl group, an isopropyl group, a butyl group, an isobutyl group, a pentyl group, a hexyl group, a heptyl group, an octyl group, a nonyl group, a decyl group, an undecyl group and an dodecyl group. These alkyl groups can have a substituent and examples of the substituent include a cyano group; an alkoxy group having 1 to 12 carbon atoms, such as a methoxy group, an ethoxy group, a propoxy group, an isopropoxy group, a butoxy group, an isobutoxy group, a pentyloxy group, a hexyloxy group, a heptyloxy group, an octyloxy group, a nonyloxy group, a decyloxy group, an undecyloxy group and a dodecyloxy group; a carbamoyl group, a hydroxyl group, an imidazolyl group and a pyrrolidino group, each being capable of having the above-mentioned alkyl groups having 1 to 12 carbon atoms.

Examples of the azo compound represented by the formula (4) include 2,2'-azobisisobutyronitrile, 2,2'-azobis(2,4-dimethyl-4-methoxyvaleronitrile), 2,2'-azobis[2-(2-imidazolin-2-yl)propane] hydrochloride, 2,2'-azobis[2-(2-imidazolin-2-yl)propane] sulfate, 2,2'-azobis(2-methylpropionamidine) hydrochloride, 2,2'-azobis[N-(2-carboxyethyl)-2-methylpropionamide], 2,2'-azobis{2-[1-(2-hydroxymethyl)-2-imidazolin-2-yl]propane} hydrochloride, 2,2'-azobis[2-(2-imidazolin-2-yl)propane], 2,2'-azobis(1-imino-1-pyrrolidino-2-methylpropane) hydrochloride, 2,2'-azobis{2-methyl-[1,1-bis(hydroxymethyl)-2-hydroxyethyl]propaneamide}, 2,2'-azobis[2-methyl-(2-hydroxyethyl)methylpropionamide], 2,2'-azobis(2,4-dimethylvaleronitrile), 2,2'-azobis(methyl 2-propionate), 2,2'-azobis(2-methylbutyronitrile), 1,1'-azobis(cyclohexane-1-carbonitrile), 2,2'-azobis[N-(2-propenyl)-2-methylpropionamide], 1-[(1-cyano-1-methylethyl)azo]formamide, 2,2'-azobis(butyl-2-methylpropionamide) and 2,2'-azobis(N-cyclohexyl-2-methylpropionamide), and 2,2'-azobis(2,4-dimethyl-4-methoxyvaleronitrile) is preferable.

The use amount of the radical generator is usually from 0.001 to 1 mol, and preferably from 0.01 to 0.1 mol, based on 1 mol of the ester (1).

The reaction of the ester (1) with the olefin (2) is usually carried out by mixing the ester (1), the olefin (2) and the radical generator.

The reaction of the ester (1) with the olefin (2) may be carried out in the presence of a solvent, or may be carried out in the absence of the solvent. Examples of the solvent include water; aliphatic hydrocarbon solvents such as hexane and heptane; aromatic hydrocarbon solvents such as toluene and xylene; chlorinated hydrocarbon solvents such as dichloromethane, dichloroethane and chlorobenzene; ether solvents such as anisole, tetrahydrofuran, tert-butyl methyl ether and cyclopentyl methyl ether; ketone solvents such as acetone and isobutyl methyl ketone; and ester solvents such as methyl acetate and ethyl acetate. These solvents may be used alone, or two or more kinds of them may be used in combination.

The reaction of the ester (1) with the olefin (2) is preferably carried out in the absence of the solvent or in the presence of the ester solvent, and more preferably carried out in the presence of ethyl acetate. The use amount of the solvent is usually from 0 to 100 parts by weight, and preferably from 1 to 10 parts by weight, based on 1 part by weight of the olefin (2).

The reaction of the ester (1) with the olefin (2) may be carried out under normal pressure condition or under pressurization condition. It is possible to carry out the reaction under normal pressure condition even in case of using an olefin (2) having a low boiling point by reacting in the presence of the solvent.

The reaction temperature is usually from -80 to 100°C, preferably from 10 to 40°C. The reaction time is usually from 1 to 100 hours.

It is possible to determine an end point of the reaction, for example, by analyzing the contents of the ester (1) and/or the ester (3) in the reaction mixture through conventional analysis means such as high performance liquid chromatography or gas chromatography.

The ester (3) can be extracted, for example, by concentrating the obtained reaction mixture. The extracted ester (3) may be further purified by conventional purification means such as distillation, crystallization or column chromatography.

Examples of the ester (3) include methyl (3-fluoropropylthio)acetate, ethyl (3-fluoropropylthio)acetate, propyl (3-fluoropropylthio)acetate, isopropyl (3-fluoropropylthio)acetate, butyl (3-fluoropropylthio)acetate, isobutyl (3-fluoropropylthio)acetate, tert-butyl (3-fluoropropylthio)acetate, methyl (3,3-difluoropropylthio)acetate, ethyl (3,3-difluoropropylthio)acetate, propyl (3,3-difluoropropylthio)acetate, isopropyl (3,3-difluoropropylthio)acetate, butyl (3,3-difluoropropylthio)acetate, isobutyl (3,3-difluoropropylthio)acetate, tert-butyl (3,3-difluoropropylthio)acetate, methyl (3,3,3-trifluoropropylthio)acetate, ethyl (3,3,3-trifluoropropylthio)acetate, propyl (3,3,3-trifluoropropylthio)acetate, isopropyl (3,3,3-trifluoropropylthio)acetate, butyl (3,3,3-trifluoropropylthio)acetate, isobutyl (3,3,3-trifluoropropylthio)acetate, tert-butyl (3,3,3-trifluoropropylthio)acetate, methyl (4-fluorobutylthio)acetate, ethyl (4-fluorobutylthio)acetate, propyl (4-fluorobutylthio)acetate, isopropyl (4-fluorobutylthio)acetate, butyl (4-fluorobutylthio)acetate, isobutyl (4-fluorobutylthio)acetate, tert-butyl (4-fluorobutylthio)acetate, methyl (4,4,4-trifluorobutylthio)acetate, ethyl (4,4,4-trifluorobutylthio)acetate, propyl (4,4,4-trifluorobutylthio)acetate, isopropyl (4,4,4-trifluorobutylthio)acetate, butyl (4,4,4-trifluorobutylthio)acetate, isobutyl (4,4,4-trifluorobutylthio)acetate, tert-butyl (4,4,4-trifluorobutylthio)acetate, methyl (3,3,4,4,4-pentafluorobutylthio)acetate, ethyl (3,3,4,4,4-pentafluorobutylthio)acetate, propyl (3,3,4,4,4-pentafluorobutylthio)acetate, isopropyl (3,3,4,4,4-pentafluorobutylthio)acetate, butyl (3,3,4,4,4-pentafluorobutylthio)acetate, isobutyl (3,3,4,4,4-pentafluorobutylthio)acetate, tert-butyl (3,3,4,4,4-pentafluorobutylthio)acetate, methyl (4-fluoropentylthio)acetate, ethyl (4-fluoropentylthio)acetate, propyl (4-fluoropentylthio)acetate, isopropyl (4-fluoropentylthio)acetate, butyl (4-fluoropentylthio)acetate, isobutyl (4-fluoropentylthio)acetate, tert-butyl (4-fluoropentylthio)acetate, methyl (4,4-difluoropentylthio)acetate, ethyl (4,4-difluoropentylthio)acetate, propyl (4,4-difluoropentylthio)acetate, isopropyl (4,4-difluoropentylthio)acetate, butyl (4,4-difluoropentylthio)acetate, isobutyl (4,4-difluoropentylthio)acetate, tert-butyl (4,4-difluoropentylthio)acetate, methyl (5-fluoropentylthio)acetate, ethyl (5-fluoropentylthio)acetate, propyl (5-fluoropentylthio)acetate, isopropyl (5-fluoropentylthio)acetate, butyl (5-fluoropentylthio)acetate, isobutyl (5-fluoropentylthio)acetate, tert-butyl (5-fluoropentylthio)acetate, methyl (5,5-difluoropentylthio)acetate, ethyl (5,5-difluoropentylthio)acetate, propyl (5,5-difluoropentylthio)acetate, isopropyl (5,5-difluoropentylthio)acetate, butyl (5,5-difluoropentylthio)acetate, isobutyl (5,5-difluoropentylthio)acetate, tert-butyl (5,5-difluoropentylthio)acetate, methyl (5,5,5-trifluoropentylthio)acetate, ethyl (5,5,5-trifluoropentylthio)acetate, propyl (5,5,5-trifluoropentylthio)acetate, isopropyl (5,5,5-trifluoropentylthio)acetate, butyl (5,5,5-trifluoropentylthio)acetate, isobutyl (5,5,5-trifluoropentylthio)acetate, tert-butyl (5,5,5-trifluoropentylthio)acetate, methyl (4,4,5,5-tetrafluoropentylthio)acetate, ethyl (4,4,5,5-tetrafluoropentylthio)acetate, propyl (4,4,5,5-tetrafluoropentylthio)acetate, isopropyl (4,4,5,5-tetrafluoropentylthio)acetate, butyl (4,4,5,5-tetrafluoropentylthio)acetate, isobutyl (4,4,5,5-tetrafluoropentylthio)acetate, tert-butyl (4,4,5,5-tetrafluoropentylthio)acetate, methyl (4,4,5,5,5-pentafluoropentylthio)acetate, ethyl (4,4,5,5,5-pentafluoropentylthio)acetate, propyl (4,4,5,5,5-pentafluoropentylthio)acetate, isopropyl (4,4,5,5,5-pentafluoropentylthio)acetate, butyl (4,4,5,5,5-pentafluoropentylthio)acetate, isobutyl (4,4,5,5,5-pentafluoropentylthio)acetate, tert-butyl (4,4,5,5,5-pentafluoropentylthio)acetate, methyl (4,4,4-trifluoro-3-trifluoromethylthio)acetate, ethyl (4,4,4-trifluoro-3-trifluoromethylthio)acetate, propyl (4,4,4-trifluoro-3-trifluoromethylthio)acetate, isopropyl (4,4,4-trifluoro-3-trifluoromethylthio)acetate, butyl (4,4,4-trifluoro-3-trifluoromethylthio)acetate, isobutyl (4,4,4-trifluoro-3-trifluoromethylthio)acetate, tert-butyl (4,4,4-trifluoro-3-trifluoromethylthio)acetate, methyl (4-fluorohexylthio)acetate, ethyl (4-fluorohexylthio)acetate, propyl (4-fluorohexylthio)acetate, isopropyl (4-fluorohexylthio)acetate, butyl (4-fluorohexylthio)acetate, isobutyl (4-fluorohexylthio)acetate, tert-butyl (4-fluorohexylthio)acetate, methyl (4,4-difluorohexylthio)acetate, ethyl (4,4-difluorohexylthio)acetate, propyl (4,4-difluorohexylthio)acetate, isopropyl (4,4-difluorohexylthio)acetate, butyl (4,4-difluorohexylthio)acetate, isobutyl (4,4-difluorohexylthio)acetate, tert-butyl (4,4-difluorohexylthio)acetate, methyl (5-fluorohexylthio)acetate, ethyl (5-fluorohexylthio)acetate, propyl (5-fluorohexylthio)acetate, isopropyl (5-fluorohexylthio)acetate, butyl (5-fluorohexylthio)acetate, isobutyl (5-fluorohexylthio)acetate, tert-butyl (5-fluorohexylthio)acetate, methyl (5,5-difluorohexylthio)acetate, ethyl (5,5-difluorohexylthio)acetate, propyl (5,5-difluorohexylthio)acetate, isopropyl (5,5-difluorohexylthio)acetate, butyl (5,5-difluorohexylthio)acetate, isobutyl (5,5-difluorohexylthio)acetate, tert-butyl (5,5-difluorohexylthio)acetate, methyl (6-fluorohexylthio)acetate, ethyl (6-fluorohexylthio)acetate, propyl (6-fluorohexylthio)acetate, isopropyl (6-fluorohexylthio)acetate, butyl (6-fluorohexylthio)acetate, isobutyl (6-fluorohexylthio)acetate, tert-butyl (6-fluorohexylthio)acetate, methyl (6,6-difluorohexylthio)acetate, ethyl (6,6-difluorohexylthio)acetate, propyl (6,6-difluorohexylthio)acetate, isopropyl (6,6-difluorohexylthio)acetate, butyl (6,6-difluorohexylthio)acetate, isobutyl (6,6-difluorohexylthio)acetate, tert-butyl (6,6-difluorohexylthio)acetate, methyl (6,6,6-trifluorohexylthio)acetate, ethyl (6,6,6-trifluorohexylthio)acetate, propyl (6,6,6-trifluorohexylthio)acetate, isopropyl (6,6,6-trifluorohexylthio)acetate, butyl (6,6,6-trifluorohexylthio)acetate, isobutyl (6,6,6-trifluorohexylthio)acetate, tert-butyl (6,6,6-trifluorohexylthio)acetate, methyl (5,5,6,6,6-pentafluorohexylthio)acetate, ethyl (5,5,6,6,6-pentafluorohexylthio)acetate, propyl (5,5,6,6,6-pentafluorohexylthio) acetate, isopropyl (5,5,6,6,6-pentafluorohexylthio) acetate, butyl (5,5,6,6,6-pentafluorohexylthio)acetate, isobutyl (5,5,6,6,6-pentafluorohexylthio)acetate, tert-butyl (5,5,6,6,6-pentafluorohexylthio)acetate, methyl (4,4,5,6,6-pentafluorohexylthio)acetate, ethyl (4,4,5,6,6-pentafluorohexylthio)acetate, propyl (4,4,5,6,6-pentafluorohexylthio)acetate, isopropyl (4,4,5,6,6-pentafluorohexylthio)acetate, butyl (4,4,5,6,6-pentafluorohexylthio)acetate, isobutyl (4,4,5,6,6-pentafluorohexylthio)acetate, tert-butyl (4,4,5,6,6-pentafluorohexylthio)acetate, methyl (4,4,5,5,6,6,6-heptafluorohexylthio)acetate, ethyl (4,4,5,5,6,6,6-heptafluorohexylthio)acetate, propyl (4,4,5,5,6,6,6-heptafluorohexylthio)acetate, isopropyl (4,4,5,5,6,6,6-heptafluorohexylthio)acetate, butyl (4,4,5,5,6,6,6-heptafluorohexylthio)acetate, isobutyl (4,4,5,5,6,6,6-heptafluorohexylthio)acetate, tert-butyl (4,4,5,5,6,6,6-heptafluorohexylthio)acetate, methyl (4-fluoroheptylthio)acetate, ethyl (4-fluoroheptylthio)acetate, propyl (4-fluoroheptylthio)acetate, isopropyl (4-fluoroheptylthio)acetate, butyl (4-fluoroheptylthio)acetate, isobutyl (4-fluoroheptylthio)acetate, tert-butyl (4-fluoroheptylthio)acetate, methyl (4,4-difluoroheptylthio)acetate, ethyl (4,4-difluoroheptylthio)acetate, propyl (4,4-difluoroheptylthio)acetate, isopropyl (4,4-difluoroheptylthio)acetate, butyl (4,4-difluoroheptylthio)acetate, isobutyl (4,4-difluoroheptylthio)acetate, tert-butyl (4,4-difluoroheptylthio)acetate, methyl (5-fluoroheptylthio)acetate, ethyl (5-fluoroheptylthio)acetate, propyl (5-fluoroheptylthio)acetate, isopropyl (5-fluoroheptylthio)acetate, butyl (5-fluoroheptylthio)acetate, isobutyl (5-fluoroheptylthio)acetate, tert-butyl (5-fluoroheptylthio)acetate, methyl (5,5-difluoroheptylthio)acetate, ethyl (5,5-difluoroheptylthio)acetate, propyl (5,5-difluoroheptylthio)acetate, isopropyl (5,5-difluoroheptylthio)acetate, butyl (5,5-difluoroheptylthio)acetate, isobutyl (5,5-difluoroheptylthio)acetate, tert-butyl (5,5-difluoroheptylthio)acetate, methyl (6-fluoroheptylthio)acetate, ethyl (6-fluoroheptylthio)acetate, propyl (6-fluoroheptylthio)acetate, isopropyl (6-fluoroheptylthio)acetate, butyl (6-fluoroheptylthio)acetate, isobutyl (6-fluoroheptylthio)acetate, tert-butyl (6-fluoroheptylthio)acetate, methyl (6,6-difluoroheptylthio)acetate, ethyl (6,6-difluoroheptylthio)acetate, propyl (6,6-difluoroheptylthio)acetate, isopropyl (6,6-difluoroheptylthio)acetate, butyl (6,6-difluoroheptylthio)acetate, isobutyl (6,6-difluoroheptylthio)acetate, tert-butyl (6,6-difluoroheptylthio)acetate, methyl (7-fluoroheptylthio)acetate, ethyl (7-fluoroheptylthio)acetate, propyl (7-fluoroheptylthio)acetate, isopropyl (7-fluoroheptylthio)acetate, butyl (7-fluoroheptylthio)acetate, isobutyl (7-fluoroheptylthio)acetate, tert-butyl (7-fluoroheptylthio)acetate, methyl (7,7-difluoroheptylthio)acetate, ethyl (7,7-difluoroheptylthio)acetate, propyl (7,7-difluoroheptylthio)acetate, isopropyl (7,7-difluoroheptylthio)acetate, butyl (7,7-difluoroheptylthio)acetate, isobutyl (7,7-difluoroheptylthio)acetate, tert-butyl (7,7-difluoroheptylthio)acetate, methyl (7,7,7-trifluoroheptylthio)acetate, ethyl (7,7,7-trifluoroheptylthio)acetate, propyl (7,7,7-trifluoroheptylthio)acetate, isopropyl (7,7,7-trifluoroheptylthio)acetate, butyl (7,7,7-trifluoroheptylthio)acetate, isobutyl (7,7,7-trifluoroheptylthio)acetate, tert-butyl (7,7,7-trifluoroheptylthio)acetate, methyl (6,6,7,7,7-pentafluoroheptylthio)acetate, ethyl (6,6,7,7,7-pentafluoroheptylthio)acetate, propyl (6,6,7,7,7-pentafluoroheptylthio)acetate, isopropyl (6,6,7,7,7-pentafluoroheptylthio)acetate, butyl (6,6,7,7,7-pentafluoroheptylthio)acetate, isobutyl (6,6,7,7,7-pentafluoroheptylthio)acetate, tert-butyl (6,6,7,7,7-pentafluoroheptylthio)acetate, methyl (5,5,6,6,7,7,7-heptafluoroheptylthio)acetate, ethyl (5,5,6,6,7,7,7-heptafluoroheptylthio)acetate, propyl (5,5,6,6,7,7,7-heptafluoroheptylthio)acetate, isopropyl (5,5,6,6,7,7,7-heptafluoroheptylthio)acetate, butyl (5,5,6,6,7,7,7-heptafluoroheptylthio)acetate, isobutyl (5,5,6,6,7,7,7-heptafluoroheptylthio)acetate, tert-butyl (5,5,6,6,7,7,7-heptafluoroheptylthio)acetate, methyl (4,4,5,5,6,6,7,7,7-nonafluoroheptylthio)acetate, ethyl (4,4,5,5,6,6,7,7,7-nonafluoroheptylthio)acetate, propyl (4,4,5,5,6,6,7,7,7-nonafluoroheptylthio)acetate, isopropyl (4,4,5,5,6,6,7,7,7-nonafluoroheptylthio)acetate, butyl (4,4,5,5,6,6,7,7,7-nonafluoroheptylthio)acetate, isobutyl (4,4,5,5,6,6,7,7,7-nonafluoroheptylthio)acetate, tert-butyl (4,4,5,5,6,6,7,7,7-nonafluoroheptylthio)acetate, methyl 2-(3-fluoropropylthio)propionate, ethyl 2-(3-fluoropropylthio)propionate, propyl 2-(3-fluoropropylthio)propionate, isopropyl 2-(3-fluoropropylthio)propionate, butyl 2-(3-fluoropropylthio)propionate, isobutyl 2-(3-fluoropropylthio)propionate, tert-butyl 2-(3-fluoropropylthio)propionate, methyl 2-(3,3-difluoropropylthio)propionate, ethyl 2-(3,3-difluoropropylthio)propionate, propyl 2-(3,3-difluoropropylthio)propionate, isopropyl 2-(3,3-difluoropropylthio)propionate, butyl 2-(3,3-difluoropropylthio)propionate, isobutyl 2-(3,3-difluoropropylthio)propionate, tert-butyl 2-(3,3-difluoropropylthio)propionate, methyl 2-(3,3,3-trifluoropropylthio)propionate, ethyl 2-(3,3,3-trifluoropropylthio)propionate, propyl 2-(3,3,3-trifluoropropylthio)propionate, isopropyl 2-(3,3,3-trifluoropropylthio)propionate, butyl 2-(3,3,3-trifluoropropylthio)propionate, isobutyl 2-(3,3,3-trifluoropropylthio)propionate, tert-butyl 2-(3,3,3-trifluoropropylthio)propionate, methyl 2-(4-fluorobutylthio)propionate, ethyl 2-(4-fluorobutylthio)propionate, propyl 2-(4-fluorobutylthio)propionate, isopropyl 2-(4-fluorobutylthio)propionate, butyl 2-(4-fluorobutylthio)propionate, isobutyl 2-(4-fluorobutylthio)propionate, tert-butyl 2-(4-fluorobutylthio)propionate, methyl 2-(4,4,4-trifluorobutylthio)propionate, ethyl 2-(4,4,4-trifluorobutylthio)propionate, propyl 2-(4,4,4-trifluorobutylthio)propionate, isopropyl 2-(4,4,4-trifluorobutylthio)propionate, butyl 2-(4,4,4-trifluorobutylthio)propionate, isobutyl 2-(4,4,4-trifluorobutylthio)propionate, tert-butyl 2-(4,4,4-trifluorobutylthio)propionate, methyl 2-(3,3,4,4,4-pentafluorobutylthio)propionate, ethyl 2-(3,3,4,4,4-pentafluorobutylthio)propionate, propyl 2-(3,3,4,4,4-pentafluorobutylthio)propionate, isopropyl 2-(3,3,4,4,4-pentafluorobutylthio)propionate, butyl 2-(3,3,4,4,4-pentafluorobutylthio)propionate, isobutyl 2-(3,3,4,4,4-pentafluorobutylthio)propionate, tert-butyl 2-(3,3,4,4,4-pentafluorobutylthio)propionate, methyl 2-(4-fluoropentylthio)propionate, ethyl 2-(4-fluoropentylthio)propionate, propyl 2-(4-fluoropentylthio)propionate, isopropyl 2-(4-fluoropentylthio)propionate, butyl 2-(4-fluoropentylthio)propionate, isobutyl 2-(4-fluoropentylthio)propionate, tert-butyl 2-(4-fluoropentylthio)propionate, methyl 2-(4,4-difluoropentylthio)propionate, ethyl 2-(4,4-difluoropentylthio)propionate, propyl 2-(4,4-difluoropentylthio)propionate, isopropyl 2-(4,4-difluoropentylthio)propionate, butyl 2-(4,4-difluoropentylthio)propionate, isobutyl 2-(4,4-difluoropentylthio)propionate, tert-butyl 2-(4,4-difluoropentylthio)propionate, methyl 2-(5-fluoropentylthio)propionate, ethyl 2-(5-fluoropentylthio)propionate, propyl 2-(5-fluoropentylthio)propionate, isopropyl 2-(5-fluoropentylthio)propionate, butyl 2-(5-fluoropentylthio)propionate, isobutyl 2-(5-fluoropentylthio)propionate, tert-butyl 2-(5-fluoropentylthio)propionate, methyl 2-(5,5-difluoropentylthio)propionate, ethyl 2-(5,5-difluoropentylthio)propionate, propyl 2-(5,5-difluoropentylthio)propionate, isopropyl 2-(5,5-difluoropentylthio)propionate, butyl 2-(5,5-difluoropentylthio)propionate, isobutyl 2-(5,5-difluoropentylthio)propionate, tert-butyl 2-(5,5-difluoropentylthio)propionate, methyl 2-(5,5,5-trifluoropentylthio)propionate, ethyl 2-(5,5,5-trifluoropentylthio)propionate, propyl 2-(5,5,5-trifluoropentylthio)propionate, isopropyl 2-(5,5,5-trifluoropentylthio)propionate, butyl 2-(5,5,5-trifluoropentylthio)propionate, isobutyl 2-(5,5,5-trifluoropentylthio)propionate, tert-butyl 2-(5,5,5-trifluoropentylthio)propionate, methyl 2-(4,4,5,5-tetrafluoropentylthio)propionate, ethyl 2-(4,4,5,5-tetrafluoropentylthio)propionate, propyl 2-(4,4,5,5-tetrafluoropentylthio)propionate, isopropyl 2-(4,4,5,5-tetrafluoropentylthio)propionate, butyl 2-(4,4,5,5-tetrafluoropentylthio)propionate, isobutyl 2-(4,4,5,5-tetrafluoropentylthio)propionate, tert-butyl 2-(4,4,5,5-tetrafluoropentylthio)propionate, methyl 2-(4,4,5,5,5-pentafluoropentylthio)propionate, ethyl 2-(4,4,5,5,5-pentafluoropentylthio)propionate, propyl 2-(4,4,5,5,5-pentafluoropentylthio)propionate, isopropyl 2-(4,4,5,5,5-pentafluoropentylthio)propionate, butyl 2-(4,4,5,5,5-pentafluoropentylthio)propionate, isobutyl 2-(4,4,5,5,5-pentafluoropentylthio)propionate, tert-butyl 2-(4,4,5,5,5-pentafluoropentylthio)propionate, methyl 2-(4,4,4-trifluoro-3-trifluoromethylthio)propionate, ethyl 2-(4,4,4-trifluoro-3-trifluoromethylthio)propionate, propyl 2-(4,4,4-trifluoro-3-trifluoromethylthio)propionate, isopropyl 2-(4,4,4-trifluoro-3-trifluoromethylthio)propionate, butyl 2-(4,4,4-trifluoro-3-trifluoromethylthio)propionate, isobutyl 2-(4,4,4-trifluoro-3-trifluoromethylthio)propionate, tert-butyl 2-(4,4,4-trifluoro-3-trifluoromethylthio)propionate, methyl 2-(4-fluorohexylthio)propionate, ethyl 2-(4-fluorohexylthio)propionate, propyl 2-(4-fluorohexylthio)propionate, isopropyl 2-(4-fluorohexylthio)propionate, butyl 2-(4-fluorohexylthio)propionate, isobutyl 2-(4-fluorohexylthio)propionate, tert-butyl 2-(4-fluorohexylthio)propionate, methyl 2-(4,4-difluorohexylthio)propionate, ethyl 2-(4,4-difluorohexylthio)propionate, propyl 2-(4,4-difluorohexylthio)propionate, isopropyl 2-(4,4-difluorohexylthio)propionate, butyl 2-(4,4-difluorohexylthio)propionate, isobutyl 2-(4,4-difluorohexylthio)propionate, tert-butyl 2-(4,4-difluorohexylthio)propionate, methyl 2-(5-fluorohexylthio)propionate, ethyl 2-(5-fluorohexylthio)propionate, propyl 2-(5-fluorohexylthio)propionate, isopropyl 2-(5-fluorohexylthio)propionate, butyl 2-(5-fluorohexylthio)propionate, isobutyl 2-(5-fluorohexylthio)propionate, tert-butyl 2-(5-fluorohexylthio)propionate, methyl 2-(5,5-difluorohexylthio)propionate, ethyl 2-(5,5-difluorohexylthio)propionate, propyl 2-(5,5-difluorohexylthio)propionate, isopropyl 2-(5,5-difluorohexylthio)propionate, butyl 2-(5,5-difluorohexylthio)propionate, isobutyl 2-(5,5-difluorohexylthio)propionate, tert-butyl 2-(5,5-difluorohexylthio)propionate, methyl 2-(6-fluorohexylthio)propionate, ethyl 2-(6-fluorohexylthio)propionate, propyl 2-(6-fluorohexylthio)propionate, isopropyl 2-(6-fluorohexylthio)propionate, butyl 2-(6-fluorohexylthio)propionate, isobutyl 2-(6-fluorohexylthio)propionate, tert-butyl 2-(6-fluorohexylthio)propionate, methyl 2-(6,6-difluorohexylthio)propionate, ethyl 2-(6,6-difluorohexylthio)propionate, propyl 2-(6,6-difluorohexylthio)propionate, isopropyl 2-(6,6-difluorohexylthio)propionate, butyl 2-(6,6-difluorohexylthio)propionate, isobutyl 2-(6,6-difluorohexylthio)propionate, tert-butyl 2-(6,6-difluorohexylthio)propionate, methyl 2-(6,6,6-trifluorohexylthio)propionate, ethyl 2-(6,6,6-trifluorohexylthio)propionate, propyl 2-(6,6,6-trifluorohexylthio)propionate, isopropyl 2-(6,6,6-trifluorohexylthio)propionate, butyl 2-(6,6,6-trifluorohexylthio)propionate, isobutyl 2-(6,6,6-trifluorohexylthio)propionate, tert-butyl 2-(6,6,6-trifluorohexylthio)propionate, methyl 2-(5,5,6,6,6-pentafluorohexylthio)propionate, ethyl 2-(5,5,6,6,6-pentafluorohexylthio)propionate, propyl 2-(5,5,6,6,6-pentafluorohexylthio)propionate, isopropyl 2-(5,5,6,6,6-pentafluorohexylthio)propionate, butyl 2-(5,5,6,6,6-pentafluorohexylthio)propionate, isobutyl 2-(5,5,6,6,6-pentafluorohexylthio)propionate, tert-butyl 2-(5,5,6,6,6-pentafluorohexylthio)propionate, methyl 2-(4,4,5,6,6-pentafluorohexylthio)propionate, ethyl 2-(4,4,5,6,6-pentafluorohexylthio)propionate, propyl 2-(4,4,5,6,6-pentafluorohexylthio)propionate, isopropyl 2-(4,4,5,6,6-pentafluorohexylthio)propionate, butyl 2-(4,4,5,6,6-pentafluorohexylthio)propionate, isobutyl 2-(4,4,5,6,6-pentafluorohexylthio)propionate, tert-butyl 2-(4,4,5,6,6-pentafluorohexylthio)propionate, methyl 2-(4,4,5,5,6,6,6-heptafluorohexylthio)propionate, ethyl 2-(4,4,5,5,6,6,6-heptafluorohexylthio)propionate, propyl 2-(4,4,5,5,6,6,6-heptafluorohexylthio)propionate, isopropyl 2-(4,4,5,5,6,6,6-heptafluorohexylthio)propionate, butyl 2-(4,4,5,5,6,6,6-heptafluorohexylthio)propionate, isobutyl 2-(4,4,5,5,6,6,6-heptafluorohexylthio)propionate, tert-butyl 2-(4,4,5,5,6,6,6-heptafluorohexylthio)propionate, methyl 2-(4-fluoroheptylthio)propionate, ethyl 2-(4-fluoroheptylthio)propionate, propyl 2-(4-fluoroheptylthio)propionate, isopropyl 2-(4-fluoroheptylthio)propionate, butyl 2-(4-fluoroheptylthio)propionate, isobutyl 2-(4-fluoroheptylthio)propionate, tert-butyl 2-(4-fluoroheptylthio)propionate, methyl 2-(4,4-difluoroheptylthio)propionate, ethyl 2-(4,4-difluoroheptylthio)propionate, propyl 2-(4,4-difluoroheptylthio)propionate, isopropyl 2-(4,4-difluoroheptylthio)propionate, butyl 2-(4,4-difluoroheptylthio)propionate, isobutyl 2-(4,4-difluoroheptylthio)propionate, tert-butyl 2-(4,4-difluoroheptylthio)propionate, methyl 2-(5-fluoroheptylthio)propionate, ethyl 2-(5-fluoroheptylthio)propionate, propyl 2-(5-fluoroheptylthio)propionate, isopropyl 2-(5-fluoroheptylthio)propionate, butyl 2-(5-fluoroheptylthio)propionate, isobutyl 2-(5-fluoroheptylthio)propionate, tert-butyl 2-(5-fluoroheptylthio)propionate, methyl 2-(5,5-difluoroheptylthio)propionate, ethyl 2-(5,5-difluoroheptylthio)propionate, propyl 2-(5,5-difluoroheptylthio)propionate, isopropyl 2-(5,5-difluoroheptylthio)propionate, butyl 2-(5,5-difluoroheptylthio)propionate, isobutyl 2-(5,5-difluoroheptylthio)propionate, tert-butyl 2-(5,5-difluoroheptylthio)propionate, methyl 2-(6-fluoroheptylthio)propionate, ethyl 2-(6-fluoroheptylthio)propionate, propyl 2-(6-fluoroheptylthio)propionate, isopropyl 2-(6-fluoroheptylthio)propionate, butyl 2-(6-fluoroheptylthio)propionate, isobutyl 2-(6-fluoroheptylthio)propionate, tert-butyl 2-(6-fluoroheptylthio)propionate, methyl 2-(6,6-difluoroheptylthio)propionate, ethyl 2-(6,6-difluoroheptylthio)propionate, propyl 2-(6,6-difluoroheptylthio)propionate, isopropyl 2-(6,6-difluoroheptylthio)propionate, butyl 2-(6,6-difluoroheptylthio)propionate, isobutyl 2-(6,6-difluoroheptylthio)propionate, tert-butyl 2-(6,6-difluoroheptylthio)propionate, methyl 2-(7-fluoroheptylthio)propionate, ethyl 2-(7-fluoroheptylthio)propionate, propyl 2-(7-fluoroheptylthio)propionate, isopropyl 2-(7-fluoroheptylthio)propionate, butyl 2-(7-fluoroheptylthio)propionate, isobutyl 2-(7-fluoroheptylthio)propionate, tert-butyl 2-(7-fluoroheptylthio)propionate, methyl 2-(7,7-difluoroheptylthio)propionate, ethyl 2-(7,7-difluoroheptylthio)propionate, propyl 2-(7,7-difluoroheptylthio)propionate, isopropyl 2-(7,7-difluoroheptylthio)propionate, butyl 2-(7,7-difluoroheptylthio)propionate, isobutyl 2-(7,7-difluoroheptylthio)propionate, tert-butyl 2-(7,7-difluoroheptylthio)propionate, methyl 2-(7,7,7-trifluoroheptylthio)propionate, ethyl 2-(7,7,7-trifluoroheptylthio)propionate, propyl 2-(7,7,7-trifluoroheptylthio)propionate, isopropyl 2-(7,7,7-trifluoroheptylthio)propionate, butyl 2-(7,7,7-trifluoroheptylthio)propionate, isobutyl 2-(7,7,7-trifluoroheptylthio)propionate, tert-butyl 2-(7,7,7-trifluoroheptylthio)propionate, methyl 2-(6,6,7,7,7-pentafluoroheptylthio)propionate, ethyl 2-(6,6,7,7,7-pentafluoroheptylthio)propionate, propyl 2-(6,6,7,7,7-pentafluoroheptylthio)propionate, isopropyl 2-(6,6,7,7,7-pentafluoroheptylthio)propionate, butyl 2-(6,6,7,7,7-pentafluoroheptylthio)propionate, isobutyl 2-(6,6,7,7,7-pentafluoroheptylthio)propionate, tert-butyl 2-(6,6,7,7,7-pentafluoroheptylthio)propionate,' methyl 2-(5,5,6,6,7,7,7-heptafluoroheptylthio)propionate, ethyl 2-(5,5,6,6,7,7,7-heptafluoroheptylthio)propionate, propyl 2-(5,5,6,6,7,7,7-heptafluoroheptylthio)propionate, isopropyl 2-(5,5,6,6,7,7,7-heptafluoroheptylthio)propionate, butyl 2-(5,5,6,6,7,7,7-heptafluoroheptylthio)propionate, isobutyl 2-(5,5,6,6,7,7,7-heptafluoroheptylthio)propionate, tert-butyl 2-(5,5,6,6,7,7,7-heptafluoroheptylthio)propionate, methyl 2-(4,4,5,5,6,6,7,7,7-nonafluoroheptylthio)propionate, ethyl 2-(4,4,5,5,6,6,7,7,7-nonafluoroheptylthio)propionate, propyl 2-(4,4,5,5,6,6,7,7,7-nonafluoroheptylthio)propionate, isopropyl 2-(4,4,5,5,6,6,7,7,7-nonafluoroheptylthio)propionate, butyl 2-(4,4,5,5,6,6,7,7,7-nonafluoroheptylthio)propionate, isobutyl 2-(4,4,5,5,6,6,7,7,7-nonafluoroheptylthio)propionate, tert-butyl 2-(4,4,5,5,6,6,7,7,7-nonafluoroheptylthio)propionate, methyl 3-methyl-2-(3-fluoropropylthio)butyrate, ethyl 3-methyl-2-(3-fluoropropylthio)butyrate, propyl 3-methyl-2-(3-fluoropropylthio)butyrate, isopropyl 3-methyl-2-(3-fluoropropylthio)butyrate, butyl 3-methyl-2-(3-fluoropropylthio)butyrate, isobutyl 3-methyl-2-(3-fluoropropylthio)butyrate, tert-butyl 3-methyl-2-(3-fluoropropylthio)butyrate, methyl 3-methyl-2-(3,3-difluoropropylthio)butyrate, ethyl 3-methyl-2-(3,3-difluoropropylthio)butyrate, propyl 3-methyl-2-(3,3-difluoropropylthio)butyrate, isopropyl 3-methyl-2-(3,3-difluoropropylthio)butyrate, butyl 3-methyl-2-(3,3-difluoropropylthio)butyrate, isobutyl 3-methyl-2-(3,3-difluoropropylthio)butyrate, tert-butyl 3-methyl-2-(3,3-difluoropropylthio)butyrate, methyl 3-methyl-2-(3,3,3-trifluoropropylthio)butyrate, ethyl 3-methyl-2-(3,3,3-trifluoropropylthio)butyrate, propyl 3-methyl-2-(3,3,3-trifluoropropylthio)butyrate, isopropyl 3-methyl-2-(3,3,3-trifluoropropylthio)butyrate, butyl 3-methyl-2-(3,3,3-trifluoropropylthio)butyrate, isobutyl 3-methyl-2-(3,3,3-trifluoropropylthio)butyrate, tert-butyl 3-methyl-2-(3,3,3-trifluoropropylthio)butyrate, methyl 3-methyl-2-(4-fluorobutylthio)butyrate, ethyl 3-methyl-2-(4-fluorobutylthio)butyrate, propyl 3-methyl-2-(4-fluorobutylthio)butyrate, isopropyl 3-methyl-2-(4-fluorobutylthio)butyrate, butyl 3-methyl-2-(4-fluorobutylthio)butyrate, isobutyl 3-methyl-2-(4-fluorobutylthio)butyrate, tert-butyl 3-methyl-2-(4-fluorobutylthio)butyrate, methyl 3-methyl-2-(4,4,4-trifluorobutylthio)butyrate, ethyl 3-methyl-2-(4,4,4-trifluorobutylthio)butyrate, propyl 3-methyl-2-(4,4,4-trifluorobutylthio)butyrate, isopropyl 3-methyl-2-(4,4,4-trifluorobutylthio)butyrate, butyl 3-methyl-2-(4,4,4-trifluorobutylthio)butyrate, isobutyl 3-methyl-2-(4,4,4-trifluorobutylthio)butyrate, tert-butyl 3-methyl-2-(4,4,4-trifluorobutylthio)butyrate, methyl 3-methyl-2-(3,3,4,4,4-pentafluorobutylthio)butyrate, ethyl 3-methyl-2-(3,3,4,4,4-pentafluorobutylthio)butyrate, propyl 3-methyl-2-(3,3,4,4,4-pentafluorobutylthio)butyrate, isopropyl 3-methyl-2-(3,3,4,4,4-pentafluorobutylthio)butyrate, butyl 3-methyl-2-(3,3,4,4,4-pentafluorobutylthio)butyrate, isobutyl 3-methyl-2-(3,3,4,4,4-pentafluorobutylthio)butyrate, tert-butyl 3-methyl-2-(3,3,4,4,4-pentafluorobutylthio)butyrate, methyl 3-methyl-2-(4-fluoropentylthio)butyrate, ethyl 3-methyl-2-(4-fluoropentylthio)butyrate, propyl 3-methyl-2-(4-fluoropentylthio)butyrate, isopropyl 3-methyl-2-(4-fluoropentylthio)butyrate, butyl 3-methyl-2-(4-fluoropentylthio)butyrate, isobutyl 3-methyl-2-(4-fluoropentylthio)butyrate, tert-butyl 3-methyl-2-(4-fluoropentylthio)butyrate, methyl 3-methyl-2-(4,4-difluoropentylthio)butyrate, ethyl 3-methyl-2-(4,4-difluoropentylthio)butyrate, propyl 3-methyl-2-(4,4-difluoropentylthio)butyrate, isopropyl 3-methyl-2-(4,4-difluoropentylthio)butyrate, butyl 3-methyl-2-(4,4-difluoropentylthio)butyrate, isobutyl 3-methyl-2-(4,4-difluoropentylthio)butyrate, tert-butyl 3-methyl-2-(4,4-difluoropentylthio)butyrate, methyl 3-methyl-2-(5-fluoropentylthio)butyrate, ethyl 3-methyl-2-(5-fluoropentylthio)butyrate, propyl 3-methyl-2-(5-fluoropentylthio)butyrate, isopropyl 3-methyl-2-(5-fluoropentylthio)butyrate, butyl 3-methyl-2-(5-fluoropentylthio)butyrate, isobutyl 3-methyl-2-(5-fluoropentylthio)butyrate, tert-butyl 3-methyl-2-(5-fluoropentylthio)butyrate, methyl 3-methyl-2-(5,5-difluoropentylthio)butyrate, ethyl 3-methyl-2-(5,5-difluoropentylthio)butyrate, propyl 3-methyl-2-(5,5-difluoropentylthio)butyrate, isopropyl 3-methyl-2-(5,5-difluoropentylthio)butyrate, butyl 3-methyl-2-(5,5-difluoropentylthio)butyrate, isobutyl 3-methyl-2-(5,5-difluoropentylthio)butyrate, tert-butyl 3-methyl-2-(5,5-difluoropentylthio)butyrate, methyl 3-methyl-2-(5,5,5-trifluoropentylthio)butyrate, ethyl 3-methyl-2-(5,5,5-trifluoropentylthio)butyrate, propyl 3-methyl-2-(5,5,5-trifluoropentylthio)butyrate, isopropyl 3-methyl-2-(5,5,5-trifluoropentylthio)butyrate, butyl 3-methyl-2-(5,5,5-trifluoropentylthio)butyrate, isobutyl 3-methyl-2-(5,5,5-trifluoropentylthio)butyrate, tert-butyl 3-methyl-2-(5,5,5-trifluoropentylthio)butyrate, methyl 3-methyl-2-(4,4,5,5-tetrafluoropentylthio)butyrate, ethyl 3-methyl-2-(4,4,5,5-tetrafluoropentylthio)butyrate, propyl 3-methyl-2-(4,4,5,5-tetrafluoropentylthio)butyrate, isopropyl 3-methyl-2-(4,4,5,5-tetrafluoropentylthio)butyrate, butyl 3-methyl-2-(4,4,5,5-tetrafluoropentylthio)butyrate, isobutyl 3-methyl-2-(4,4,5,5-tetrafluoropentylthio)butyrate, tert-butyl 3-methyl-2-(4,4,5,5-tetrafluoropentylthio)butyrate, methyl 3-methyl-2-(4,4,5,5,5-pentafluoropentylthio)butyrate, ethyl 3-methyl-2-(4,4,5,5,5-pentafluoropentylthio)butyrate, propyl 3-methyl-2-(4,4,5,5,5-pentafluoropentylthio)butyrate, isopropyl 3-methyl-2-(4,4,5,5,5-pentafluoropentylthio)butyrate, butyl 3-methyl-2-(4,4,5,5,5-pentafluoropentylthio)butyrate, isobutyl 3-methyl-2-(4,4,5,5,5-pentafluoropentylthio)butyrate, tert-butyl 3-methyl-2-(4,4,5,5,5-pentafluoropentylthio)butyrate, methyl 3-methyl-2-(4,4,4-trifluoro-3-trifluoromethylthio)butyrate, ethyl 3-methyl-2-(4,4,4-trifluoro-3-trifluoromethylthio)butyrate, propyl 3-methyl-2-(4,4,4-trifluoro-3-trifluoromethylthio)butyrate, isopropyl 3-methyl-2-(4,4,4-trifluoro-3-trifluoromethylthio)butyrate, butyl 3-methyl-2-(4,4,4-trifluoro-3-trifluoromethylthio)butyrate, isobutyl 3-methyl-2-(4,4,4-trifluoro-3-trifluoromethylthio)butyrate, tert-butyl 3-methyl-2-(4,4,4-trifluoro-3-trifluoromethylthio)butyrate, methyl 3-methyl-2-(4-fluorohexylthio)butyrate, ethyl 3-methyl-2-(4-fluorohexylthio)butyrate, propyl 3-methyl-2-(4-fluorohexylthio)butyrate, isopropyl 3-methyl-2-(4-fluorohexylthio)butyrate, butyl 3-methyl-2-(4-fluorohexylthio)butyrate, isobutyl 3-methyl-2-(4-fluorohexylthio)butyrate, tert-butyl 3-methyl-2-(4-fluorohexylthio)butyrate, methyl 3-methyl-2-(4,4-difluorohexylthio)butyrate, ethyl 3-methyl-2-(4,4-difluorohexylthio)butyrate, propyl 3-methyl-2-(4,4-difluorohexylthio)butyrate, isopropyl 3-methyl-2-(4,4-difluorohexylthio)butyrate, butyl 3-methyl-2-(4,4-difluorohexylthio)butyrate, isobutyl 3-methyl-2-(4,4-difluorohexylthio)butyrate, tert-butyl 3-methyl-2-(4,4-difluorohexylthio)butyrate, methyl 3-methyl-2-(5-fluorohexylthio)butyrate, ethyl 3-methyl-2-(5-fluorohexylthio)butyrate, propyl 3-methyl-2-(5-fluorohexylthio)butyrate, isopropyl 3-methyl-2-(5-fluorohexylthio)butyrate, butyl 3-methyl-2-(5-fluorohexylthio)butyrate, isobutyl 3-methyl-2-(5-fluorohexylthio)butyrate, tert-butyl 3-methyl-2-(5-fluorohexylthio)butyrate, methyl 3-methyl-2-(5,5-difluorohexylthio)butyrate, ethyl 3-methyl-2-(5,5-difluorohexylthio)butyrate, propyl 3-methyl-2-(5,5-difluorohexylthio)butyrate, isopropyl 3-methyl-2-(5,5-difluorohexylthio)butyrate, butyl 3-methyl-2-(5,5-difluorohexylthio)butyrate, isobutyl 3-methyl-2-(5,5-difluorohexylthio)butyrate, tert-butyl 3-methyl-2-(5,5-difluorohexylthio)butyrate, methyl 3-methyl-2-(6-fluorohexylthio)butyrate, ethyl 3-methyl-2-(6-fluorohexylthio)butyrate, propyl 3-methyl-2-(6-fluorohexylthio)butyrate, isopropyl 3-methyl-2-(6-fluorohexylthio)butyrate, butyl 3-methyl-2-(6-fluorohexylthio)butyrate, isobutyl 3-methyl-2-(6-fluorohexylthio)butyrate, tert-butyl 3-methyl-2-(6-fluorohexylthio)butyrate, methyl 3-methyl-2-(6,6-difluorohexylthio)butyrate, ethyl 3-methyl-2-(6,6-difluorohexylthio)butyrate, propyl 3-methyl-2-(6,6-difluorohexylthio)butyrate, isopropyl 3-methyl-2-(6,6-difluorohexylthio)butyrate, butyl 3-methyl-2-(6,6-difluorohexylthio)butyrate, isobutyl 3-methyl-2-(6,6-difluorohexylthio)butyrate, tert-butyl 3-methyl-2-(6,6-difluorohexylthio)butyrate, methyl 3-methyl-2-(6,6,6-trifluorohexylthio)butyrate, ethyl 3-methyl-2-(6,6,6-trifluorohexylthio)butyrate, propyl 3-methyl-2-(6,6,6-trifluorohexylthio)butyrate, isopropyl 3-methyl-2-(6,6,6-trifluorohexylthio)butyrate, butyl 3-methyl-2-(6,6,6-trifluorohexylthio)butyrate, isobutyl 3-methyl-2-(6,6,6-trifluorohexylthio)butyrate, tert-butyl 3-methyl-2-(6,6,6-trifluorohexylthio)butyrate, methyl 3-methyl-2-(5,5,6,6,6-pentafluorohexylthio)butyrate, ethyl 3-methyl-2-(5,5,6,6,6-pentafluorohexylthio)butyrate, propyl 3-methyl-2-(5,5,6,6,6-pentafluorohexylthio)butyrate, isopropyl 3-methyl-2-(5,5,6,6,6-pentafluorohexylthio)butyrate, butyl 3-methyl-2-(5,5,6,6,6-pentafluorohexylthio)butyrate, isobutyl 3-methyl-2-(5,5,6,6,6-pentafluorohexylthio)butyrate, tert-butyl 3-methyl-2-(5,5,6,6,6-pentafluorohexylthio)butyrate, methyl 3-methyl-2-(4,4,5,6,6-pentafluorohexylthio)butyrate, ethyl 3-methyl-2-(4,4,5,6,6-pentafluorohexylthio)butyrate, propyl 3-methyl-2-(4,4,5,6,6-pentafluorohexylthio)butyrate, isopropyl 3-methyl-2-(4,4,5,6,6-pentafluorohexylthio)butyrate, butyl 3-methyl-2-(4,4,5,6,6-pentafluorohexylthio)butyrate, isobutyl 3-methyl-2-(4,4,5,6,6-pentafluorohexylthio)butyrate, tert-butyl 3-methyl-2-(4,4,5,6,6-pentafluorohexylthio)butyrate, methyl 3-methyl-2-(4,4,5,5,6,6,6-heptafluorohexylthio)butyrate, ethyl 3-methyl-2-(4,4,5,5,6,6,6-heptafluorohexylthio)butyrate, propyl 3-methyl-2-(4,4,5,5,6,6,6-heptafluorohexylthio)butyrate, isopropyl 3-methyl-2-(4,4,5,5,6,6,6-heptafluorohexylthio)butyrate, butyl 3-methyl-2-(4,4,5,5,6,6,6-heptafluorohexylthio)butyrate, isobutyl 3-methyl-2-(4,4,5,5,6,6,6-heptafluorohexylthio)butyrate, tert-butyl 3-methyl-2-(4,4,5,5,6,6,6-heptafluorohexylthio)butyrate, methyl 3-methyl-2-(4-fluoroheptylthio)butyrate, ethyl 3-methyl-2-(4-fluoroheptylthio)butyrate, propyl 3-methyl-2-(4-fluoroheptylthio)butyrate, isopropyl 3-methyl-2-(4-fluoroheptylthio)butyrate, butyl 3-methyl-2-(4-fluoroheptylthio)butyrate, isobutyl 3-methyl-2-(4-fluoroheptylthio)butyrate, tert-butyl 3-methyl-2-(4-fluoroheptylthio)butyrate, methyl 3-methyl-2-(4,4-difluoroheptylthio)butyrate, ethyl 3-methyl-2-(4,4-difluoroheptylthio)butyrate, propyl 3-methyl-2-(4,4-difluoroheptylthio)butyrate, isopropyl 3-methyl-2-(4,4-difluoroheptylthio)butyrate, butyl 3-methyl-2-(4,4-difluoroheptylthio)butyrate, isobutyl 3-methyl-2-(4,4-difluoroheptylthio)butyrate, tert-butyl 3-methyl-2-(4,4-difluoroheptylthio)butyrate, methyl 3-methyl-2-(5-fluoroheptylthio)butyrate, ethyl 3-methyl-2-(5-fluoroheptylthio)butyrate, propyl 3-methyl-2-(5-fluoroheptylthio)butyrate, isopropyl 3-methyl-2-(5-fluoroheptylthio)butyrate, butyl 3-methyl-2-(5-fluoroheptylthio)butyrate, isobutyl 3-methyl-2-(5-fluoroheptylthio)butyrate, tert-butyl 3-methyl-2-(5-fluoroheptylthio)butyrate, methyl 3-methyl-2-(5,5-difluoroheptylthio)butyrate, ethyl 3-methyl-2-(5,5-difluoroheptylthio)butyrate, propyl 3-methyl-2-(5,5-difluoroheptylthio)butyrate, isopropyl 3-methyl-2-(5,5-difluoroheptylthio)butyrate, butyl 3-methyl-2-(5,5-difluoroheptylthio)butyrate, isobutyl 3-methyl-2-(5,5-difluoroheptylthio)butyrate, tert-butyl 3-methyl-2-(5,5-difluoroheptylthio)butyrate, methyl 3-methyl-2-(6-fluoroheptylthio)butyrate, ethyl 3-methyl-2-(6-fluoroheptylthio)butyrate, propyl 3-methyl-2-(6-fluoroheptylthio)butyrate, isopropyl 3-methyl-2-(6-fluoroheptylthio)butyrate, butyl 3-methyl-2-(6-fluoroheptylthio)butyrate, isobutyl 3-methyl-2-(6-fluoroheptylthio)butyrate, tert-butyl 3-methyl-2-(6-fluoroheptylthio)butyrate, methyl 3-methyl-2-(6,6-difluoroheptylthio)butyrate, ethyl 3-methyl-2-(6,6-difluoroheptylthio)butyrate, propyl 3-methyl-2-(6,6-difluoroheptylthio)butyrate, isopropyl 3-methyl-2-(6,6-difluoroheptylthio)butyrate, butyl 3-methyl-2-(6,6-difluoroheptylthio)butyrate, isobutyl 3-methyl-2-(6,6-difluoroheptylthio)butyrate, tert-butyl 3-methyl-2-(6,6-difluoroheptylthio)butyrate, methyl 3-methyl-2-(7-fluoroheptylthio)butyrate, ethyl 3-methyl-2-(7-fluoroheptylthio)butyrate, propyl 3-methyl-2-(7-fluoroheptylthio)butyrate, isopropyl 3-methyl-2-(7-fluoroheptylthio)butyrate, butyl 3-methyl-2-(7-fluoroheptylthio)butyrate, isobutyl 3-methyl-2-(7-fluoroheptylthio)butyrate, tert-butyl 3-methyl-2-(7-fluoroheptylthio)butyrate, methyl 3-methyl-2-(7,7-difluoroheptylthio)butyrate, ethyl 3-methyl-2-(7,7-difluoroheptylthio)butyrate, propyl 3-methyl-2-(7,7-difluoroheptylthio)butyrate, isopropyl 3-methyl-2-(7,7-difluoroheptylthio)butyrate, butyl 3-methyl-2-(7,7-difluoroheptylthio)butyrate, isobutyl 3-methyl-2-(7,7-difluoroheptylthio)butyrate, tert-butyl 3-methyl-2-(7,7-difluoroheptylthio)butyrate, methyl 3-methyl-2-(7,7,7-trifluoroheptylthio)butyrate, ethyl 3-methyl-2-(7,7,7-trifluoroheptylthio)butyrate, propyl 3-methyl-2-(7,7,7-trifluoroheptylthio)butyrate, isopropyl 3-methyl-2-(7,7,7-trifluoroheptylthio)butyrate, butyl 3-methyl-2-(7,7,7-trifluoroheptylthio)butyrate, isobutyl 3-methyl-2-(7,7,7-trifluoroheptylthio)butyrate, tert-butyl 3-methyl-2-(7,7,7-trifluoroheptylthio)butyrate, methyl 3-methyl-2-(6,6,7,7,7-pentafluoroheptylthio)butyrate, ethyl 3-methyl-2-(6,6,7,7,7-pentafluoroheptylthio)butyrate, propyl 3-methyl-2-(6,6,7,7,7-pentafluoroheptylthio)butyrate, isopropyl 3-methyl-2-(6,6,7,7,7-pentafluoroheptylthio)butyrate, butyl 3-methyl-2-(6,6,7,7,7-pentafluoroheptylthio)butyrate, isobutyl 3-methyl-2-(6,6,7,7,7-pentafluoroheptylthio)butyrate, tert-butyl 3-methyl-2-(6,6,7,7,7-pentafluoroheptylthio)butyrate, methyl 3-methyl-2-(5,5,6,6,7,7,7-heptafluoroheptylthio)butyrate, ethyl 3-methyl-2-(5,5,6,6,7,7,7-heptafluoroheptylthio)butyrate, propyl 3-methyl-2-(5,5,6,6,7,7,7-heptafluoroheptylthio)butyrate, isopropyl 3-methyl-2-(5,5,6,6,7,7,7-heptafluoroheptylthio)butyrate, butyl 3-methyl-2-(5,5,6,6,7,7,7-heptafluoroheptylthio)butyrate, isobutyl 3-methyl-2-(5,5,6,6,7,7,7-heptafluoroheptylthio)butyrate, tert-butyl 3-methyl-2-(5,5,6,6,7,7,7-heptafluoroheptylthio)butyrate, methyl 3-methyl-2-(4,4,5,5,6,6,7,7,7-nonafluoroheptylthio)butyrate, ethyl 3-methyl-2-(4,4,5,5,6,6,7,7,7-nonafluoroheptylthio)butyrate, propyl 3-methyl-2-(4,4,5,5,6,6,7,7,7-nonafluoroheptylthio)butyrate, isopropyl 3-methyl-2-(4,4,5,5,6,6,7,7,7-nonafluoroheptylthio)butyrate, butyl 3-methyl-2-(4,4,5,5,6,6,7,7,7-nonafluoroheptylthio)butyrate, isobutyl 3-methyl-2-(4,4,5,5,6,6,7,7,7-nonafluoroheptylthio)butyrate, tert-butyl 3-methyl-2-(4,4,5,5,6,6,7,7,7-nonafluoroheptylthio)butyrate, methyl 2-(3-fluoropropylthio)valerate, ethyl 2-(3-fluoropropylthio)valerate, propyl 2-(3-fluoropropylthio)valerate, isopropyl 2-(3-fluoropropylthio)valerate, butyl 2-(3-fluoropropylthio)valerate, isobutyl 2-(3-fluoropropylthio)valerate, tert-butyl 2-(3-fluoropropylthio)valerate, methyl 2-(3,3-difluoropropylthio)valerate, ethyl 2-(3,3-difluoropropylthio)valerate, propyl 2-(3,3-difluoropropylthio)valerate, isopropyl 2-(3,3-difluoropropylthio)valerate, butyl 2-(3,3-difluoropropylthio)valerate, isobutyl 2-(3,3-difluoropropylthio)valerate, tert-butyl 2-(3,3-difluoropropylthio)valerate, methyl 2-(3,3,3-trifluoropropylthio)valerate, ethyl 2-(3,3,3-trifluoropropylthio)valerate, propyl 2-(3,3,3-trifluoropropylthio)valerate, isopropyl 2-(3,3,3-trifluoropropylthio)valerate, butyl 2-(3,3,3-trifluoropropylthio)valerate, isobutyl 2-(3,3,3-trifluoropropylthio)valerate, tert-butyl 2-(3,3,3-trifluoropropylthio)valerate, methyl 2-(4-fluorobutylthio)valerate, ethyl 2-(4-fluorobutylthio)valerate, propyl 2-(4-fluorobutylthio)valerate, isopropyl 2-(4-fluorobutylthio)valerate, butyl 2-(4-fluorobutylthio)valerate, isobutyl 2-(4-fluorobutylthio)valerate, tert-butyl 2-(4-fluorobutylthio)valerate, methyl 2-(4,4,4-trifluorobutylthio)valerate, ethyl 2-(4,4,4-trifluorobutylthio)valerate, propyl 2-(4,4,4-trifluorobutylthio)valerate, isopropyl 2-(4,4,4-trifluorobutylthio)valerate, butyl 2-(4,4,4-trifluorobutylthio)valerate, isobutyl 2-(4,4,4-trifluorobutylthio)valerate, tert-butyl 2-(4,4,4-trifluorobutylthio)valerate, methyl 2-(3,3,4,4,4-pentafluorobutylthio)valerate, ethyl 2-(3,3,4,4,4-pentafluorobutylthio)valerate, propyl 2-(3,3,4,4,4-pentafluorobutylthio)valerate, isopropyl 2-(3,3,4,4,4-pentafluorobutylthio)valerate, butyl 2-(3,3,4,4,4-pentafluorobutylthio)valerate, isobutyl 2-(3,3,4,4,4-pentafluorobutylthio)valerate, tert-butyl 2-(3,3,4,4,4-pentafluorobutylthio)valerate, methyl 2-(4-fluoropentylthio)valerate, ethyl 2-(4-fluoropentylthio)valerate, propyl 2-(4-fluoropentylthio)valerate, isopropyl 2-(4-fluoropentylthio)valerate, butyl 2-(4-fluoropentylthio)valerate, isobutyl 2-(4-fluoropentylthio)valerate, tert-butyl 2-(4-fluoropentylthio)valerate, methyl 2-(4,4-difluoropentylthio)valerate, ethyl 2-(4,4-difluoropentylthio)valerate, propyl 2-(4,4-difluoropentylthio)valerate, isopropyl 2-(4,4-difluoropentylthio)valerate, butyl 2-(4,4-difluoropentylthio)valerate, isobutyl 2-(4,4-difluoropentylthio)valerate, tert-butyl 2-(4,4-difluoropentylthio)valerate, methyl 2-(5-fluoropentylthio)valerate, ethyl 2-(5-fluoropentylthio)valerate, propyl 2-(5-fluoropentylthio)valerate, isopropyl 2-(5-fluoropentylthio)valerate, butyl 2-(5-fluoropentylthio)valerate, isobutyl 2-(5-fluoropentylthio)valerate, tert-butyl 2-(5-fluoropentylthio)valerate, methyl 2-(5,5-difluoropentylthio)valerate, ethyl 2-(5,5-difluoropentylthio)valerate, propyl 2-(5,5-difluoropentylthio)valerate, isopropyl 2-(5,5-difluoropentylthio)valerate, butyl 2-(5,5-difluoropentylthio)valerate, isobutyl 2-(5,5-difluoropentylthio)valerate, tert-butyl 2-(5,5-difluoropentylthio)valerate, methyl 2-(5,5,5-trifluoropentylthio)valerate, ethyl 2-(5,5,5-trifluoropentylthio)valerate, propyl 2-(5,5,5-trifluoropentylthio)valerate, isopropyl 2-(5,5,5-trifluoropentylthio)valerate, butyl 2-(5,5,5-trifluoropentylthio)valerate, isobutyl 2-(5,5,5-trifluoropentylthio)valerate, tert-butyl 2-(5,5,5-trifluoropentylthio)valerate, methyl 2-(4,4,5,5-tetrafluoropentylthio)valerate, ethyl 2-(4,4,5,5-tetrafluoropentylthio)valerate, propyl 2-(4,4,5,5-tetrafluoropentylthio)valerate, isopropyl 2-(4,4,5,5-tetrafluoropentylthio)valerate, butyl 2-(4,4,5,5-tetrafluoropentylthio)valerate, isobutyl 2-(4,4,5,5-tetrafluoropentylthio)valerate, tert-butyl 2-(4,4,5,5-tetrafluoropentylthio)valerate, methyl 2-(4,4,5,5,5-pentafluoropentylthio)valerate, ethyl 2-(4,4,5,5,5-pentafluoropentylthio)valerate, propyl 2-(4,4,5,5,5-pentafluoropentylthio)valerate, isopropyl 2-(4,4,5,5,5-pentafluoropentylthio)valerate, butyl 2-(4,4,5,5,5-pentafluoropentylthio)valerate, isobutyl 2-(4,4,5,5,5-pentafluoropentylthio)valerate, tert-butyl 2-(4,4,5,5,5-pentafluoropentylthio)valerate, methyl 2-(4,4,4-trifluoro-3-trifluoromethylthio)valerate, ethyl 2-(4,4,4-trifluoro-3-trifluoromethylthio)valerate, propyl 2-(4,4,4-trifluoro-3-trifluoromethylthio)valerate, isopropyl 2-(4,4,4-trifluoro-3-trifluoromethylthio)valerate, butyl 2-(4,4,4-trifluoro-3-trifluoromethylthio)valerate, isobutyl 2-(4,4,4-trifluoro-3-trifluoromethylthio)valerate, tert-butyl 2-(4,4,4-trifluoro-3-trifluoromethylthio)valerate, methyl 2-(4-fluorohexylthio)valerate, ethyl 2-(4-fluorohexylthio)valerate, propyl 2-(4-fluorohexylthio)valerate, isopropyl 2-(4-fluorohexylthio)valerate, butyl 2-(4-fluorohexylthio)valerate, isobutyl 2-(4-fluorohexylthio)valerate, tert-butyl 2-(4-fluorohexylthio)valerate, methyl 2-(4,4-difluorohexylthio)valerate, ethyl 2-(4,4-difluorohexylthio)valerate, propyl 2-(4,4-difluorohexylthio)valerate, isopropyl 2-(4,4-difluorohexylthio)valerate, butyl 2-(4,4-difluorohexylthio)valerate, isobutyl 2-(4,4-difluorohexylthio)valerate, tert-butyl 2-(4,4-difluorohexylthio)valerate, methyl 2-(5-fluorohexylthio)valerate, ethyl 2-(5-fluorohexylthio)valerate, propyl 2-(5-fluorohexylthio)valerate, isopropyl 2-(5-fluorohexylthio)valerate, butyl 2-(5-fluorohexylthio)valerate, isobutyl 2-(5-fluorohexylthio)valerate, tert-butyl 2-(5-fluorohexylthio)valerate, methyl 2-(5,5-difluorohexylthio)valerate, ethyl 2-(5,5-difluorohexylthio)valerate, propyl 2-(5,5-difluorohexylthio)valerate, isopropyl 2-(5,5-difluorohexylthio)valerate, butyl 2-(5,5-difluorohexylthio)valerate, isobutyl 2-(5,5-difluorohexylthio)valerate, tert-butyl 2-(5,5-difluorohexylthio)valerate, methyl 2-(6-fluorohexylthio)valerate, ethyl 2-(6-fluorohexylthio)valerate, propyl 2-(6-fluorohexylthio)valerate, isopropyl 2-(6-fluorohexylthio)valerate, butyl 2-(6-fluorohexylthio)valerate, isobutyl 2-(6-fluorohexylthio)valerate, tert-butyl 2-(6-fluorohexylthio)valerate, methyl 2-(6,6-difluorohexylthio)valerate, ethyl 2-(6,6-difluorohexylthio)valerate, propyl 2-(6,6-difluorohexylthio)valerate, isopropyl 2-(6,6-difluorohexylthio)valerate, butyl 2-(6,6-difluorohexylthio)valerate, isobutyl 2-(6,6-difluorohexylthio)valerate, tert-butyl 2-(6,6-difluorohexylthio)valerate, methyl 2-(6,6,6-trifluorohexylthio)valerate, ethyl 2-(6,6,6-trifluorohexylthio)valerate, propyl 2-(6,6,6-trifluorohexylthio)valerate, isopropyl 2-(6,6,6-trifluorohexylthio)valerate, butyl 2-(6,6,6-trifluorohexylthio)valerate, isobutyl 2-(6,6,6-trifluorohexylthio)valerate, tert-butyl 2-(6,6,6-trifluorohexylthio)valerate, methyl 2-(5,5,6,6,6-pentafluorohexylthio)valerate, ethyl 2-(5,5,6,6,6-pentafluorohexylthio)valerate, propyl 2-(5,5,6,6,6-pentafluorohexylthio)valerate, isopropyl 2-(5,5,6,6,6-pentafluorohexylthio)valerate, butyl 2-(5,5,6,6,6-pentafluorohexylthio)valerate, isobutyl 2-(5,5,6,6,6-pentafluorohexylthio)valerate, tert-butyl 2-(5,5,6,6,6-pentafluorohexylthio)valerate, methyl 2-(4,4,5,6,6-pentafluorohexylthio)valerate, ethyl 2-(4,4,5,6,6-pentafluorohexylthio)valerate, propyl 2-(4,4,5,6,6-pentafluorohexylthio)valerate, isopropyl 2-(4,4,5,6,6-pentafluorohexylthio)valerate, butyl 2-(4,4,5,6,6-pentafluorohexylthio)valerate, isobutyl 2-(4,4,5,6,6-pentafluorohexylthio)valerate, tert-butyl 2-(4,4,5,6,6-pentafluorohexylthio)valerate, methyl 2-(4,4,5,5,6,6,6-heptafluorohexylthio)valerate, ethyl 2-(4,4,5,5,6,6,6-heptafluorohexylthio)valerate, propyl 2-(4,4,5,5,6,6,6-heptafluorohexylthio)valerate, isopropyl 2-(4,4,5,5,6,6,6-heptafluorohexylthio)valerate, butyl 2-(4,4,5,5,6,6,6-heptafluorohexylthio)valerate, isobutyl 2-(4,4,5,5,6,6,6-heptafluorohexylthio)valerate, tert-butyl 2-(4,4,5,5,6,6,6-heptafluorohexylthio)valerate, methyl 2-(4-fluoroheptylthio)valerate, ethyl 2-(4-fluoroheptylthio)valerate, propyl 2-(4-fluoroheptylthio)valerate, isopropyl 2-(4-fluoroheptylthio)valerate, butyl 2-(4-fluoroheptylthio)valerate, isobutyl 2-(4-fluoroheptylthio)valerate, tert-butyl 2-(4-fluoroheptylthio)valerate, methyl 2-(4,4-difluoroheptylthio)valerate, ethyl 2-(4,4-difluoroheptylthio)valerate, propyl 2-(4,4-difluoroheptylthio)valerate, isopropyl 2-(4,4-difluoroheptylthio)valerate, butyl 2-(4,4-difluoroheptylthio)valerate, isobutyl 2-(4,4-difluoroheptylthio)valerate, tert-butyl 2-(4,4-difluoroheptylthio)valerate, methyl 2-(5-fluoroheptylthio)valerate, ethyl 2-(5-fluoroheptylthio)valerate, propyl 2-(5-fluoroheptylthio)valerate, isopropyl 2-(5-fluoroheptylthio)valerate, butyl 2-(5-fluoroheptylthio)valerate, isobutyl 2-(5-fluoroheptylthio)valerate, tert-butyl 2-(5-fluoroheptylthio)valerate, methyl 2-(5,5-difluoroheptylthio)valerate, ethyl 2-(5,5-difluoroheptylthio)valerate, propyl 2-(5,5-difluoroheptylthio)valerate, isopropyl 2-(5,5-difluoroheptylthio)valerate, butyl 2-(5,5-difluoroheptylthio)valerate, isobutyl 2-(5,5-difluoroheptylthio)valerate, tert-butyl 2-(5,5-difluoroheptylthio)valerate, methyl 2-(6-fluoroheptylthio)valerate, ethyl 2-(6-fluoroheptylthio)valerate, propyl 2-(6-fluoroheptylthio)valerate, isopropyl 2-(6-fluoroheptylthio)valerate, butyl 2-(6-fluoroheptylthio)valerate, isobutyl 2-(6-fluoroheptylthio)valerate, tert-butyl 2-(6-fluoroheptylthio)valerate, methyl 2-(6,6-difluoroheptylthio)valerate, ethyl 2-(6,6-difluoroheptylthio)valerate, propyl 2-(6,6-difluoroheptylthio)valerate, isopropyl 2-(6,6-difluoroheptylthio)valerate, butyl 2-(6,6-difluoroheptylthio)valerate, isobutyl 2-(6,6-difluoroheptylthio)valerate, tert-butyl 2-(6,6-difluoroheptylthio)valerate, methyl 2-(7-fluoroheptylthio)valerate, ethyl 2-(7-fluoroheptylthio)valerate, propyl 2-(7-fluoroheptylthio)valerate, isopropyl 2-(7-fluoroheptylthio)valerate, butyl 2-(7-fluoroheptylthio)valerate, isobutyl 2-(7-fluoroheptylthio)valerate, tert-butyl 2-(7-fluoroheptylthio)valerate, methyl 2-(7,7-difluoroheptylthio)valerate, ethyl 2-(7,7-difluoroheptylthio)valerate, propyl 2-(7,7-difluoroheptylthio)valerate, isopropyl 2-(7,7-difluoroheptylthio)valerate, butyl 2-(7,7-difluoroheptylthio)valerate, isobutyl 2-(7,7-difluoroheptylthio)valerate, tert-butyl 2-(7,7-difluoroheptylthio)valerate, methyl 2-(7,7,7-trifluoroheptylthio)valerate, ethyl 2-(7,7,7-trifluoroheptylthio)valerate, propyl 2-(7,7,7-trifluoroheptylthio)valerate, isopropyl 2-(7,7,7-trifluoroheptylthio)valerate, butyl 2-(7,7,7-trifluoroheptylthio)valerate, isobutyl 2-(7,7,7-trifluoroheptylthio)valerate, tert-butyl 2-(7,7,7-trifluoroheptylthio)valerate, methyl 2-(6,6,7,7,7-pentafluoroheptylthio)valerate, ethyl 2-(6,6,7,7,7-pentafluoroheptylthio)valerate, propyl 2-(6,6,7,7,7-pentafluoroheptylthio)valerate, isopropyl 2-(6,6,7,7,7-pentafluoroheptylthio)valerate, butyl 2-(6,6,7,7,7-pentafluoroheptylthio)valerate, isobutyl 2-(6,6,7,7,7-pentafluoroheptylthio)valerate, tert-butyl 2-(6,6,7,7,7-pentafluoroheptylthio)valerate, methyl 2-(5,5,6,6,7,7,7-heptafluoroheptylthio)valerate, ethyl 2-(5,5,6,6,7,7,7-heptafluoroheptylthio)valerate, propyl 2-(5,5,6,6,7,7,7-heptafluoroheptylthio)valerate, isopropyl 2-(5,5,6,6,7,7,7-heptafluoroheptylthio)valerate, butyl 2-(5,5,6,6,7,7,7-heptafluoroheptylthio)valerate, isobutyl 2-(5,5,6,6,7,7,7-heptafluoroheptylthio)valerate, tert-butyl 2-(5,5,6,6,7,7,7-heptafluoroheptylthio)valerate, methyl 2-(4,4,5,5,6,6,7,7,7-nonafluoroheptylthio)valerate, ethyl 2-(4,4,5,5,6,6,7,7,7-nonafluoroheptylthio)valerate, propyl 2-(4,4,5,5,6,6,7,7,7-nonafluoroheptylthio)valerate, isopropyl 2-(4,4,5,5,6,6,7,7,7-nonafluoroheptylthio)valerate, butyl 2-(4,4,5,5,6,6,7,7,7-nonafluoroheptylthio)valerate, isobutyl 2-(4,4,5,5,6,6,7,7,7-nonafluoroheptylthio)valerate, tert-butyl 2-(4,4,5,5,6,6,7,7,7-nonafluoroheptylthio)valerate, methyl 4-methyl-2-(3-fluoropropylthio)valerate, ethyl 4-methyl-2-(3-fluoropropylthio)valerate, propyl 4-methyl-2-(3-fluoropropylthio)valerate, isopropyl 4-methyl-2-(3-fluoropropylthio)valerate, butyl 4-methyl-2-(3-fluoropropylthio)valerate, isobutyl 4-methyl-2-(3-fluoropropylthio)valerate, tert-butyl 4-methyl-2-(3-fluoropropylthio)valerate, methyl 4-methyl-2-(3,3-difluoropropylthio)valerate, ethyl 4-methyl-2-(3,3-difluoropropylthio)valerate, propyl 4-methyl-2-(3,3-difluoropropylthio)valerate, isopropyl 4-methyl-2-(3,3-difluoropropylthio)valerate, butyl 4-methyl-2-(3,3-difluoropropylthio)valerate, isobutyl 4-methyl-2-(3,3-difluoropropylthio)valerate, tert-butyl 4-methyl-2-(3,3-difluoropropylthio)valerate, methyl 4-methyl-2-(3,3,3-trifluoropropylthio)valerate, ethyl 4-methyl-2-(3,3,3-trifluoropropylthio)valerate, propyl 4-methyl-2-(3,3,3-trifluoropropylthio)valerate, isopropyl 4-methyl-2-(3,3,3-trifluoropropylthio)valerate, butyl 4-methyl-2-(3,3,3-trifluoropropylthio)valerate, isobutyl 4-methyl-2-(3,3,3-trifluoropropylthio)valerate, tert-butyl 4-methyl-2-(3,3,3-trifluoropropylthio)valerate, methyl 4-methyl-2-(4-fluorobutylthio)valerate, ethyl 4-methyl-2-(4-fluorobutylthio)valerate, propyl 4-methyl-2-(4-fluorobutylthio)valerate, isopropyl 4-methyl-2-(4-fluorobutylthio)valerate, butyl 4-methyl-2-(4-fluorobutylthio)valerate, isobutyl 4-methyl-2-(4-fluorobutylthio)valerate, tert-butyl 4-methyl-2-(4-fluorobutylthio)valerate, methyl 4-methyl-2-(4,4,4-trifluorobutylthio)valerate, ethyl 4-methyl-2-(4,4,4-trifluorobutylthio)valerate, propyl 4-methyl-2-(4,4,4-trifluorobutylthio)valerate, isopropyl 4-methyl-2-(4,4,4-trifluorobutylthio)valerate, butyl 4-methyl-2-(4,4,4-trifluorobutylthio)valerate, isobutyl 4-methyl-2-(4,4,4-trifluorobutylthio)valerate, tert-butyl 4-methyl-2-(4,4,4-trifluorobutylthio)valerate, methyl 4-methyl-2-(3,3,4,4,4-pentafluorobutylthio)valerate, ethyl 4-methyl-2-(3,3,4,4,4-pentafluorobutylthio)valerate, propyl 4-methyl-2-(3,3,4,4,4-pentafluorobutylthio)valerate, isopropyl 4-methyl-2-(3,3,4,4,4-pentafluorobutylthio)valerate, butyl 4-methyl-2-(3,3,4,4,4-pentafluorobutylthio)valerate, isobutyl 4-methyl-2-(3,3,4,4,4-pentafluorobutylthio)valerate, tert-butyl 4-methyl-2-(3,3,4,4,4-pentafluorobutylthio)valerate, methyl 4-methyl-2-(4-fluoropentylthio)valerate, ethyl 4-methyl-2-(4-fluoropentylthio)valerate, propyl 4-methyl-2-(4-fluoropentylthio)valerate, isopropyl 4-methyl-2-(4-fluoropentylthio)valerate, butyl 4-methyl-2-(4-fluoropentylthio)valerate, isobutyl 4-methyl-2-(4-fluoropentylthio)valerate, tert-butyl 4-methyl-2-(-4-fluoropentylthio)valerate, methyl 4-methyl-2-(4,4-difluoropentylthio)valerate, ethyl 4-methyl-2-(4,4-difluoropentylthio)valerate, propyl 4-methyl-2-(4,4-difluoropentylthio)valerate, isopropyl 4-methyl-2-(4,4-difluoropentylthio)valerate, butyl 4-methyl-2-(4,4-difluoropentylthio)valerate, isobutyl 4-methyl-2-(4,4-difluoropentylthio)valerate, tert-butyl 4-methyl-2-(4,4-difluoropentylthio)valerate, methyl 4-methyl-2-(5-fluoropentylthio)valerate, ethyl 4-methyl-2-(5-fluoropentylthio)valerate, propyl 4-methyl-2-(5-fluoropentylthio)valerate, isopropyl 4-methyl-2-(5-fluoropentylthio)valerate, butyl 4-methyl-2-(5-fluoropentylthio)valerate, isobutyl 4-methyl-2-(5-fluoropentylthio)valerate, tert-butyl 4-methyl-2-(5-fluoropentylthio)valerate, methyl 4-methyl-2-(5,5-difluoropentylthio)valerate, ethyl 4-methyl-2-(5,5-difluoropentylthio)valerate, propyl 4-methyl-2-(5,5-difluoropentylthio)valerate, isopropyl 4-methyl-2-(5,5-difluoropentylthio)valerate, butyl 4-methyl-2-(5,5-difluoropentylthio)valerate, isobutyl 4-methyl-2-(5,5-difluoropentylthio)valerate, tert-butyl 4-methyl-2-(5,5-difluoropentylthio)valerate, methyl 4-methyl-2-(5,5,5-trifluoropentylthio)valerate, ethyl 4-methyl-2-(5,5,5-trifluoropentylthio)valerate, propyl 4-methyl-2-(5,5,5-trifluoropentylthio)valerate, isopropyl 4-methyl-2-(5,5,5-trifluoropentylthio)valerate, butyl 4-methyl-2-(5,5,5-trifluoropentylthio)valerate, isobutyl 4-methyl-2-(5,5,5-trifluoropentylthio)valerate, tert-butyl 4-methyl-2-(5,5,5-trifluoropentylthio)valerate, methyl 4-methyl-2-(4,4,5,5-tetrafluoropentylthio)valerate, ethyl 4-methyl-2-(4,4,5,5-tetrafluoropentylthio)valerate, propyl 4-methyl-2-(4,4,5,5-tetrafluoropentylthio)valerate, isopropyl 4-methyl-2-(4,4,5,5-tetrafluoropentylthio)valerate, butyl 4-methyl-2-(4,4,5,5-tetrafluoropentylthio)valerate, isobutyl 4-methyl-2-(4,4,5,5-tetrafluoropentylthio)valerate, tert-butyl 4-methyl-2-(4,4,5,5-tetrafluoropentylthio)valerate, methyl 4-methyl-2-(4,4,5,5,5-pentafluoropentylthio)valerate, ethyl 4-methyl-2-(4,4,5,5,5-pentafluoropentylthio)valerate, propyl 4-methyl-2-(4,4,5,5,5-pentafluoropentylthio)valerate, isopropyl 4-methyl-2-(4,4,5,5,5-pentafluoropentylthio)valerate, butyl 4-methyl-2-(4,4,5,5,5-pentafluoropentylthio)valerate, isobutyl 4-methyl-2-(4,4,5,5,5-pentafluoropentylthio)valerate, tert-butyl 4-methyl-2-(4,4,5,5,5-pentafluoropentylthio)valerate, methyl 4-methyl-2-(4,4,4-trifluoro-3-trifluoromethylthio)valerate, ethyl 4-methyl-2-(4,4,4-trifluoro-3-trifluoromethylthio)valerate, propyl 4-methyl-2-(4,4,4-trifluoro-3-trifluoromethylthio)valerate, isopropyl 4-methyl-2-(4,4,4-trifluoro-3-trifluoromethylthio)valerate, butyl 4-methyl-2-(4,4,4-trifluoro-3-trifluoromethylthio)valerate, isobutyl 4-methyl-2-(4,4,4-trifluoro-3-trifluoromethylthio)valerate, tert-butyl 4-methyl-2-(4,4,4-trifluoro-3-trifluoromethylthio)valerate, methyl 4-methyl-2-(4-fluorohexylthio)valerate, ethyl 4-methyl-2-(4-fluorohexylthio)valerate, propyl 4-methyl-2-(4-fluorohexylthio)valerate, isopropyl 4-methyl-2-(4-fluorohexylthio)valerate, butyl 4-methyl-2-(4-fluorohexylthio)valerate, isobutyl 4-methyl-2-(4-fluorohexylthio)valerate, tert-butyl 4-methyl-2-(4-fluorohexylthio)valerate, methyl 4-methyl-2-(4,4-difluorohexylthio)valerate, ethyl 4-methyl-2-(4,4-difluorohexylthio)valerate, propyl 4-methyl-2-(4,4-difluorohexylthio)valerate, isopropyl 4-methyl-2-(4,4-difluorohexylthio)valerate, butyl 4-methyl-2-(4,4-difluorohexylthio)valerate, isobutyl 4-methyl-2-(4,4-difluorohexylthio)valerate, tert-butyl 4-methyl-2-(4,4-difluorohexylthio)valerate, methyl 4-methyl-2-(5-fluorohexylthio)valerate, ethyl 4-methyl-2-(5-fluorohexylthio)valerate, propyl 4-methyl-2-(5-fluorohexylthio)valerate, isopropyl 4-methyl-2-(5-fluorohexylthio)valerate, butyl 4-methyl-2-(5-fluorohexylthio)valerate, isobutyl 4-methyl-2-(5-fluorohexylthio)valerate, tert-butyl 4-methyl-2-(5-fluorohexylthio)valerate, methyl 4-methyl-2-(5,5-difluorohexylthio)valerate, ethyl 4-methyl-2-(5,5-difluorohexylthio)valerate, propyl 4-methyl-2-(5,5-difluorohexylthio)valerate, isopropyl 4-methyl-2-(5,5-difluorohexylthio)valerate, butyl 4-methyl-2-(5,5-difluorohexylthio)valerate, isobutyl 4-methyl-2-(5,5-difluorohexylthio)valerate, tert-butyl 4-methyl-2-(5,5-difluorohexylthio)valerate, methyl 4-methyl-2-(6-fluorohexylthio)valerate, ethyl 4-methyl-2-(6-fluorohexylthio)valerate, propyl 4-methyl-2-(6-fluorohexylthio)valerate, isopropyl 4-methyl-2-(6-fluorohexylthio)valerate, butyl 4-methyl-2-(6-fluorohexylthio)valerate, isobutyl 4-methyl-2-(6-fluorohexylthio)valerate, tert-butyl 4-methyl-2-(6-fluorohexylthio)valerate, methyl 4-methyl-2-(6,6-difluorohexylthio)valerate, ethyl 4-methyl-2-(6,6-difluorohexylthio)valerate, propyl 4-methyl-2-(6,6-difluorohexylthio)valerate, isopropyl 4-methyl-2-(6,6-difluorohexylthio)valerate, butyl 4-methyl-2-(6,6-difluorohexylthio)valerate, isobutyl 4-methyl-2-(6,6-difluorohexylthio)valerate, tert-butyl 4-methyl-2-(6,6-difluorohexylthio)valerate, methyl 4-methyl-2-(6,6,6-trifluorohexylthio)valerate, ethyl 4-methyl-2-(6,6,6-trifluorohexylthio)valerate, propyl 4-methyl-2-(6,6,6-trifluorohexylthio)valerate, isopropyl 4-methyl-2-(6,6,6-trifluorohexylthio)valerate, butyl 4-methyl-2-(6,6,6-trifluorohexylthio)valerate, isobutyl 4-methyl-2-(6,6,6-trifluorohexylthio)valerate, tert-butyl 4-methyl-2-(6,6,6-trifluorohexylthio)valerate, methyl 4-methyl-2-(5,5,6,6,6-pentafluorohexylthio)valerate, ethyl 4-methyl-2-(5,5,6,6,6-pentafluorohexylthio)valerate, propyl 4-methyl-2-(5,5,6,6,6-pentafluorohexylthio)valerate, isopropyl 4-methyl-2-(5,5,6,6,6-pentafluorohexylthio)valerate, butyl 4-methyl-2-(5,5,6,6,6-pentafluorohexylthio)valerate, isobutyl 4-methyl-2-(5,5,6,6,6-pentafluorohexylthio)valerate, tert-butyl 4-methyl-2-(5,5,6,6,6-pentafluorohexylthio)valerate, methyl 4-methyl-2-(4,4,5,6,6-pentafluorohexylthio)valerate, ethyl 4-methyl-2-(4,4,5,6,6-pentafluorohexylthio)valerate, propyl 4-methyl-2-(4,4,5,6,6-pentafluorohexylthio)valerate, isopropyl 4-methyl-2-(4,4,5,6,6-pentafluorohexylthio)valerate, butyl 4-methyl-2-(4,4,5,6,6-pentafluorohexylthio)valerate, isobutyl 4-methyl-2-(4,4,5,6,6-pentafluorohexylthio)valerate, tert-butyl 4-methyl-2-(4,4,5,6,6-pentafluorohexylthio)valerate, methyl 4-methyl-2-(4,4,5,5,6,6,6-heptafluorohexylthio)valerate, ethyl 4-methyl-2-(4,4,5,5,6,6,6-heptafluorohexylthio)valerate, propyl 4-methyl-2-(4,4,5,5,6,6,6-heptafluorohexylthio)valerate, isopropyl 4-methyl-2-(4,4,5,5,6,6,6-heptafluorohexylthio)valerate, butyl 4-methyl-2-(4,4,5,5,6,6,6-heptafluorohexylthio)valerate, isobutyl 4-methyl-2-(4,4,5,5,6,6,6-heptafluorohexylthio)valerate, tert-butyl 4-methyl-2-(4,4,5,5,6,6,6-heptafluorohexylthio)valerate, methyl 4-methyl-2-(4-fluoroheptylthio)valerate, ethyl 4-methyl-2-(4-fluoroheptylthio)valerate, propyl 4-methyl-2-(4-fluoroheptylthio)valerate, isopropyl 4-methyl-2-(4-fluoroheptylthio)valerate, butyl 4-methyl-2-(4-fluoroheptylthio)valerate, isobutyl 4-methyl-2-(4-fluoroheptylthio)valerate, tert-butyl 4-methyl-2-(4-fluoroheptylthio)valerate, methyl 4-methyl-2-(4,4-difluoroheptylthio)valerate, ethyl 4-methyl-2-(4,4-difluoroheptylthio)valerate, propyl 4-methyl-2-(4,4-difluoroheptylthio)valerate, isopropyl 4-methyl-2-(4,4-difluoroheptylthio)valerate, butyl 4-methyl-2-(4,4-difluoroheptylthio)valerate, isobutyl 4-methyl-2-(4,4-difluoroheptylthio)valerate, tert-butyl 4-methyl-2-(4,4-difluoroheptylthio)valerate, methyl 4-methyl-2-(5-fluoroheptylthio)valerate, ethyl 4-methyl-2-(5-fluoroheptylthio)valerate, propyl 4-methyl-2-(5-fluoroheptylthio)valerate, isopropyl 4-methyl-2-(5-fluoroheptylthio)valerate, butyl 4-methyl-2-(5-fluoroheptylthio)valerate, isobutyl 4-methyl-2-(5-fluoroheptylthio)valerate, tert-butyl 4-methyl-2-(5-fluoroheptylthio)valerate, methyl 4-methyl-2-(5,5-difluoroheptylthio)valerate, ethyl 4-methyl-2-(5,5-difluoroheptylthio)valerate, propyl 4-methyl-2-(5,5-difluoroheptylthio)valerate, isopropyl 4-methyl-2-(5,5-difluoroheptylthio)valerate, butyl 4-methyl-2-(5,5-difluoroheptylthio)valerate, isobutyl 4-methyl-2-(5,5-difluoroheptylthio)valerate, tert-butyl 4-methyl-2-(5,5-difluoroheptylthio)valerate, methyl 4-methyl-2-(6-fluoroheptylthio)valerate, ethyl 4-methyl-2-(6-fluoroheptylthio)valerate, propyl 4-methyl-2-(6-fluoroheptylthio)valerate, isopropyl 4-methyl-2-(6-fluoroheptylthio)valerate, butyl 4-methyl-2-(6-fluoroheptylthio)valerate, isobutyl 4-methyl-2-(6-fluoroheptylthio)valerate, tert-butyl 4-methyl-2-(6-fluoroheptylthio)valerate, methyl 4-methyl-2-(6,6-difluoroheptylthio)valerate, ethyl 4-methyl-2-(6,6-difluoroheptylthio)valerate, propyl 4-methyl-2-(6,6-difluoroheptylthio)valerate, isopropyl 4-methyl-2-(6,6-difluoroheptylthio)valerate, butyl 4-methyl-2-(6,6-difluoroheptylthio)valerate, isobutyl 4-methyl-2-(6,6-difluoroheptylthio)valerate, tert-butyl 4-methyl-2-(6,6-difluoroheptylthio)valerate, methyl 4-methyl-2-(7-fluoroheptylthio)valerate, ethyl 4-methyl-2-(7-fluoroheptylthio)valerate, propyl 4-methyl-2-(7-fluoroheptylthio)valerate, isopropyl 4-methyl-2-(7-fluoroheptylthio)valerate, butyl 4-methyl-2-(7-fluoroheptylthio)valerate, isobutyl 4-methyl-2-(7-fluoroheptylthio)valerate, tert-butyl 4-methyl-2-(7-fluoroheptylthio)valerate, methyl 4-methyl-2-(7,7-difluoroheptylthio)valerate, ethyl 4-methyl-2-(7,7-difluoroheptylthio)valerate, propyl 4-methyl-2-(7,7-difluoroheptylthio)valerate, isopropyl 4-methyl-2-(7,7-difluoroheptylthio)valerate, butyl 4-methyl-2-(7,7-difluoroheptylthio)valerate, isobutyl 4-methyl-2-(7,7-difluoroheptylthio)valerate, tert-butyl 4-methyl-2-(7,7-difluoroheptylthio)valerate, methyl 4-methyl-2-(7,7,7-trifluoroheptylthio)valerate, ethyl 4-methyl-2-(7,7,7-trifluoroheptylthio)valerate, propyl 4-methyl-2-(7,7,7-trifluoroheptylthio)valerate, isopropyl 4-methyl-2-(7,7,7-trifluoroheptylthio)valerate, butyl 4-methyl-2-(7,7,7-trifluoroheptylthio)valerate, isobutyl 4-methyl-2-(7,7,7-trifluoroheptylthio)valerate, tert-butyl 4-methyl-2-(7,7,7-trifluoroheptylthio)valerate, methyl 4-methyl-2-(6,6,7,7,7-pentafluoroheptylthio)valerate, ethyl 4-methyl-2-(6,6,7,7,7-pentafluoroheptylthio)valerate, propyl 4-methyl-2-(6,6,7,7,7-pentafluoroheptylthio)valerate, isopropyl 4-methyl-2-(6,6,7,7,7-pentafluoroheptylthio)valerate, butyl 4-methyl-2-(6,6,7,7,7-pentafluoroheptylthio)valerate, isobutyl 4-methyl-2-(6,6,7,7,7-pentafluoroheptylthio)valerate, tert-butyl 4-methyl-2-(6,6,7,7,7-pentafluoroheptylthio)valerate, methyl 4-methyl-2-(5,5,6,6,7,7,7-heptafluoroheptylthio)valerate, ethyl 4-methyl-2-(5,5,6,6,7,7,7-heptafluoroheptylthio)valerate, propyl 4-methyl-2-(5,5,6,6,7,7,7-heptafluoroheptylthio)valerate, isopropyl 4-methyl-2-(5,5,6,6,7,7,7-heptafluoroheptylthio)valerate, butyl 4-methyl-2-(5,5,6,6,7,7,7-heptafluoroheptylthio)valerate, isobutyl 4-methyl-2-(5,5,6,6,7,7,7-heptafluoroheptylthio)valerate, tert-butyl 4-methyl-2-(5,5,6,6,7,7,7-heptafluoroheptylthio)valerate, methyl 4-methyl-2-(4,4,5,5,6,6,7,7,7-nonafluoroheptylthio)valerate, ethyl 4-methyl-2-(4,4,5,5,6,6,7,7,7-nonafluoroheptylthio)valerate, propyl 4-methyl-2-(4,4,5,5,6,6,7,7,7-nonafluoroheptylthio)valerate, isopropyl 4-methyl-2-(4,4,5,5,6,6,7,7,7-nonafluoroheptylthio)valerate, butyl 4-methyl-2-(4,4,5,5,6,6,7,7,7-nonafluoroheptylthio)valerate, isobutyl 4-methyl-2-(4,4,5,5,6,6,7,7,7-nonafluoroheptylthio)valerate, tert-butyl 4-methyl-2-(4,4,5,5,6,6,7,7,7-nonafluoroheptylthio)valerate, methyl 2-(3-fluoropropylthio)caproate, ethyl 2-(3-fluoropropylthio)caproate, propyl 2-(3-fluoropropylthio)caproate, isopropyl 2-(3-fluoropropylthio)caproate, butyl 2-(3-fluoropropylthio)caproate, isobutyl 2-(3-fluoropropylthio)caproate, tert-butyl 2-(3-fluoropropylthio)caproate, methyl 2-(3,3-difluoropropylthio)caproate, ethyl 2-(3,3-difluoropropylthio)caproate, propyl 2-(3,3-difluoropropylthio)caproate, isopropyl 2-(3,3-difluoropropylthio)caproate, butyl 2-(3,3-difluoropropylthio)caproate, isobutyl 2-(3,3-difluoropropylthio)caproate, tert-butyl 2-(3,3-difluoropropylthio)caproate, methyl 2-(3,3,3-trifluoropropylthio)caproate, ethyl 2-(3,3,3-trifluoropropylthio)caproate, propyl 2-(3,3,3-trifluoropropylthio)caproate, isopropyl 2-(3,3,3-trifluoropropylthio)caproate, butyl 2-(3,3,3-trifluoropropylthio)caproate, isobutyl 2-(3,3,3-trifluoropropylthio)caproate, tert-butyl 2-(3,3,3-trifluoropropylthio)caproate, methyl 2-(4-fluorobutylthio)caproate, ethyl 2-(4-fluorobutylthio)caproate, propyl 2-(4-fluorobutylthio)caproate, isopropyl 2-(4-fluorobutylthio)caproate, butyl 2-(4-fluorobutylthio)caproate, isobutyl 2-(4-fluorobutylthio)caproate, tert-butyl 2-(4-fluorobutylthio)caproate, methyl 2-(4,4,4-trifluorobutylthio)caproate, ethyl 2-(4,4,4-trifluorobutylthio)caproate, propyl 2-(4,4,4-trifluorobutylthio)caproate, isopropyl 2-(4,4,4-trifluorobutylthio)caproate, butyl 2-(4,4,4-trifluorobutylthio)caproate, isobutyl 2-(4,4,4-trifluorobutylthio)caproate, tert-butyl 2-(4,4,4-trifluorobutylthio)caproate, methyl 2-(3,3,4,4,4-pentafluorobutylthio)caproate, ethyl 2-(3,3,4,4,4-pentafluorobutylthio)caproate, propyl 2-(3,3,4,4,4-pentafluorobutylthio)caproate, isopropyl 2-(3,3,4,4,4-pentafluorobutylthio)caproate, butyl 2-(3,3,4,4,4-pentafluorobutylthio)caproate, isobutyl 2-(3,3,4,4,4-pentafluorobutylthio)caproate, tert-butyl 2-(3,3,4,4,4-pentafluorobutylthio)caproate, methyl 2-(4-fluoropentylthio)caproate, ethyl 2-(4-fluoropentylthio)caproate, propyl 2-(4-fluoropentylthio)caproate, isopropyl 2-(4-fluoropentylthio)caproate, butyl 2-(4-fluoropentylthio)caproate, isobutyl 2-(4-fluoropentylthio)caproate, tert-butyl 2-(4-fluoropentylthio)caproate, methyl 2-(4,4-difluoropentylthio)caproate, ethyl 2-(4,4-difluoropentylthio)caproate, propyl 2-(4,4-difluoropentylthio)caproate, isopropyl 2-(4,4-difluoropentylthio)caproate, butyl 2-(4,4-difluoropentylthio)caproate, isobutyl 2-(4,4-difluoropentylthio)caproate, tert-butyl 2-(4,4-difluoropentylthio)caproate, methyl 2-(5-fluoropentylthio)caproate, ethyl 2-(5-fluoropentylthio)caproate, propyl 2-(5-fluoropentylthio)caproate, isopropyl 2-(5-fluoropentylthio)caproate, butyl 2-(5-fluoropentylthio)caproate, isobutyl 2-(5-fluoropentylthio)caproate, tert-butyl 2-(5-fluoropentylthio)caproate, methyl 2-(5,5-difluoropentylthio)caproate, ethyl 2-(5,5-difluoropentylthio)caproate, propyl 2-(5,5-difluoropentylthio)caproate, isopropyl 2-(5,5-difluoropentylthio)caproate, butyl 2-(5,5-difluoropentylthio)caproate, isobutyl 2-(5,5-difluoropentylthio)caproate, tert-butyl 2-(5,5-difluoropentylthio)caproate, methyl 2-(5,5,5-trifluoropentylthio)caproate, ethyl 2-(5,5,5-trifluoropentylthio)caproate, propyl 2-(5,5,5-trifluoropentylthio)caproate, isopropyl 2-(5,5,5-trifluoropentylthio)caproate, butyl 2-(5,5,5-trifluoropentylthio)caproate, isobutyl 2-(5,5,5-trifluoropentylthio)caproate, tert-butyl 2-(5,5,5-trifluoropentylthio)caproate, methyl 2-(4,4,5,5-tetrafluoropentylthio)caproate, ethyl 2-(4,4,5,5-tetrafluoropentylthio)caproate, propyl 2-(4,4,5,5-tetrafluoropentylthio)caproate, isopropyl 2-(4,4,5,5-tetrafluoropentylthio)caproate, butyl 2-(4,4,5,5-tetrafluoropentylthio)caproate, isobutyl 2-(4,4,5,5-tetrafluoropentylthio)caproate, tert-butyl 2-(4,4,5,5-tetrafluoropentylthio)caproate, methyl 2-(4,4,5,5,5-pentafluoropentylthio)caproate, ethyl 2-(4,4,5,5,5-pentafluoropentylthio)caproate, propyl 2-(4,4,5,5,5-pentafluoropentylthio)caproate, isopropyl 2-(4,4,5,5,5-pentafluoropentylthio)caproate, butyl 2-(4,4,5,5,5-pentafluoropentylthio)caproate, isobutyl 2-(4,4,5,5,5-pentafluoropentylthio)caproate, tert-butyl 2-(4,4,5,5,5-pentafluoropentylthio)caproate, methyl 2-(4,4,4-trifluoro-3-trifluoromethylthio)caproate, ethyl 2-(4,4,4-trifluoro-3-trifluoromethylthio)caproate, propyl 2-(4,4,4-trifluoro-3-trifluoromethylthio)caproate, isopropyl 2-(4,4,4-trifluoro-3-trifluoromethylthio)caproate, butyl 2-(4,4,4-trifluoro-3-trifluoromethylthio)caproate, isobutyl 2-(4,4,4-trifluoro-3-trifluoromethylthio)caproate, tert-butyl 2-(4,4,4-trifluoro-3-trifluoromethylthio)caproate, methyl 2-(4-fluorohexylthio)caproate, ethyl 2-(4-fluorohexylthio)caproate, propyl 2-(4-fluorohexylthio)caproate, isopropyl 2-(4-fluorohexylthio)caproate, butyl 2-(4-fluorohexylthio)caproate, isobutyl 2-(4-fluorohexylthio)caproate, tert-butyl 2-(4-fluorohexylthio)caproate, methyl 2-(4,4-difluorohexylthio)caproate, ethyl 2-(4,4-difluorohexylthio)caproate, propyl 2-(4,4-difluorohexylthio)caproate, isopropyl 2-(4,4-difluorohexylthio)caproate, butyl 2-(4,4-difluorohexylthio)caproate, isobutyl 2-(4,4-difluorohexylthio)caproate, tert-butyl 2-(4,4-difluorohexylthio)caproate, methyl 2-(5-fluorohexylthio)caproate, ethyl 2-(5-fluorohexylthio)caproate, propyl 2-(5-fluorohexylthio)caproate, isopropyl 2-(5-fluorohexylthio)caproate, butyl 2-(5-fluorohexylthio)caproate, isobutyl 2-(5-fluorohexylthio)caproate, tert-butyl 2-(5-fluorohexylthio)caproate, methyl 2-(5,5-difluorohexylthio)caproate, ethyl 2-(5,5-difluorohexylthio)caproate, propyl 2-(5,5-difluorohexylthio)caproate, isopropyl 2-(5,5-difluorohexylthio)caproate, butyl 2-(5,5-difluorohexylthio)caproate, isobutyl 2-(5,5-difluorohexylthio)caproate, tert-butyl 2-(5,5-difluorohexylthio)caproate, methyl 2-(6-fluorohexylthio)caproate, ethyl 2-(6-fluorohexylthio)caproate, propyl 2-(6-fluorohexylthio)caproate, isopropyl 2-(6-fluorohexylthio)caproate, butyl 2-(6-fluorohexylthio)caproate, isobutyl 2-(6-fluorohexylthio)caproate, tert-butyl 2-(6-fluorohexylthio)caproate, methyl 2-(6,6-difluorohexylthio)caproate, ethyl 2-(6,6-difluorohexylthio)caproate, propyl 2-(6,6-difluorohexylthio)caproate, isopropyl 2-(6,6-difluorohexylthio)caproate, butyl 2-(6,6-difluorohexylthio)caproate, isobutyl 2-(6,6-difluorohexylthio)caproate, tert-butyl 2-(6,6-difluorohexylthio)caproate, methyl 2-(6,6,6-trifluorohexylthio)caproate, ethyl 2-(6,6,6-trifluorohexylthio)caproate, propyl 2-(6,6,6-trifluorohexylthio)caproate, isopropyl 2-(6,6,6-trifluorohexylthio)caproate, butyl 2-(6,6,6-trifluorohexylthio)caproate, isobutyl 2-(6,6,6-trifluorohexylthio)caproate, tert-butyl 2-(6,6,6-trifluorohexylthio)caproate, methyl 2-(5,5,6,6,6-pentafluorohexylthio)caproate, ethyl 2-(5,5,6,6,6-pentafluorohexylthio)caproate, propyl 2-(5,5,6,6,6-pentafluorohexylthio)caproate, isopropyl 2-(5,5,6,6,6-pentafluorohexylthio)caproate, butyl 2-(5,5,6,6,6-pentafluorohexylthio)caproate, isobutyl 2-(5,5,6,6,6-pentafluorohexylthio)caproate, tert-butyl 2-(5,5,6,6,6-pentafluorohexylthio)caproate, methyl 2-(4,4,5,6,6-pentafluorohexylthio)caproate, ethyl 2-(4,4,5,6,6-pentafluorohexylthio)caproate, propyl 2-(4,4,5,6,6-pentafluorohexylthio)caproate, isopropyl 2-(4,4,5,6,6-pentafluorohexylthio)caproate, butyl 2-(4,4,5,6,6-pentafluorohexylthio)caproate, isobutyl 2-(4,4,5,6,6-pentafluorohexylthio)caproate, tert-butyl 2-(4,4,5,6,6-pentafluorohexylthio)caproate, methyl 2-(4,4,5,5,6,6,6-heptafluorohexylthio)caproate, ethyl 2-(4,4,5,5,6,6,6-heptafluorohexylthio)caproate, propyl 2-(4,4,5,5,6,6,6-heptafluorohexylthio)caproate, isopropyl 2-(4,4,5,5,6,6,6-heptafluorohexylthio)caproate, butyl 2-(4,4,5,5,6,6,6-heptafluorohexylthio)caproate, isobutyl 2-(4,4,5,5,6,6,6-heptafluorohexylthio)caproate, tert-butyl 2-(4,4,5,5,6,6,6-heptafluorohexylthio)caproate, methyl 2-(4-fluoroheptylthio)caproate, ethyl 2-(4-fluoroheptylthio)caproate, propyl 2-(4-fluoroheptylthio)caproate, isopropyl 2-(4-fluoroheptylthio)caproate, butyl 2-(4-fluoroheptylthio)caproate, isobutyl 2-(4-fluoroheptylthio)caproate, tert-butyl 2-(4-fluoroheptylthio)caproate, methyl 2-(4,4-difluoroheptylthio)caproate, ethyl 2-(4,4-difluoroheptylthio)caproate, propyl 2-(4,4-difluoroheptylthio)caproate, isopropyl 2-(4,4-difluoroheptylthio)caproate, butyl 2-(4,4-difluoroheptylthio)caproate, isobutyl 2-(4,4-difluoroheptylthio)caproate, tert-butyl 2-(4,4-difluoroheptylthio)caproate, methyl 2-(5-fluoroheptylthio)caproate, ethyl 2-(5-fluoroheptylthio)caproate, propyl 2-(5-fluoroheptylthio)caproate, isopropyl 2-(5-fluoroheptylthio)caproate, butyl 2-(5-fluoroheptylthio)caproate, isobutyl 2-(5-fluoroheptylthio)caproate, tert-butyl 2-(5-fluoroheptylthio)caproate, methyl 2-(5,5-difluoroheptylthio)caproate, ethyl 2-(5,5-difluoroheptylthio)caproate, propyl 2-(5,5-difluoroheptylthio)caproate, isopropyl 2-(5,5-difluoroheptylthio)caproate, butyl 2-(5,5-difluoroheptylthio)caproate, isobutyl 2-(5,5-difluoroheptylthio)caproate, tert-butyl 2-(5,5-difluoroheptylthio)caproate, methyl 2-(6-fluoroheptylthio)caproate, ethyl 2-(6-fluoroheptylthio)caproate, propyl 2-(6-fluoroheptylthio)caproate, isopropyl 2-(6-fluoroheptylthio)caproate, butyl 2-(6-fluoroheptylthio)caproate, isobutyl 2-(6-fluoroheptylthio)caproate, tert-butyl 2-(6-fluoroheptylthio)caproate, methyl 2-(6,6-difluoroheptylthio)caproate, ethyl 2-(6,6-difluoroheptylthio)caproate, propyl 2-(6,6-difluoroheptylthio)caproate, isopropyl 2-(6,6-difluoroheptylthio)caproate, butyl 2-(6,6-difluoroheptylthio)caproate, isobutyl 2-(6,6-difluoroheptylthio)caproate, tert-butyl 2-(6,6-difluoroheptylthio)caproate, methyl 2-(7-fluoroheptylthio)caproate, ethyl 2-(7-fluoroheptylthio)caproate, propyl 2-(7-fluoroheptylthio)caproate, isopropyl 2-(7-fluoroheptylthio)caproate, butyl 2-(7-fluoroheptylthio)caproate, isobutyl 2-(7-fluoroheptylthio)caproate, tert-butyl 2-(7-fluoroheptylthio)caproate, methyl 2-(7,7-difluoroheptylthio)caproate, ethyl 2-(7,7-difluoroheptylthio)caproate, propyl 2-(7,7-difluoroheptylthio)caproate, isopropyl 2-(7,7-difluoroheptylthio)caproate, butyl 2-(7,7-difluoroheptylthio)caproate, isobutyl 2-(7,7-difluoroheptylthio)caproate, tert-butyl 2-(7,7-difluoroheptylthio)caproate, methyl 2-(7,7,7-trifluoroheptylthio)caproate, ethyl 2-(7,7,7-trifluoroheptylthio)caproate, propyl 2-(7,7,7-trifluoroheptylthio)caproate, isopropyl 2-(7,7,7-trifluoroheptylthio)caproate, butyl 2-(7,7,7-trifluoroheptylthio)caproate, isobutyl 2-(7,7,7-trifluoroheptylthio)caproate, tert-butyl 2-(7,7,7-trifluoroheptylthio)caproate, methyl 2-(6,6,7,7,7-pentafluoroheptylthio)caproate, ethyl 2-(6,6,7,7,7-pentafluoroheptylthio)caproate, propyl 2-(6,6,7,7,7-pentafluoroheptylthio)caproate, isopropyl 2-(6,6,7,7,7-pentafluoroheptylthio)caproate, butyl 2-(6,6,7,7,7-pentafluoroheptylthio)caproate, isobutyl 2-(6,6,7,7,7-pentafluoroheptylthio)caproate, tert-butyl 2-(6,6,7,7,7-pentafluoroheptylthio)caproate, methyl 2-(5,5,6,6,7,7,7-heptafluoroheptylthio)caproate, ethyl 2-(5,5,6,6,7,7,7-heptafluoroheptylthio)caproate, propyl 2-(5,5,6,6,7,7,7-heptafluoroheptylthio)caproate, isopropyl 2-(5,5,6,6,7,7,7-heptafluoroheptylthio)caproate, butyl 2-(5,5,6,6,7,7,7-heptafluoroheptylthio)caproate, isobutyl 2-(5,5,6,6,7,7,7-heptafluoroheptylthio)caproate, tert-butyl 2-(5,5,6,6,7,7,7-heptafluoroheptylthio)caproate, methyl 2-(4,4,5,5,6,6,7,7,7-nonafluoroheptylthio)caproate, ethyl 2-(4,4,5,5,6,6,7,7,7-nonafluoroheptylthio)caproate, propyl 2-(4,4,5,5,6,6,7,7,7-nonafluoroheptylthio)caproate, isopropyl 2-(4,4,5,5,6,6,7,7,7-nonafluoroheptylthio)caproate, butyl 2-(4,4,5,5,6,6,7,7,7-nonafluoroheptylthio)caproate, isobutyl 2-(4,4,5,5,6,6,7,7,7-nonafluoroheptylthio)caproate, tert-butyl 2-(4,4,5,5,6,6,7,7,7-nonafluoroheptylthio)caproate, methyl 2-(3-fluoropropylthio)enanthate, ethyl 2-(3-fluoropropylthio)enanthate, propyl 2-(3-fluoropropylthio)enanthate, isopropyl 2-(3-fluoropropylthio)enanthate, butyl 2-(3-fluoropropylthio)enanthate, isobutyl 2-(3-fluoropropylthio)enanthate, tert-butyl 2-(3-fluoropropylthio)enanthate, methyl 2-(3,3-difluoropropylthio)enanthate, ethyl 2-(3,3-difluoropropylthio)enanthate, propyl 2-(3,3-difluoropropylthio)enanthate, isopropyl 2-(3,3-difluoropropylthio)enanthate, butyl 2-(3,3-difluoropropylthio)enanthate, isobutyl 2-(3,3-difluoropropylthio)enanthate, tert-butyl 2-(3,3-difluoropropylthio)enanthate, methyl 2-(3,3,3-trifluoropropylthio)enanthate, ethyl 2-(3,3,3-trifluoropropylthio)enanthate, propyl 2-(3,3,3-trifluoropropylthio)enanthate, isopropyl 2-(3,3,3-trifluoropropylthio)enanthate, butyl 2-(3,3,3-trifluoropropylthio)enanthate, isobutyl 2-(3,3,3-trifluoropropylthio)enanthate, tert-butyl 2-(3,3,3-trifluoropropylthio)enanthate, methyl 2-(4-fluorobutylthio)enanthate, ethyl 2-(4-fluorobutylthio)enanthate, propyl 2-(4-fluorobutylthio)enanthate, isopropyl 2-(4-fluorobutylthio)enanthate, butyl 2-(4-fluorobutylthio)enanthate, isobutyl 2-(4-fluorobutylthio)enanthate, tert-butyl 2-(4-fluorobutylthio)enanthate, methyl 2-(4,4,4-trifluorobutylthio)enanthate, ethyl 2-(4,4,4-trifluorobutylthio)enanthate, propyl 2-(4,4,4-trifluorobutylthio)enanthate, isopropyl 2-(4,4,4-trifluorobutylthio)enanthate, butyl 2-(4,4,4-trifluorobutylthio)enanthate, isobutyl 2-(4,4,4-trifluorobutylthio)enanthate, tert-butyl 2-(4,4,4-trifluorobutylthio)enanthate, methyl 2-(3,3,4,4,4-pentafluorobutylthio)enanthate, ethyl 2-(3,3,4,4,4-pentafluorobutylthio)enanthate, propyl 2-(3,3,4,4,4-pentafluorobutylthio)enanthate, isopropyl 2-(3,3,4,4,4-pentafluorobutylthio)enanthate, butyl 2-(3,3,4,4,4-pentafluorobutylthio)enanthate, isobutyl 2-(3,3,4,4,4-pentafluorobutylthio)enanthate, tert-butyl 2-(3,3,4,4,4-pentafluorobutylthio)enanthate, methyl 2-(4-fluoropentylthio)enanthate, ethyl 2-(4-fluoropentylthio)enanthate, propyl 2-(4-fluoropentylthio)enanthate, isopropyl 2-(4-fluoropentylthio)enanthate, butyl 2-(4-fluoropentylthio)enanthate, isobutyl 2-(4-fluoropentylthio)enanthate, tert-butyl 2-(4-fluoropentylthio)enanthate, methyl 2-(4,4-difluoropentylthio)enanthate, ethyl 2-(4,4-difluoropentylthio)enanthate, propyl 2-(4,4-difluoropentylthio)enanthate, isopropyl 2-(4,4-difluoropentylthio)enanthate, butyl 2-(4,4-difluoropentylthio)enanthate, isobutyl 2-(4;4-difluoropentylthio)enanthate, tert-butyl 2-(4,4-difluoropentylthio)enanthate, methyl 2-(5-fluoropentylthio)enanthate, ethyl 2-(5-fluoropentylthio)enanthate, propyl 2-(5-fluoropentylthio)enanthate, isopropyl 2-(5-fluoropentylthio)enanthate, butyl 2-(5-fluoropentylthio)enanthate, isobutyl 2-(5-fluoropentylthio)enanthate, tert-butyl 2-(5-fluoropentylthio)enanthate, methyl 2-(5,5-difluoropentylthio)enanthate, ethyl 2-(5,5-difluoropentylthio)enanthate, propyl 2-(5,5-difluoropentylthio)enanthate, isopropyl 2-(5,5-difluoropentylthio)enanthate, butyl 2-(5,5-difluoropentylthio)enanthate, isobutyl 2-(5,5-difluoropentylthio)enanthate, tert-butyl 2-(5,5-difluoropentylthio)enanthate, methyl 2-(5,5,5-trifluoropentylthio)enanthate, ethyl 2-(5,5,5-trifluoropentylthio)enanthate, propyl 2-(5,5,5-trifluoropentylthio)enanthate, isopropyl 2-(5,5,5-trifluoropentylthio)enanthate, butyl 2-(5,5,5-trifluoropentylthio)enanthate, isobutyl 2-(5,5,5-trifluoropentylthio)enanthate, tert-butyl 2-(5,5,5-trifluoropentylthio)enanthate, methyl 2-(4,4,5,5-tetrafluoropentylthio)enanthate, ethyl 2-(4,4,5,5-tetrafluoropentylthio)enanthate, propyl 2-(4,4,5,5-tetrafluoropentylthio)enanthate, isopropyl 2-(4,4,5,5-tetrafluoropentylthio)enanthate, butyl 2-(4,4,5,5-tetrafluoropentylthio)enanthate, isobutyl 2-(4,4,5,5-tetrafluoropentylthio)enanthate, tert-butyl 2-(4,4,5,5-tetrafluoropentylthio)enanthate, methyl 2-(4,4,5,5,5-pentafluoropentylthio)enanthate, ethyl 2-(4,4,5,5,5-pentafluoropentylthio)enanthate, propyl 2-(4,4,5,5,5-pentafluoropentylthio)enanthate, isopropyl 2-(4,4,5,5,5-pentafluoropentylthio)enanthate, butyl 2-(4,4,5,5,5-pentafluoropentylthio)enanthate, isobutyl 2-(4,4,5,5,5-pentafluoropentylthio)enanthate, tert-butyl 2-(4,4,5,5,5-pentafluoropentylthio)enanthate, methyl 2-(4,4,4-trifluoro-3-trifluoromethylthio)enanthate, ethyl 2-(4,4,4-trifluoro-3-trifluoromethylthio)enanthate, propyl 2-(4,4,4-trifluoro-3-trifluoromethylthio)enanthate, isopropyl 2-(4,4,4-trifluoro-3-trifluoromethylthio)enanthate, butyl 2-(4,4,4-trifluoro-3-trifluoromethylthio)enanthate, isobutyl 2-(4,4,4-trifluoro-3-trifluoromethylthio)enanthate, tert-butyl 2-(4,4,4-trifluoro-3-trifluoromethylthio)enanthate, methyl 2-(4-fluorohexylthio)enanthate, ethyl 2-(4-fluorohexylthio)enanthate, propyl 2-(4-fluorohexylthio)enanthate, isopropyl 2-(4-fluorohexylthio)enanthate, butyl 2-(4-fluorohexylthio)enanthate, isobutyl 2-(4-fluorohexylthio)enanthate, tert-butyl 2-(4-fluorohexylthio)enanthate, methyl 2-(4,4-difluorohexylthio)enanthate, ethyl 2-(4,4-difluorohexylthio)enanthate, propyl 2-(4,4-difluorohexylthio)enanthate, 2-(4,4-difluorohexylthio)enanthate isopropyl, butyl 2-(4,4-difluorohexylthio)enanthate, isobutyl 2-(4,4-difluorohexylthio)enanthate, tert-butyl 2-(4,4-difluorohexylthio)enanthate, methyl 2-(5-fluorohexylthio)enanthate, ethyl 2-(5-fluorohexylthio)enanthate, propyl 2-(5-fluorohexylthio)enanthate, isopropyl 2-(5-fluorohexylthio)enanthate, butyl 2-(5-fluorohexylthio)enanthate, isobutyl 2-(5-fluorohexylthio)enanthate, tert-butyl 2-(5-fluorohexylthio)enanthate, methyl 2-(5,5-difluorohexylthio)enanthate, ethyl 2-(5,5-difluorohexylthio)enanthate, propyl 2-(5,5-difluorohexylthio)enanthate, isopropyl 2-(5,5-difluorohexylthio)enanthate, butyl 2-(5,5-difluorohexylthio)enanthate, isobutyl 2-(5,5-difluorohexylthio)enanthate, tert-butyl 2-(5,5-difluorohexylthio)enanthate, methyl 2-(6-fluorohexylthio)enanthate, ethyl 2-(6-fluorohexylthio)enanthate, propyl 2-(6-fluorohexylthio)enanthate, isopropyl 2-(6-fluorohexylthio)enanthate, butyl 2-(6-fluorohexylthio)enanthate, isobutyl 2-(6-fluorohexylthio)enanthate, tert-butyl 2-(6-fluorohexylthio)enanthate, methyl 2-(6,6-difluorohexylthio)enanthate, ethyl 2-(6,6-difluorohexylthio)enanthate, propyl 2-(6,6-difluorohexylthio)enanthate, isopropyl 2-(6,6-difluorohexylthio)enanthate, butyl 2-(6,6-difluorohexylthio)enanthate, isobutyl 2-(6,6-difluorohexylthio)enanthate, tert-butyl 2-(6,6-difluorohexylthio)enanthate, methyl 2-(6,6,6-trifluorohexylthio)enanthate, ethyl 2-(6,6,6-trifluorohexylthio)enanthate, propyl 2-(6,6,6-trifluorohexylthio)enanthate, isopropyl 2-(6,6,6-trifluorohexylthio)enanthate, butyl 2-(6,6,6-trifluorohexylthio)enanthate, isobutyl 2-(6,6,6-trifluorohexylthio)enanthate, tert-butyl 2-(6,6,6-trifluorohexylthio)enanthate, methyl 2-(5,5,6,6,6-pentafluorohexylthio)enanthate, ethyl 2-(5,5,6,6,6-pentafluorohexylthio)enanthate, propyl 2-(5,5,6,6,6-pentafluorohexylthio)enanthate, isopropyl 2-(5,5,6,6,6-pentafluorohexylthio)enanthate, butyl 2-(5,5,6,6,6-pentafluorohexylthio)enanthate, isobutyl 2-(5,5,6,6,6-pentafluorohexylthio)enanthate, tert-butyl 2-(5,5,6,6,6-pentafluorohexylthio)enanthate, methyl 2-(4,4,5,6,6-pentafluorohexylthio)enanthate, ethyl 2-(4,4,5,6,6-pentafluorohexylthio)enanthate, propyl 2-(4,4,5,6,6-pentafluorohexylthio)enanthate, isopropyl 2-(4,4,5,6,6-pentafluorohexylthio)enanthate, butyl 2-(4,4,5,6,6-pentafluorohexylthio)enanthate, isobutyl 2-(4,4,5,6,6-pentafluorohexylthio)enanthate, tert-butyl 2-(4,4,5,6,6-pentafluorohexylthio)enanthate, methyl 2-(4,4,5,5,6,6,6-heptafluorohexylthio)enanthate, ethyl 2-(4,4,5,5,6,6,6-heptafluorohexylthio)enanthate, propyl 2-(4,4,5,5,6,6,6-heptafluorohexylthio)enanthate, isopropyl 2-(4,4,5,5,6,6,6-heptafluorohexylthio)enanthate, butyl 2-(4,4,5,5,6,6,6-heptafluorohexylthio)enanthate, isobutyl 2-(4,4,5,5,6,6,6-heptafluorohexylthio)enanthate, tert-butyl 2-(4,4,5,5,6,6,6-heptafluorohexylthio)enanthate, methyl 2-(4-fluoroheptylthio)enanthate, ethyl 2-(4-fluoroheptylthio)enanthate, propyl 2-(4-fluoroheptylthio)enanthate, isopropyl 2-(4-fluoroheptylthio)enanthate, butyl 2-(4-fluoroheptylthio)enanthate, isobutyl 2-(4-fluoroheptylthio)enanthate, tert-butyl 2-(4-fluoroheptylthio)enanthate, methyl 2-(4,4-difluoroheptylthio)enanthate, ethyl 2-(4,4-difluoroheptylthio)enanthate, propyl 2-(4,4-difluoroheptylthio)enanthate, isopropyl 2-(4,4-difluoroheptylthio)enanthate, butyl 2-(4,4-difluoroheptylthio)enanthate, isobutyl 2-(4,4-difluoroheptylthio)enanthate, tert-butyl 2-(4,4-difluoroheptylthio)enanthate, methyl 2-(5-fluoroheptylthio)enanthate, ethyl 2-(5-fluoroheptylthio)enanthate, propyl 2-(5-fluoroheptylthio)enanthate, isopropyl 2-(5-fluoroheptylthio)enanthate, butyl 2-(5-fluoroheptylthio)enanthate, isobutyl 2-(5-fluoroheptylthio)enanthate, tert-butyl 2-(5-fluoroheptylthio)enanthate, methyl 2-(5,5-difluoroheptylthio)enanthate, ethyl 2-(5,5-difluoroheptylthio)enanthate, propyl 2-(5,5-difluoroheptylthio)enanthate, isopropyl 2-(5,5-difluoroheptylthio)enanthate, butyl 2-(5,5-difluoroheptylthio)enanthate, isobutyl 2-(5,5-difluoroheptylthio)enanthate, tert-butyl 2-(5,5-difluoroheptylthio)enanthate, methyl 2-(6-fluoroheptylthio)enanthate, ethyl 2-(6-fluoroheptylthio)enanthate, propyl 2-(6-fluoroheptylthio)enanthate, isopropyl 2-(6-fluoroheptylthio)enanthate, butyl 2-(6-fluoroheptylthio)enanthate, isobutyl 2-(6-fluoroheptylthio)enanthate, tert-butyl 2-(6-fluoroheptylthio)enanthate, methyl 2-(6,6-difluoroheptylthio)enanthate, ethyl 2-(6,6-difluoroheptylthio)enanthate, propyl 2-(6,6-difluoroheptylthio)enanthate, isopropyl 2-(6,6-difluoroheptylthio)enanthate, butyl 2-(6,6-difluoroheptylthio)enanthate, isobutyl 2-(6,6-difluoroheptylthio)enanthate, tert-butyl 2-(6,6-difluoroheptylthio)enanthate, methyl 2-(7-fluoroheptylthio)enanthate, ethyl 2-(7-fluoroheptylthio)enanthate, propyl 2-(7-fluoroheptylthio)enanthate, isopropyl 2-(7-fluoroheptylthio)enanthate, butyl 2-(7-fluoroheptylthio)enanthate, isobutyl 2-(7-fluoroheptylthio)enanthate, tert-butyl 2-(7-fluoroheptylthio)enanthate, methyl 2-(7,7-difluoroheptylthio)enanthate, ethyl 2-(7,7-difluoroheptylthio)enanthate, propyl 2-(7,7-difluoroheptylthio)enanthate, isopropyl 2-(7,7-difluoroheptylthio)enanthate, butyl 2-(7,7-difluoroheptylthio)enanthate, isobutyl 2-(7,7-difluoroheptylthio)enanthate, tert-butyl 2-(7,7-difluoroheptylthio)enanthate, methyl 2-(7,7,7-trifluoroheptylthio)enanthate, ethyl 2-(7,7,7-trifluoroheptylthio)enanthate, propyl 2-(7,7,7-trifluoroheptylthio)enanthate, isopropyl 2-(7,7,7-trifluoroheptylthio)enanthate, butyl 2-(7,7,7-trifluoroheptylthio)enanthate, isobutyl 2-(7,7,7-trifluoroheptylthio)enanthate, tert-butyl 2-(7,7,7-trifluoroheptylthio)enanthate, methyl 2-(6,6,7,7,7-pentafluoroheptylthio)enanthate, ethyl 2-(6,6,7,7,7-pentafluoroheptylthio)enanthate, propyl 2-(6,6,7,7,7-pentafluoroheptylthio)enanthate, isopropyl 2-(6,6,7,7,7-pentafluoroheptylthio)enanthate, butyl 2-(6,6,7,7,7-pentafluoroheptylthio)enanthate, isobutyl 2-(6,6,7,7,7-pentafluoroheptylthio)enanthate, tert-butyl 2-(6,6,7,7,7-pentafluoroheptylthio)enanthate, methyl 2-(5,5,6,6,7,7,7-heptafluoroheptylthio)enanthate, ethyl 2-(5,5,6,6,7,7,7-heptafluoroheptylthio)enanthate, propyl 2-(5,5,6,6,7,7,7-heptafluoroheptylthio)enanthate, isopropyl 2-(5,5,6,6,7,7,7-heptafluoroheptylthio)enanthate, butyl 2-(5,5,6,6,7,7,7-heptafluoroheptylthio)enanthate, isobutyl 2-(5,5,6,6,7,7,7-heptafluoroheptylthio)enanthate, tert-butyl 2-(5,5,6,6,7,7,7-heptafluoroheptylthio)enanthate, methyl 2-(4,4,5,5,6,6,7,7,7-nonafluoroheptylthio)enanthate, ethyl 2-(4,4,5,5,6,6,7,7,7-nonafluoroheptylthio)enanthate, propyl 2-(4,4,5,5,6,6,7,7,7-nonafluoroheptylthio)enanthate, isopropyl 2-(4,4,5,5,6,6,7,7,7-nonafluoroheptylthio)enanthate, butyl 2-(4,4,5,5,6,6,7,7,7-nonafluoroheptylthio)enanthate, isobutyl 2-(4,4,5,5,6,6,7,7,7-nonafluoroheptylthio)enanthate and tert-butyl 2-(4,4,5,5,6,6,7,7,7-nonafluoroheptylthio)enanthate.

Examples of preferable ester (3) include methyl (3-fluoropropylthio)acetate, ethyl (3-fluoropropylthio)acetate, propyl 3-fluoropropylthio)thioacetate, isopropyl (3-fluoropropylthio)acetate, (3-fluoropropylthio)acetate butyl, isobutyl (3-fluoropropylthio)acetate, tert-butyl (3-fluoropropylthio)acetate, methyl (3,3-difluoropropylthio)acetate, ethyl (3,3-difluoropropylthio)acetate, propyl (3,3-difluoropropylthio)acetate, isopropyl (3,3-difluoropropylthio)acetate, butyl (3,3-difluoropropylthio)acetate, isobutyl (3,3-difluoropropylthio)acetate, tert-butyl (3,3-difluoropropylthio)acetate, methyl (3,3,3-trifluoropropylthio)acetate, ethyl (3,3,3-trifluoropropylthio)acetate, propyl (3,3,3-trifluoropropylthio)acetate, isopropyl (3,3,3-trifluoropropylthio)acetate, butyl (3,3,3-trifluoropropylthio)acetate, isobutyl (3,3,3-trifluoropropylthio)acetate and tert-butyl (3,3,3-trifluoropropylthio)acetate, and methyl (3,3,3-trifluoropropylthio)acetate is more preferable.

The obtained ester (3) can be lead to a nitrile compound (hereinafter abbreviated to a nitrile (7)) represented by the formula (7): 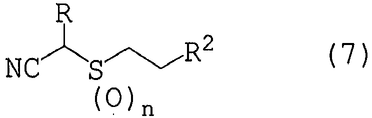
wherein R and R² have the same meanings as defined above, and n represents 1 or 2, as an intermediate for the synthesis of an organic sulfur compound, which exerts an excellent control effect against harmful arthropods.

The process of leading the ester (3) to the nitrile (7) will be described below.

The ester (3) may be extracted from the reaction mixture obtained by the reaction of the ester (1) with the olefin (2), or the reaction mixture containing the ester (3) may be used as it is.

Examples of the process of leading the ester (3) to the nitrile (7) include the following processes (A), (B) and (C):
(A) a process in which the ester (3) is oxidized to obtain an ester compound (hereinafter abbreviated to an ester (5)) represented by the formula (5): 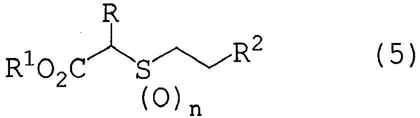
   wherein R, R¹ and R² have the same meanings as defined above, and n represents 1 or 2, and the obtained ester (5) is reacted with ammonia to obtain an amide compound (hereinafter abbreviated to an amide (6)) represented by the formula (6): 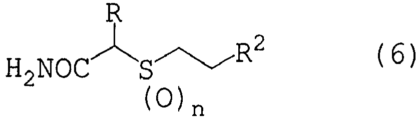
   wherein R, R² and n have the same meanings as defined above, and then a carbamoyl group (-CONH₂) of the obtained amide (6) is converted into a cyano group (-CN);
(B) a process in which the ester (3) is reacted with ammonia to obtain a (fluoroalkylthio)acetamide (hereinafter abbreviated to an amide (8)) represented by the formula (8) : 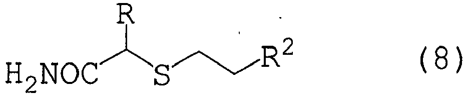 wherein R and R² have the same meanings as defined above and the obtained amide (8) is oxidized to obtain an amide (6), and then a carbamoyl group (-CONH₂) of the amide (6) is converted into a cyano group (-CN); and
(C) a process in which the ester (3) is reacted with ammonia to obtain an amide (8), a carbamoyl group (-CONH₂) of the obtained amide (8) is converted into a cyano group (-CN) to obtain a (fluoroalkylthio)acetonitrile (hereinafter abbreviated to a nitrile (9)) represented by the formula (9): 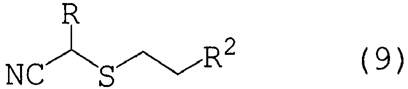
   wherein R and R² have the same meanings as defined above and then the obtained nitrile (9) is oxidized.

The process (A) will be described. The oxidation of the ester (3) in the process (A) is usually carried out by reacting the ester (3) with an oxidizing agent. Examples of the oxidizing agent include oxygen; organic peroxides such as tert-butyl hydroperoxide, cumen hydroperoxide, peracetic acid, trifluoroperacetic acid and m-chloroperbenzoic acid; halogen acids or salts thereof, such as hypochlorous acid or a salt thereof, and chloric acid or a salt thereof; perhalogen acids or salts thereof, such as perchloric acid or a salt thereof, perbromic acid or a salt thereof, and periodic acid or a salt thereof; peroxosulfates such as potassium monopersulfate; permanganates such as potassium permanganate; chromates such as potassium chromate; percarbonates such as sodium percarbonate; and hydrogen peroxide; and hydrogen peroxide is preferable. The use amount of the oxidizing agent is usually from 1 to 10 mol based on 1 mol of the ester (3). It is possible to control a formation ratio of an ester (5) wherein n is 1 to an ester (5) wherein n is 2 by appropriately adjusting the use amount of the oxidizing agent. When the ester (5) wherein n is 1 is preferentially produced, it is preferable to use an oxidizing agent in an amount of less than 1.5 mol, and more preferably the oxidizing agent in an amount of less than 1.3 mol, based on 1 mol of the ester (3). When the ester (5) wherein n is 2 is preferentially produced, it is preferable to use an oxidizing agent in an amount of 1.5 mol or more, and more preferably the oxidizing agent in an amount of 2 mol or more, based on 1 mol of the ester (3).

The oxidation of the ester (3) can be carried out in the coexistence of a catalyst. Examples of the catalyst include alkali metal carbonates such as sodium carbonate and potassium carbonate; alkali metal hydrogen carbonates such as sodium hydrogen carbonate and potassium hydrogen carbonate; tungsten metal, tungsten compounds containing tungsten and group 13 elements, such as tungsten boride; tungsten compounds containing tungsten and group 14 elements, such as tungsten carbide; tungsten compounds containing tungsten and group 15 elements, such as tungsten nitride; tungsten compounds containing tungsten and group 16 elements, such as tungsten oxide, tungstic acid, sodium tungstate and tungsten sulfide; molybdenum compounds, for example, molybdenum metal, molybdenum compounds containing molybdenum and group 13 elements, such as molybdenum boride, molybdenum compounds containing molybdenum and group 14 elements, such as molybdenum carbide, molybdenum compounds containing molybdenum and group 15 elements, such as molybdenum nitride; molybdenum compounds containing molybdenum and group 16 elements, such as molybdenum oxide, molybdic acid, sodium molybdate and molybdenum sulfide; rhenium metal, rhenium oxide, vanadium metal, vanadium oxide, platinum, platinum oxide, palladium, platinum oxide, palladium acetate and palladium chloride. Among these catalysts, tungsten compounds are preferable, and tungsten compounds containing tungsten and group 16 elements are more preferable.

The use amount of the catalyst is usually from 0.0001 to 1 mol, and preferably from 0.001 to 0.1 mol, based on 1 mol of the ester (3).

The ester (3) is usually oxidized by mixing the ester (3) with an oxidizing agent in the presence of a solvent. Examples of the solvent include alcohol solvents such as methanol and ethanol; halogenated hydrocarbon solvents such as dichloromethane, chloroform and chlorobenzene; aromatic hydrocarbon solvents such as toluene and xylene, aliphatic carboxylic acid solvents such as acetic acid and trifluoroacetic acid; ester solvents such as ethyl acetate; ketone solvents such as acetone and ethyl methyl ketone; nitrile solvents such as acetonitrile; amide solvents such as N,N-dimethylformamide; water and mixed solvents thereof. There is no limitation on the use amount of the solvent.

The reaction temperature of oxidation is usually from -50 to 200°C, and the reaction time is usually from 1 to 72 hours.

After completion of the reaction, for example, the ester (5) can be extracted by optionally decomposing the oxidizing agent remaining in the reaction mixture using sodium sulfite, sodium thiosulfate and the like, and washing with water, an aqueous sodium thiosulfate solution, sodium bicarbonate water and the like, followed by concentration. The extracted ester (5) can be further purified by conventional purification means such as distillation, crystallization or column chromatography. The ester (5) can be used to the subsequent step without being purified.

Examples of the ester (5) wherein n is 2 include methyl (3-fluoropropylsulfonyl)acetate, ethyl (3-fluoropropylsulfonyl)acetate, propyl (3-fluoropropylsulfonyl)acetate, isopropyl (3-fluoropropylsulfonyl)acetate, butyl (3-fluoropropylsulfonyl)acetate, isobutyl (3-fluoropropylsulfonyl)acetate, tert-butyl (3-fluoropropylsulfonyl)acetate, methyl (3,3-difluoropropylsulfonyl)acetate, ethyl (3,3-difluoropropylsulfonyl)acetate, propyl (3,3-difluoropropylsulfonyl)acetate, isopropyl (3,3-difluoropropylsulfonyl)acetate, butyl (3,3-difluoropropylsulfonyl)acetate, isobutyl (3,3-difluoropropylsulfonyl)acetate, tert-butyl (3,3-difluoropropylsulfonyl)acetate, methyl (3,3,3-trifluoropropylsulfonyl)acetate, ethyl (3,3,3-trifluoropropylsulfonyl)acetate, propyl (3,3,3-trifluoropropylsulfonyl)acetate, isopropyl (3,3,3-trifluoropropylsulfonyl)acetate, butyl (3,3,3-trifluoropropylsulfonyl)acetate, isobutyl (3,3,3-trifluoropropylsulfonyl acetate and tert-butyl (3,3,3-trifluoropropylsulfonyl)acetate.

Examples of the ester (5) wherein n is 1 include methyl (3-fluoropropylsulfinyl)acetate, ethyl (3-fluoropropylsulfinyl)acetate, propyl (3-fluoropropylsulfinyl)thioacetate, isopropyl (3-fluoropropylsulfinyl)acetate, butyl (3-fluoropropylsulfinyl)acetate, isobutyl (3-fluoropropylsulfinyl)acetate, tert-butyl (3-fluoropropylsulfinyl)acetate, methyl (3,3-difluoropropylsulfinyl)acetate, ethyl (3,3-difluoropropylsulfinyl)acetate, propyl (3,3-difluoropropylsulfinyl)acetate, isopropyl (3,3-difluoropropylsulfinyl)acetate, butyl (3,3-difluoropropylsulfinyl)acetate, isobutyl (3,3-difluoropropylsulfinyl)acetate, tert-butyl (3,3-difluoropropylsulfinyl)acetate, methyl (3,3,3-trifluoropropylsulfinyl)acetate, ethyl (3,3,3-trifluoropropylsulfinyl)acetate, propyl (3,3,3-trifluoropropylsulfinyl)acetate, isopropyl (3,3,3-trifluoropropylsulfinyl)acetate, butyl (3,3,3-trifluoropropylsulfinyl)acetate, isobutyl (3,3,3-trifluoropropylsulfinyl acetate and tert-butyl (3,3,3-trifluoropropylsulfinyl)acetate.

An amide (6) can be obtained by reacting the ester (5) with ammonia.

An ammonia gas may be used as ammonia, and ammonia water may be used. It is also possible to use an ammonia solution obtained by dissolving ammonia gas in alcohol solvents such as methanol and ethanol.

The use amount of ammonia is usually 1 mol or more based on 1 mol of the ester (5), and there is no limitation on the upper limit of the use amount. However, when the amount is too large, it is likely to become economically disadvantageous. Therefore, the use amount of ammonia is practically 10 mol or less based on 1 mol of the ester (5).

The reaction of the ester (5) with ammonia is usually carried out by mixing the ester (5) with ammonia. Such a reaction is usually carried in the presence of a solvent, and examples of the solvent include aliphatic hydrocarbon solvents such as hexane and heptane; aromatic hydrocarbon solvents such as toluene and xylene; halogenated hydrocarbon solvents such as dichloromethane, chloroform and chlorobenzene; ether solvents such as tert-butyl methyl ether and tetrahydrofuran; alcohol solvents such as methanol and ethanol; ester solvents such as ethyl acetate and butyl acetate; nitrile solvents such as acetonitrile; ketone solvents such as acetone and ethyl methyl ketone; amide solvents such as N,N-dimethylformamide; amine solvents such as triethylamine and pyridine; water and mixed solvents thereof. There is no limitation on the use amount of the solvent.

The reaction temperature is usually from -10 to 50°C, and the reaction time is usually from 10 minutes to 72 hours.

After completion of the reaction, the amide (6) can be extracted, for example, by filtering a crystal precipitated in the reaction mixture. It is also possible to extract the amide (6) by concentrating the obtained reaction mixture. The extracted amide (6) can be further purified by conventional purification means such as recrystallization.

Examples of the amide (6) include (3-fluoropropylsulfinyl)acetamide, (3,3-difluoropropylsulfinyl)acetamide, (3,3,3-trifluoropropylsulfinyl)acetamide, (3-fluoropropylsulfonyl)acetamide, (3,3-difluoropropylsulfonyl)acetamide and (3,3,3-trifluoropropylsulfonyl)acetamide.

A nitrile (7) can be obtained by converting a carbamoyl group (-CONH₂) of the obtained amide (6) into a cyano group (-CN).

Examples of the process of converting a carbamoyl group of the obtained amide (6) into a cyano group include a process in which the amide (6) is reacted with a dehydrating agent. Examples of the dehydrating agent include a dehydrating agent which is usually used in a process in which a carbamoyl group is converted into a cyano group. Specific examples thereof include thionyl chloride, sulfuryl chloride, carbonyl chloride, triphosgene, dicyclohexylcarbodiimide, diisopropylcarbodiimide, di-tert-butylcarbodiimide, phosphorus oxychloride, phosphorus trichloride, phosphorus pentachloride, trifluoroacetic anhydride/base (pyridine, etc.) and methanesulfonyl chloride/base (pyridine, etc.). Among these dehydrating agents, phosphorus oxychloride, thionyl chloride, trifluoroacetic anhydride/pyridine and methanesulfonyl chloride/pyridine are preferable, and phosphorus oxychloride is more preferable.

The use amount of the dehydrating agent is usually from 1 to 10 mol based on 1 mol of the amide (6).

The reaction of the amide (6) with the dehydrating agent is usually carried out by mixing the amide (6) with dehydrating agent in the absence or presence of a solvent. Examples of the solvent include aromatic hydrocarbon solvents such as toluene and xylene; nitrile solvents such as acetonitrile; and the like. There is no limitation on the use amount. The reaction temperature is usually from 0 to 120°C, and preferably from 40 to 80°C. The reaction time is usually from 10 minutes to 24 hours.

After completion of the reaction, for example, the reaction mixture, water, an aqueous solution of an alkali, such as an aqueous sodium hydrogen carbonate solution, an aqueous sodium carbonate solution or an aqueous sodium hydroxide solution, or an aqueous solution of an acid such as hydrochloric acid are mixed and the mixture is extracted with an organic solvent which is insoluble in water to obtain an organic layer containing a nitrile (7), and then the nitrile (7) can be extracted by concentrating the organic layer. The extracted nitrile (7) can be further purified by conventional purification means such as recrystallization or column chromatography.

Examples of the nitrile (7) include (3-fluoropropylsulfinyl)acetonitrile, (3,3-difluoropropylsulfinyl)acetonitrile, (3,3,3-trifluoropropylsulfinyl)acetonitrile, (3-fluoropropylsulfonyl)acetonitrile, (3,3-difluoropropylsulfonyl)acetonitrile and (3,3,3-trifluoropropylsulfonyl)acetonitrile.

Subsequently, the process (B) will be described. The reaction of the ester (3) with ammonia in the process (B) can be carried out in the same manner as in the above-mentioned reaction of the ester (5) with ammonia. Specifically, the reaction is carried out by mixing the ester (3) with ammonia using, as ammonia, an ammonia gas, ammonia water, or an ammonia solution obtained by dissolving an ammonia gas in alcohol solvents such as methanol and ethanol. The use amount of ammonia is usually 1 mol or more based on 1 mol of the ester (3). There is no limitation on the upper limit. However, when the amount is too large, it is likely to become economically disadvantageous. Therefore, the use amount of ammonia is practically 10 mol or less based on 1 mol of the ester (3). Such a reaction is usually carried out in the presence of a solvent. Examples of the solvent include aliphatic hydrocarbon solvents such as hexane and heptane; aromatic hydrocarbon solvents such as toluene and xylene; halogenated hydrocarbon solvents such as dichloromethane, chloroform and chlorobenzene; ether solvents such as tert-butyl methyl ether and tetrahydrofuran; alcohol solvents such as methanol and ethanol; ester solvents such as ethyl acetate and butyl acetate; nitrile solvents such as acetonitrile; ketone solvents such as acetone and ethyl methyl ketone; amide solvents such as N,N-dimethylformamide; amine solvents such as triethylamine and pyridine; water and mixed solvents thereof. There is no limitation on the use amount of the solvent.

The reaction temperature is usually from -10 to 50°C, and the reaction time is usually from 10 minutes to 72 hours.

After completion of the reaction, an amide (8) can be extracted, for example, by filtering a crystal precipitated in the reaction mixture. It is also possible to extract the amide (8) by concentrating the obtained reaction mixture. The extracted amide (8) can be further purified by conventional purification means such as recrystallization.

Examples of the amide (8) include (3-fluoropropylthio)acetamide, (3,3-difluoropropylthio)acetamide and (3,3,3-trifluoropropylthio)acetamide.

An amide (6) is obtained by oxidizing the obtained amide (8). The oxidation of the amide (8) can be carried out in the same manner as in the above-mentioned oxidation of the ester (3). Specifically, the oxidation is carried out by reacting the amide (8) with the oxidizing agent. Examples of the oxidizing agent include oxygen; organic peroxides such as tert-butyl hydroperoxide, cumen hydroperoxide, peracetic acid, trifluoroperacetic acid and m-chloroperbenzoic acid; halogen acids or salts thereof, such as hypochlorous acid or a salt thereof, and chloric acid or a salt thereof; perhalogen acids or salts thereof, such as perchloric acid or a salt thereof, perbromic acid or a salt thereof, and periodic acid or a salt thereof; peroxosulfates such as potassium monopersulfate; permanganates such as potassium permanganate; chromates such as potassium chromate; percarbonates such as sodium percarbonate; and hydrogen peroxide; and hydrogen peroxide is preferable. The use amount of the oxidizing agent is usually from 1 to 10 mol based on 1 mol of the amide (8). It is possible to control a formation ratio of an amide (6) wherein n is 1 to an amide (6) wherein n is 2 by appropriately adjusting the use amount of the oxidizing agent. When the amide (6) wherein n is 1 is preferentially produced, it is preferable to use an oxidizing agent in an amount of less than 1.5 mol, and more preferably the oxidizing agent in an amount of less than 1.3 mol, based on 1 mol of the amide (8). When the amide (6) wherein n is 2 is preferentially produced, it is preferable to use an oxidizing agent in an amount of 1.5 mol or more, and more preferably the oxidizing agent in an amount of 2 mol or more, based on 1 mol of the amide (8).

The oxidation of the amide (8) can be carried out in the presence of a catalyst. Examples of the catalyst include the same catalyst as that used in the oxidation of the ester (3), and a tungsten compound is preferable, and a tungsten compound such as a tungsten compound containing tungsten and group 16 elements are more preferable. The use amount of the catalyst is usually from 0.0001 to 1 mol, and preferably from 0.001 to 0.1 mol, based on 1 mol of the amide (8).

The oxidation of the amide (8) is usually carried out by mixing the amide (8) with the oxidizing agent in the presence of a solvent. Examples of the solvent include alcohol solvents such as methanol and ethanol; halogenated hydrocarbon solvents such as dichloromethane, chloroform and chlorobenzene; aromatic hydrocarbon solvents such as toluene and xylene, aliphatic carboxylic acid solvents such as acetic acid and trifluoroacetic acid; ester solvents such as ethyl acetate; ketone solvents such as acetone and ethyl methyl ketone; nitrile solvents such as acetonitrile; amide solvents such as N,N-dimethylformamide; water and mixed solvents thereof. There is no limitation on the use amount of the solvent.

The reaction temperature of the oxidation is usually from -50 to 200°C, and the reaction time is usually from 1 to 72 hours.

After completion of the reaction, for example, the ester (6) can be extracted by optionally decomposing the oxidizing agent remaining in the reaction mixture using sodium sulfite, sodium thiosulfate and the like, and washing with water, an aqueous sodium thiosulfate solution, sodium bicarbonate water and the like, followed by concentration. The extracted amide (6) can be further purified by conventional purification means such as recrystallization. The amide (6) can be used for the subsequent step without being purified.

A nitrile (7) can be obtained by converting a carbamoyl group (-CONH₂) of the amide (6) into a cyano group (-CN) in the same manner as in the process described in the above-mentioned process (A).

Finally, the process (C) will be described. The reaction of an ester (3) with ammonia in the process (C) can be carried out in the same manner as described in the above-mentioned process (B). A nitrile (9) can be obtained by converting a carbamoyl group (-CONH₂) of the obtained amide (8) into a cyano group (-CN).

Examples of the process of converting a carbamoyl group (-CONH₂) of the amide (8) into a cyano group (-CN) include the same process in which a carbamoyl group (-CONH₂) of the amide (6) is converted into a cyano group (-CN) in the above-mentioned process (A). Specific examples thereof include a process in which an amide (8) is reacted with a dehydrating agent. Examples of the dehydrating agent include a dehydrating agent which is usually used in a process in which a carbamoyl group is converted into a cyano group. Specific examples of the dehydrating agent include thionyl chloride, sulfuryl chloride, carbonyl chloride, triphosgene, dicyclohexylcarbodiimide, diisopropylcarbodiimide, di-tert-butylcarbodiimide, phosphorus oxychloride, phosphorus trichloride, phosphorus pentachloride, trifluoroacetic anhydride/base (pyridine, etc.) and methanesulfonyl chloride/base (pyridine, etc.). Among these dehydrating agents, phosphorus oxychloride, thionyl chloride, trifluoroacetic anhydride/pyridine and methanesulfonyl chloride/pyridine are preferable, and phosphorus oxychloride is more preferable.

The use amount of the dehydrating agent is usually from 1 to 10 mol based on 1 mol of the amide (8).

The reaction of the amide (8) with the dehydrating agent is usually carried out by mixing the amide (8) with the dehydrating agent in the absence or presence of a solvent. Examples of the solvent include aromatic hydrocarbon solvents such as toluene and xylene; and nitrile solvents such as acetonitrile. There is no limitation on the use amount. The reaction temperature is usually from 0 to 120°C, and preferably from 40 to 80°C. The reaction time is usually from 10 minutes to 24 hours.

After completion of the reaction, for example, the reaction mixture, water, an aqueous solution of an alkali, such as sodium bicarbonate water, or an aqueous solution of an acid such as hydrochloric acid are mixed and the mixture is extracted with an organic solvent which is insoluble in water to obtain an organic layer containing a nitrile (9), and then the nitrile (9) can be extracted by concentrating the organic layer. The extracted nitrile (9) can be further purified by conventional purification means such as recrystallization or column chromatography.

Examples of the nitrile (9) include (3-fluoropropylthio)acetonitrile, (3,3-difluoropropylthio)acetonitrile and (3,3,3-trifluoropropylthio)acetonitrile.

The nitrile (7) can be obtained by oxidizing the obtained nitrile (9). The oxidation of the nitrile (9) can be carried out in the same manner as in the above-mentioned oxidation of the ester (3). Specifically, the oxidation can be carried out by reacting the nitrile (9) with the oxidizing agent. Examples of the oxidizing agent include oxygen; organic peroxides such as tert-butyl hydroperoxide, cumen hydroperoxide, peracetic acid, trifluoroperacetic acid and m-chloroperbenzoic acid; halogen acids or salts thereof, such as hypochlorous acid or a salt thereof and chloric acid or a salt thereof; perhalogen acids or salts thereof, such as perchloric acid or a salt thereof, perbromic acid or a salt thereof, and periodic acid or a salt thereof; peroxosulfates such as potassium monopersulfate; permanganates such as potassium permanganate; chromates such as potassium chromate; percarbonates such as sodium percarbonate; and hydrogen peroxide; and hydrogen peroxide is preferable. The use amount of the oxidizing agent is usually from 1 to 10 mol based on 1 mol of the nitrile (9). It is possible to control a formation ratio of a nitrile (7) wherein n is 1 to a nitrile (7) wherein n is 2 by appropriately adjusting the use amount of the oxidizing agent. When the nitrile (7) wherein n is 1 is preferentially produced, it is preferable to use an oxidizing agent in an amount of less than 1.5 mol, and more preferably the oxidizing agent in an amount of less than 1.3 mol, based on 1 mol of the nitrile (9). When the nitrile (7) wherein n is 2 is preferentially produced, it is preferable to use an oxidizing agent in an amount of 1.5 mol or more, and more preferably the oxidizing agent in an amount of 2 mol or more, based on 1 mol of the nitrile (9).

The oxidation of the nitrile (9) can be carried out in the coexistence of a catalyst. Examples of the catalyst include the same catalyst as that used in the oxidation of the ester (3). A tungsten compound is preferable, and a tungsten compound containing tungsten and group 16 elements is more preferable. The use amount of the catalyst is usually from 0.01 to 1 mol based on 1 mol of the nitrile (9).

The oxidation of the nitrile (9) is usually carried out by mixing the nitrile (9) with the oxidizing agent in the presence of a solvent. Examples of the solvent include alcohol solvents such as methanol and ethanol; halogenated hydrocarbon solvents such as dichloromethane, chloroform and chlorobenzene; aromatic hydrocarbon solvents such as toluene and xylene, aliphatic carboxylic acid solvents such as acetic acid and trifluoroacetic acid; ester solvents such as ethyl acetate; ketone solvents such as acetone and ethyl methyl ketone; nitrile solvents such as acetonitrile; amide solvents such as N,N-dimethylformamide; water and mixed solvents thereof. There is no limitation on the use amount of the solvent.

The reaction temperature of oxidation is usually from -50 to 200°C, and the reaction time is usually from 1 to 72 hours.

After completion of the reaction, for example, the nitrile (7) can be extracted by optionally decomposing the oxidizing agent remaining in the reaction mixture using sodium sulfite, sodium thiosulfate and the like, and washing with water, an aqueous sodium thiosulfate solution, sodium bicarbonate water and the like, followed by concentration. The extracted nitrile (7) can be further purified by conventional purification means such as crystallization.

### EXAMPLES

The present invention will be described in further detail below by way of Examples, but the present invention is not limited to the following Examples. Analysis was carried out by an internal standard high performance liquid chromatography method.

### Example 1

3,3,3-trifluoropropene (9.8 g), methyl thioglycolate (2.0 g) and 2,2'-azobis(2,4-dimethyl-4-methoxyvaleronitrile) (0.30 g) were charged in an autoclave cooled to -78°C. The obtained mixture was maintained at 20°C for 20 hours while stirring. The obtained reaction mixture was purified by silica gel chromatography to obtain methyl (3,3,3-trifluoropropylthio)acetate (3.75 g). Yield: 98% (based on methyl thioglycolate).

### Example 2

Methyl thioglycolate (55 g) and 2,2'-azobis(2,4-dimethyl-4-methoxyvaleronitrile) (8.0 g) were dissolved in ethyl acetate (150 g) at 20°C. 3,3,3-trifluoropropene (50 g) was blown into the obtained solution. The obtained mixture was stirred at 20°C for 8 hours. The obtained reaction mixture was maintained at 50°C for 1 hour thereby decomposing 2,2'-azobis(2,4-dimethyl-4-methoxyvaleronitrile), followed by concentration to obtain an oily product. The oily product was analyzed. As a result, it was found that the oily product contains methyl (3,3,3-trifluoropropylthio)acetate (89 g). Yield: 84% (based on methyl thioglycolate).
¹H-NMR(CDCl₃, internal standard:
tetramethylsilane)δ(ppm):2.370-2.488(2H,m), 2.809-2.849(2H,m), 3.263(2H,s),3.758(3H,s)
boiling point:190.3°C(750Torr)

### Example 3

The oily product (25 g) containing methyl (3,3,3-trifluoropropylthio)acetate (20 g) obtained in Example 2 and water (0.81 g) and sodium tungstate dihydrate (0.27 g) were charged in a 100 mL recovery flask. 35% hydrogen peroxide water (9.9 g) was added dropwise to the obtained mixture at an inner temperature of 25°C over 4 hours. After adjusting the inner temperature of the obtained mixture to 55°C, 35% hydrogen peroxide water (9.9 g) was added dropwise over 2 hours. The obtained mixture was maintained at an inner temperature of 55°C for 3 hours. After cooling the obtained reaction mixture, an aqueous 5% sodium thiosulfate solution (10 g) was added and the oil layer was separated. The oil layer was washed with water (10 g) to obtain a pale yellow oil layer (20.5 g). The obtained oil layer was subjected to gas chromatography analysis. As a result, it was found that methyl 2-(3,3,3-trifluoropropylsulfonyl)acetate (17.0 g) is contained. Yield: 91%.

The obtained oil layer (20 g) and diisopropyl ether (20 g) were charged in a 50 mL recovery flask. The obtained mixture was heated to 35°C and then cooled to 0°C over 1 hour. As a result, a white solid was precipitated. The white solid was extracted by filtration and was washed with cold diisopropyl ether (10 g) to obtain methyl 2-(3,3,3-trifluoropropylsulfonyl)acetate (15.2 g) having a purity of 98%.
¹H-NMR(CDCl₃, internal standard :tetramethylsilane) δ(ppm):2.654-2.771(2H,m), 3.508-3.550(2H,m), 3.854(3H,s), 4.042(2H,s)

### Example 4

Methyl 2-(3,3,3-trifluoropropylsulfonyl)acetate (2.0 g) obtained in Example 3, a 11% ammonia/methanol solution (12.7 g) and methanol (3.0 g) were charged in a 50 mL recovery flask. The obtained mixture was stirred at 25°C for 2 hours and then the solvent was removed by a rotary evaporator to obtain a white solid (1.85 g). The white solid was subjected to gas chromatography analysis. As a result, it was found that 2-(3,3,3-trifluoropropylsulfonyl)acetamide (1.80 g) is contained. Content: 97%, Yield: 100%.
¹H-NMR ((CD₃)₂SO, internal standard :tetramethylsilane)δ(ppm):2.729-2.852(2H,m), 3.580-3.621(2H,m), 4.169(2H,s), 7.531(1H,s), 7.795(1H,s)

### Example 5

2-(3,3,3-trifluoropropylsulfonyl)acetamide (1.0 g) obtained in Example 4 and phosphorus oxychloride (3.3 mL) were charged in a 20 mL recovery flask. The obtained mixture was heated at 60°C for 2 hours. The obtained reaction mixture was added in sodium bicarbonate water (sodium bicarbonate/water = 1/2) and the precipitated solid was extracted by filtration to obtain a light brown solid (0.92 g). The solid was washed with water and then dried. The obtained solid was subjected to gas chromatography analysis. As a result, it was found that 2-(3,3,3-trifluoropropylsulfonyl)acetonitrile (0.87 g) is contained. Content: 95%, Yield: 95%.
¹H-NMR (CDCl₃, internal standard:tetramethylsilane)δ(ppm):2.723-2.838(2H,m), 3.503-3.544(2H,m), 4.061(2H,s)

### Example 6

3,3,3-trifluoropropene (45.3 g) was blown into a solution obtained by dissolving methyl thioglycolate (50.0 g, purity of 99%) in ethyl acetate (200 g) at 20°C. 2,2'-azobis(2,4-dimethyl-4-methoxyvaleronitrile) (3.03 g, purity of 95%) was added to the obtained solution. The obtained mixture was stirred at 20°C for 6 hours, stirred at 25°C for 14 hours and then stirred at 30°C for 9 hours. The obtained reaction mixture was maintained at 50°C for 1 hour thereby decomposing the remaining 2,2'-azobis(2,4-dimethyl-4-methoxyvaleronitrile), followed by concentration under reduced pressure condition to obtain an oily product (97.4 g). The oily product was analyzed. As a result, it was found that the oily product contains methyl 2-(3,3,3-trifluoropropylthio)acetate (79.4 g). Yield: 84% (based on methyl thioglycolate).

### Example 7

3,3,3-trifluoropropene (180 g) was blown into a solution obtained by dissolving methyl thioglycolate (200 g, purity of 99%), 2,2'-azobis(2,4-dimethyl-4-methoxyvaleronitrile) (12.1 g, purity of 95%) in ethyl acetate (800 g) over 5 hours while maintaining at 18 to 20°C. The obtained mixture was stirred at 20°C for 3 hours, stirred at 25°C for 14 hours and then stirred at 30°C for 9 hours. The obtained reaction mixture was maintained at 50°C for 1 hour thereby decomposing the remaining 2,2'-azobis(2,4-dimethyl-4-methoxyvaleronitrile), followed by concentration under reduced pressure condition to obtain an oily product (435 g). The oily product was analyzed. As a result, it was found that the oily product contains 2-methyl (3,3,3-trifluoropropylthio)acetate (313 g). Yield: 83% (based on methyl thioglycolate).

### Example 8

3,3,3-trifluoropropene (21.5 g) was blown into a solution obtained by dissolving methyl thioglycolate (20.0 g, purity of 99%), 2,2'-azobis(2,4-dimethyl-4-methoxyvaleronitrile) (1.21 g, purity of 95%) in ethyl acetate (80.0 g) over 3 hours while maintaining at 39 to 41°C. The obtained mixture was stirred at 40 to 41°C for 21 hours and the obtained reaction mixture was concentrated under reduced pressure condition to obtain an oily product (41.0 g). The oily product was analyzed. As a result, it was found that the oily product contains methyl 2-(3,3,3-trifluoropropylthio)acetate (29.8 g) Yield: 79% (based on methyl thioglycolate).

### Example 9

30% ammonia water was added dropwise to a solution obtained by dissolving methyl 2-(3,3,3-trifluoropropylthio)acetate (50.3 g, purity of 93%) in methanol (125 g) at an inner temperature of 23°C to 25°C over 45 minutes. The obtained mixture was stirred at 23°C to 25°C for 16 hours and then 35% hydrochloric acid (78.3 g) was added dropwise over 42 minutes. During the dropwise addition of hydrochloric acid, the inner temperature rose from 24°C to 53°C. The obtained reaction mixture was concentrated under reduced pressure condition. Water (250 g) was added to the obtained residue and the obtained mixture was extracted twice with tert-butyl methyl ether (300 g). The obtained organic layers were combined and then dried over anhydrous magnesium sulfate. Magnesium sulfate was removed by filtration and then the obtained filtrate was concentrated under reduced pressure to obtain a crude crystal (47.2 g). The obtained crude crystal and diisopropyl ether (93.1 g) were mixed. The obtained mixture was stirred at an inner temperature of 47°C to 53°C for 15 minutes and then the inner temperature was gradually cooled to room temperature over 2 hours while stirring, followed by stirring at an inner temperature of 0.2°C for 7 hours and 40 minutes. The precipitated crystal was extracted by filtration, washed with diisopropyl ether (39.4 g) and then dried under reduced pressure to obtain 2-(3,3,3-trifluoropropylthio)acetamide (32.0 g). Purity: 99.8%, Yield: 74%.
¹H-NMR (CDCl₃, internal standard:tetramethylsilane)δ(ppm):2.397-2.488(2H,m), 2.763-2.803(2H,m), 3.245(2H,s), 6.221(1H,s), 6.558(1H,s)

### Example 10

A solution obtained by dissolving methanesulfonyl chloride (0.15 g) in toluene (1.00 g) was added dropwise to a solution obtained by dissolving 2-(3,3,3-trifluoropropylthio)acetamide (0.22 g) in toluene (2.14 g) at room temperature, and then a solution obtained by dissolving pyridine (0.13 g) in toluene (0.98 g) was added dropwise. The obtained mixture was stirred at room temperature for 21.5 hours and then pyridine (0.13 g) was added at room temperature. The obtained mixture was stirred at room temperature for 4 days. Furthermore, the obtained mixture was heated at 63°C for 5.5 hours and then cooled to room temperature, followed by the addition of 3 droplets of methanesulfonyl chloride. The obtained mixture was heated at 63°C for 8 hours and then cooled to room temperature, followed by the addition of pyridine (0.14 g) and methanesulfonyl chloride (5 droplets). The obtained mixture was heated at 63°C for 9.5 hours and then cooled to room temperature. Water and 35% hydrochloric acid (10 droplets) were added to the obtained reaction mixture, followed by extraction with tert-butyl methyl ether. The obtained oil layer was washed with water and then dried over anhydrous magnesium sulfate. After removing magnesium sulfate by filtration, the solvent was distilled off by heating the obtained filtrate at a bath temperature of 40°C to 50°C under reduced pressure condition to obtain a crude product containing 2-(3,3,3-trifluoropropylthio)acetonitrile. The content of 2-(3,3,3-trifluoropropylthio)acetonitrile was 91.4%.

The obtained crude product containing 2-(3,3,3-trifluoropropylthio)acetonitrile, sodium tungstate dihydrate and hydrogen peroxide water were mixed to obtain 2-(3,3,3-trifluoropropylsulfonyl)acetonitrile.
¹H-NMR(CDCl₃, internal standard:tetramethylsilane)δ(ppm):2.465-2.555(2H,m), 2.926-2.966(2H,m), 3.357(2H,s)
boiling point:200.0°C(750Torr)

### Example 11

Methyl 2-(3,3,3-trifluoropropylsulfonyl)acetate (12.0 g, purity of 73%) and methanol (8.70 g) were charged in a 100 mL recovery flask. An aqueous 28% ammonia solution (6.80 g) was added dropwise to the obtained mixture. The obtained mixture was stirred at 27 to 34°C for 3 hours. The obtained reaction mixture was cooled to 0 to 5°C, and then filtered to obtain a white solid (7.15 g). The white solid was subjected to gas chromatography analysis. As a result, it was found that the white solid contains (3,3,3-trifluoropropylsulfonyl)acetamide (6.95 g). Yield: 84%.

### Example 12

Methyl 2-(3,3,3-trifluoropropylsulfonyl)acetate (12.0 g, purity of 73%) and water (17.5 g) were charged in a 100 mL recovery flask. An aqueous 28% ammonia solution (6.80 g) was added dropwise to the obtained mixture. The obtained mixture was stirred at 22 to 30°C for 3 hours. The obtained reaction mixture was cooled to 0 to 5°C, and then filtered to obtain a white solid (6.77 g). The white solid was subjected to gas chromatography analysis. As a result, it was found that the white solid contains 2-(3,3,3-trifluoropropylsulfonyl)acetamide (6.46 g). Yield: 78%.

### Example 13

An ethyl acetate solution (20.0 g, content of 43%) containing methyl 2-(3,3,3-trifluoropropylsulfonyl)acetate and methanol (8.8 g) were charged in a 100 mL recovery flask. An aqueous 28% ammonia solution (6.80 g) was added dropwise to the obtained mixture. The obtained mixture was stirred at 25°C for 21 hours. The obtained reaction mixture was cooled to 0 to 5°C and then filtered to obtain a white solid (6.69 g). The white solid was subjected to gas chromatography analysis. As a result, it was found that the white solid contains 2-(3,3,3-trifluoropropylsulfonyl)acetamide (6.09 g). Yield: 75%.

### Example 14

An ethyl acetate solution (20.0 g, content of 43%) containing methyl 2-(3,3,3-trifluoropropylsulfonyl)acetate and water (17.5 g) were charged in a 100 mL recovery flask. An aqueous 28% ammonia solution (6.80 g) was added dropwise to the obtained mixture. The obtained mixture was stirred at 25°C for 19 hours. The obtained reaction mixture was cooled to 0 to 5°C and then filtered to obtain a white solid (6.29 g). The white solid was subjected to gas chromatography analysis. As a result, it was found that the white solid contains 2-(3,3,3-trifluoropropylsulfonyl)acetamide (6.06 g). Yield: 75%.

### Example 15

After adjusting the inner temperature of a suspension containing 2-(3,3,3-trifluoropropylsulfonyl)acetamide (5.00 g, purity of 99%) and acetonitrile (7.50 g) to 63°C, phosphorus oxychloride (3.90 g) was added dropwise to the suspension over 30 minutes. The obtained mixture was stirred at 64 to 66°C for 4 hours. The obtained reaction mixture was added dropwise in a mixture of 7% sodium bicarbonate water (82.2 g) and ethyl acetate (10.0 g). The obtained mixture was allowed to undergo liquid separation and then the oil layer was concentrated by a rotary evaporator to obtain a white solid (4.32 g). The white solid was subjected to gas chromatography analysis. As a result, it was found that the white solid contains 2-(3,3,3-trifluoropropylsulfonyl)acetonitrile (4.30 g). Yield: 94%.

### Example 16

An ammonia gas (63.0 g) was blown into a solution obtained by dissolving methyl 2-(3,3,3-trifluoropropylsulfonyl)acetate (450 g, purity of 65%) in methanol (450 g) at 0 to 3°C. The obtained mixture was stirred at the same temperature for 18 hours. The obtained reaction mixture was filtered to obtain a yellowish white solid. The yellowish white solid was washed with cold methanol (250 g) and then dried to obtain a white solid (236 g). The white solid was subjected to gas chromatography analysis. As a result, it was found that the white solid contains 2-(3,3,3-trifluoropropylsulfonyl)acetamide (235 g). Yield: 86%.

### Example 17

Thionyl chloride (1.00 g) was added to a solution obtained by dissolving 2-(3,3,3-trifluoropropylsulfonyl)acetamide (1.80 g, purity of 99%) in acetonitrile (3.60 g). The obtained mixture was stirred at an inner temperature of 70 to 75°C for 1.5 hours and then thionyl chloride (0.90 g) was further added. The obtained mixture was stirred at an inner temperature of 70°C for 0.5 hour. The obtained reaction mixture was subjected to gas chromatography analysis. As a result, it was found that 2-(3,3,3-trifluoropropylsulfonyl)acetonitrile (3.2%) is formed.

### Example 18

Trifluoroacetic anhydride (3.00 g) was added dropwise to a solution obtained by dissolving 2-(3,3,3-trifluoropropylsulfonyl)acetamide (2.00 g, purity of 99%) in pyridine (3.50 g) over 10 minutes under ice cooling while stirring, and then pyridine (1.40 g) was added. The obtained mixture was stirred at 22 to 32°C for 22 hours. Water (10.9 g) was added to the obtained reaction mixture and the obtained mixture was extracted with tert-butyl methyl ether (15.4 g). Toluene (40.2 g) was added to the obtained oil layer and the solvent was removed by a rotary evaporator to obtain a black oily product (2.47 g). The black oily product was subjected to gas chromatography analysis. As a result, it was found that the black oily product contains 2-(3,3,3-trifluoropropylsulfonyl)acetonitrile (0.53 g). Yield: 29%.

### Example 19

35% hydrogen peroxide water (3.20 g) was added dropwise to a mixture containing 2-(3,3,3-trifluoropropylthio)acetamide (5.00 g), water (25.1 g) and sodium tungstate dihydrate (0.02 g) at an inner temperature of 50 to 59°C over 25 minutes while vigorously stirring the mixture. The obtained mixture was at an inner temperature of 44 to 48°C and then 35% hydrogen peroxide water (3.40 g) was added dropwise over 5 minutes. The obtained mixture was stirred at 45 to 48°C for 2 hours. After cooling the obtained reaction mixture, the precipitated solid was extracted by filtration. The extracted solid was washed with water and then dried to obtain a white solid (4.86 g). The white solid was subjected to gas chromatography analysis. As a result, the content of 2-(3,3,3-trifluoropropylsulfonyl)acetamide was 100%. Yield: 83%.

In the same manner as in Example 18, 2-(3,3,3-trifluoropropylsulfonyl)acetonitrile can be obtained by reacting the obtained 2-(3,3,3-trifluoropropylsulfonyl)acetamide with trifluoroacetic anhydride/pyridine.

### INDUSTRIAL APPLICABILITY

According to the present invention, a novel method for producing a (fluoroalkylthio)acetic acid ester can be provided.

## Claims

1. A process for producing a (fluoroalkylthio)acetic acid ester represented by the formula (3): 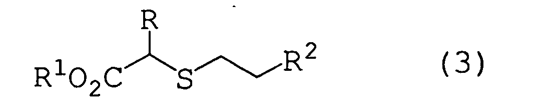
wherein R, R¹ and R² have the same meanings as defined below, which comprises reacting a thioglycolic acid ester represented by the formula (1): 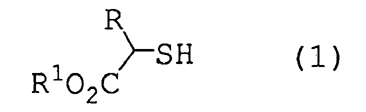
wherein R represents a hydrogen atom or an alkyl group having 1 to 5 carbon atoms, and R¹ represents an alkyl group having 1 to 4 carbon atoms, with fluoroolefin represented by the formula (2): 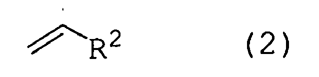
wherein R² represents a fluoroalkyl group having 1 to 5 carbon atoms, in the presence of a radical generator.

2. The process according to claim 1, wherein the radical generator is an azo compound represented by the formula (4): 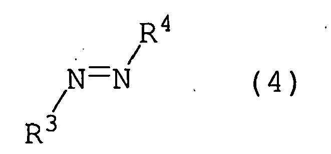
wherein R³ and R⁴ each independently represents an alkyl group capable of having a substituent.

3. The process according to claim 2, wherein the azo compound represented by the formula (4) is 2,2'-azobis(2,4-dimethyl-4-methoxyvaleronitrile).

4. The process according to any one of claims 1 to 3, wherein R¹ is a methyl group.

5. The process according to any one of claims 1 to 4, wherein R² is a trifluoromethyl group.

6. The process according to any one of claims 1 to 5, wherein the reaction is carried out in the presence of ethyl acetate.

7. A process for producing an ester compound represented by the formula (5): 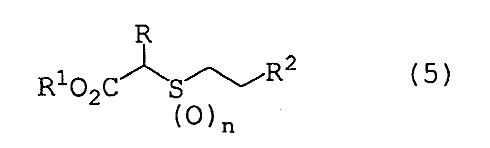
wherein R, R¹ and R² have the same meanings as defined below, and n represents 1 or 2, which comprises obtaining a (fluoroalkylthio)acetic acid ester represented by the formula (3): 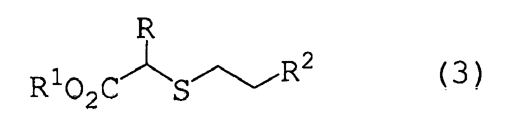
wherein R represents a hydrogen atom or an alkyl group having 1 to 5 carbon atoms, R¹ represents an alkyl group having 1 to 4 carbon atoms, and R² represents a fluoroalkyl group having 1 to 5 carbon atoms, by the process according to claim 1, and then oxidizing the obtained (fluoroalkylthio)acetic acid ester represented by the formula (3).

8. A process for producing an amide compound represented by the formula (6): 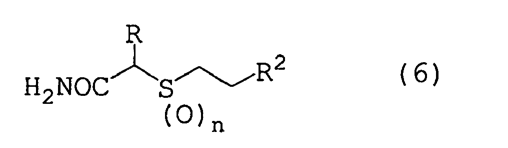
wherein R, R² and n have the same meanings as defined below, which comprises obtaining an ester compound represented by the formula (5): 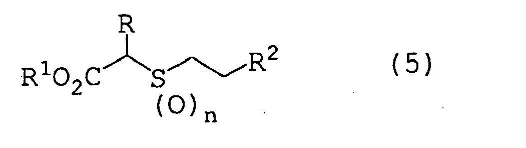
wherein R represents a hydrogen atom or an alkyl group having 1 to 5 carbon atoms, R¹ represents an alkyl group having 1 to 4 carbon atoms, R² represents a fluoroalkyl group having 1 to 5 carbon atoms, and n represents 1 or 2, by the process according to claim 7, and then reacting the obtained ester compound represented by the formula (5) with ammonia.

9. A process for producing a nitrile compound represented by the formula (7): 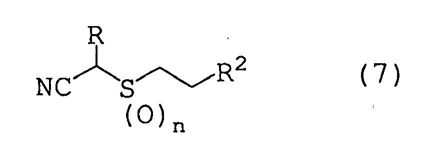
wherein R, R² and n have the same meanings as defined below, which comprises obtaining an amide compound represented by the formula (6): 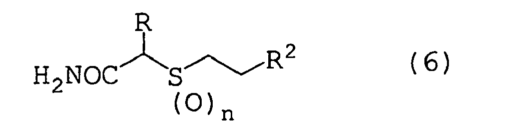
wherein R represents a hydrogen atom or an alkyl group having 1 to 5 carbon atoms, R² represents a fluoroalkyl group having 1 to 5 carbon atoms, and n represents 1 or 2, by the process according to claim 8, and then converting a carbamoyl group (-CONH₂) of the obtained amide compound represented by the formula (6) into a cyano group (-CN).

10. A process for producing a (fluoroalkylthio)acetamide represented by the formula (8): 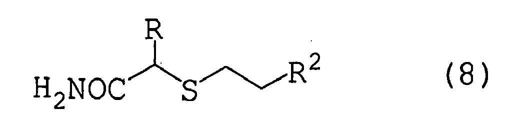
wherein R and R² have the same meanings as defined below, which comprises obtaining a (fluoroalkylthio)acetic acid ester represented by the formula (3): 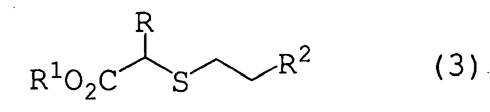
wherein R represents a hydrogen atom or an alkyl group having 1 to 5 carbon atoms, R¹ represents an alkyl group having 1 to 4 carbon atoms, and R² represents a fluorine-containing alkyl group having 1 to 5 carbon atoms, by the process according to claim 1, and then reacting the obtained (fluoroalkylthio)acetic acid ester represented by the formula (3) with ammonia.

11. A process for producing an amide compound represented by the formula (6): 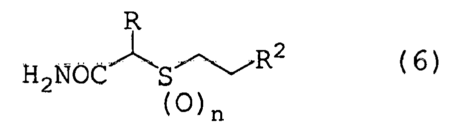
wherein R and R² have the same meanings as defined below, and n represents 1 or 2, which comprises obtaining a (fluoroalkylthio)acetamide represented by the formula (8): 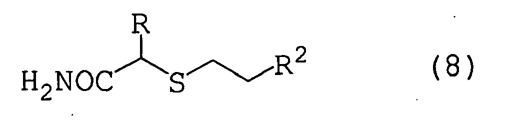
wherein R represents a hydrogen atom or an alkyl group having 1 to 5 carbon atoms, and R² represents a fluorine-containing alkyl group having 1 to 5 carbon atoms, by the process according to claim 10, and then oxidizing the obtained (fluoroalkylthio)acetamide represented by the formula (8).

12. A process for producing a nitrile compound represented by the formula (7) 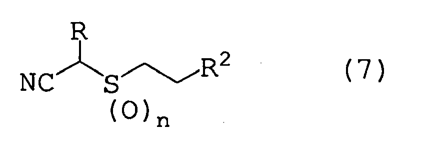
wherein R, R² and n have the same meanings as defined below, which comprises obtaining an amide compound represented by the formula (6): 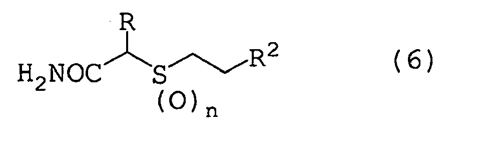
wherein R represents a hydrogen atom or an alkyl group having 1 to 5 carbon atoms, R² represents a fluorine-containing alkyl group having 1 to 5 carbon atoms, and n represents 1 or 2, by the process according to claim 11, and then converting a carbamoyl group (-CONH₂) of the obtained amide compound represented by the formula (6) into a cyano group (-CN).

13. A process for producing a (fluoroalkylthio)acetonitrile represented by the formula (9) : 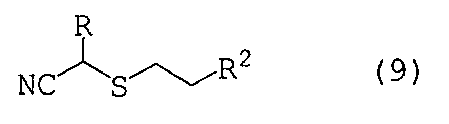
wherein R and R² have the same meanings as defined below, which comprises obtaining a (fluoroalkylthio)acetamide represented by the formula (8): 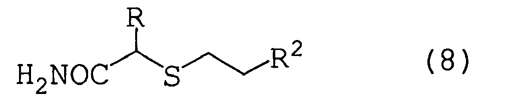
wherein R represents a hydrogen atom or an alkyl group having 1 to 5 carbon atoms, and R² represents a fluorine-containing alkyl group having 1 to 5 carbon atoms, by the process according to claim 10, and then converting a carbamoyl group (-CONH₂) of the obtained (fluoroalkylthio)acetamide represented by the formula (8) into a cyano group (-CN).

14. A process for producing a nitrile compound represented by the formula (7): 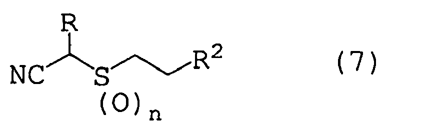
wherein R and R² have the same meanings as defined below, and n represents 1 or 2), which comprises obtaining an (fluoroalkylthio)acetonitrile represented by the formula (9) : 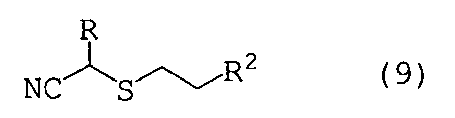
wherein R represents a hydrogen atom or an alkyl group having 1 to 5 carbon atoms, and R² represents a fluorine-containing alkyl group having 1 to 5 carbon atoms, by the process according to claim 13, and then oxidizing the obtained (fluoroalkylthio)acetonitrile represented by the formula (9).

## Patentansprüche

1. Verfahren zur Herstellung eines Fluoralkylthioessigsäureesters der Formel (3): 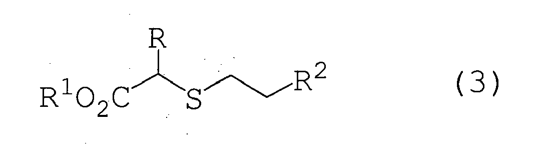
worin R, R¹ und R² die unten genannte Bedeutung haben, welches die Umsetzung eines Thioglycolsäureesters der Formel (1): 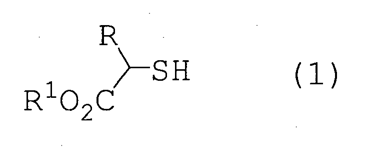
worin R ein Wasserstoffatom oder eine Alkylgruppe mit 1 bis 5 Kohlenstoffatomen bedeutet und R¹ eine Alkylgruppe mit 1 bis 4 Kohlenstoffatomen bedeutet, mit einem Fluorolefin der Formel (2): 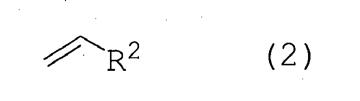
worin R² eine Fluoralkylgruppe mit 1 bis 5 Kohlenstoffatomen bedeutet, in Gegenwart eines Radikalbildners umfasst.

2. Verfahren nach Anspruch 1, wobei der Radikalbildner eine Azoverbindung der Formel (4)ist: 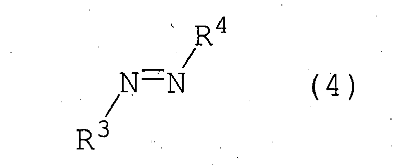
worin R³ und R⁴ jeweils unabhängig eine Alkylgruppe bedeuten, die einen Substituenten tragen kann.

3. Verfahren nach Anspruch 2, wobei die Azoverbindung der Formel (4) 2,2'-Azobis(2,4-dimethyl-4-methoxyvaleronitril) ist.

4. Verfahren nach einem der Ansprüche 1 bis 3, wobei R¹ eine Methylgruppe ist.

5. Verfahren nach einem der Ansprüche 1 bis 4, wobei R² eine Trifluormethylgruppe ist.

6. Verfahren nach einem der Ansprüche 1 bis 5, wobei die Umsetzung in Gegenwart von Ethylacetat durchgeführt wird.

7. Verfahren zur Herstellung einer Esterverbindung der Formel (5) : 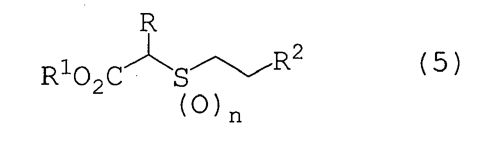
worin R, R¹ und R² die unten genannte Bedeutung haben und n 1 oder 2 bedeutet, welches die Herstellung eines Fluoralkylthioessigsäureesters der Formel (3): 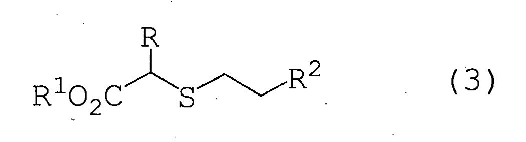
worin R ein Wasserstoffatom oder eine Alkylgruppe mit 1 bis 5 Kohlenstoffatomen bedeutet, R¹ eine Alkylgruppe mit 1 bis 4 Kohlenstoffatomen bedeutet, und R² eine Fluoralkylgruppe mit 1 bis 5 Kohlenstoffatomen bedeutet, durch das Verfahren nach Anspruch 1 und die anschließende Oxidation des erhaltenen Fluoralkylthioessigsäureesters der Formel (3) umfasst.

8. Verfahren zur Herstellung einer Amidverbindung der Formel (6) : 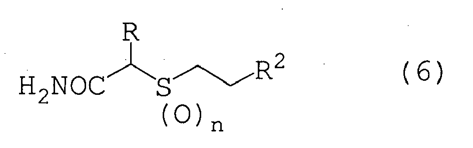
worin R, R² und n die unten genannte Bedeutung haben, welches die Herstellung einer Esterverbindung der Formel (5) : 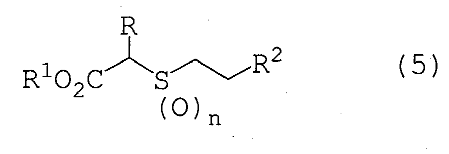
worin R ein Wasserstoffatom oder eine Alkylgruppe mit 1 bis 5 Kohlenstoffatomen bedeutet, R¹ eine Alkylgruppe mit 1 bis 4 Kohlenstoffatomen bedeutet, R² eine Fluoralkylgruppe mit 1 bis 5 Kohlenstoffatomen bedeutet und n 1 oder 2 bedeutet, durch das Verfahren nach Anspruch 7 und die anschließende Umsetzung der erhaltenen Esterverbindung der Formel (5) mit Ammoniak umfasst.

9. Verfahren zur Herstellung einer Nitrilverbindung der Formel (7) : 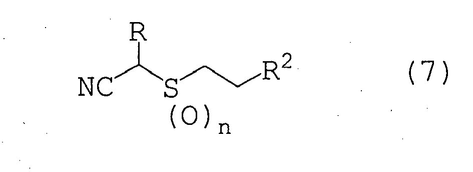
worin R, R² und n die unten genannte Bedeutung haben, welches die Herstellung einer Amidverbindung der Formel (6): 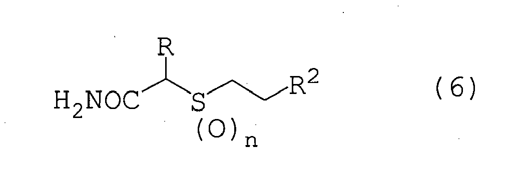
worin R ein Wasserstoffatom oder eine Alkylgruppe mit 1 bis 5 Kohlenstoffatomen bedeutet, R² eine Fluoralkylgruppe mit 1 bis 5 Kohlenstoffatomen bedeutet und n 1 oder 2 bedeutet, durch das Verfahren nach Anspruch 8 und die anschließende Umwandlung der Carbamoylgruppe (-CONH₂) der erhaltenen Amidverbindung der Formel (6) in eine Cyangruppe (-CN) umfasst.

10. Verfahren zur Herstellung eines Fluoralkylthioacetamids der Formel (8): 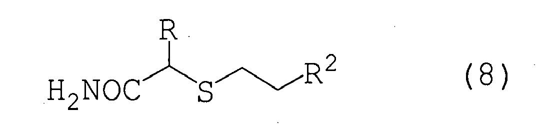
worin R und R² die unten genannte Bedeutung haben, welches die Herstellung eines Fluoralkylthioessigsäueesters der Formel (3): 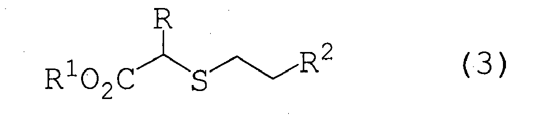
worin R ein Wasserstoffatom oder eine Alkylgruppe mit 1 bis 5 Kohlenstoffatomen bedeutet, R¹ eine Alkylgruppe mit 1 bis 4 Kohlenstoffatomen bedeutet und R² eine Fluor enthaltende Alkylgruppe mit 1 bis 5 Kohlenstoffatomen bedeutet, durch das Verfahren nach Anspruch 1 und die anschließende Umsetzung des erhaltenen Fluoralkylthioessigsäureesters der Formel (3) mit Ammoniak umfasst.

11. Verfahren zur Herstellung einer Amidverbindung der Formel (6): 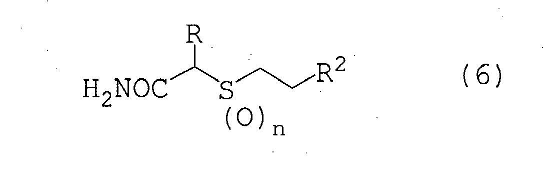
worin R und R² die unten genannte Bedeutung haben und n 1 oder 2 bedeutet, welches die Herstellung eines Fluoralkylthioacetamids der Formel (8): 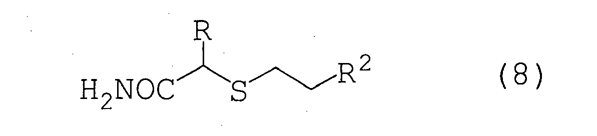
worin R ein Wasserstoffatom oder eine Alkylgruppe mit 1 bis 5 Kohlenstoffatomen bedeutet und R² eine Fluor enthaltende Alkylgruppe mit 1 bis 5 Kohlenstoffatomen bedeutet, durch das Verfahren nach Anspruch 10 und die anschließende Oxidation des erhaltenen Fluoralkylthioacetamids der Formel (8) umfasst.

12. Verfahren zur Herstellung einer Nitrilverbindung der Formel (7) 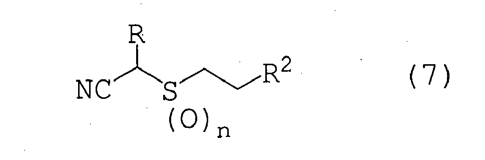
worin R, R² und n die unten genannte Bedeutung haben, welches die Herstellung einer Amidverbindung der Formel (6): 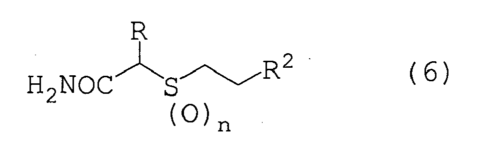
worin R ein Wasserstoffatom oder eine Alkylgruppe mit 1 bis 5 Kohlenstoffatomen bedeutet, R² eine Fluor enthaltende Alkylgruppe mit 1 bis 5 Kohlenstoffatomen bedeutet und n 1 oder 2 bedeutet, durch das Verfahren nach Anspruch 11 und die anschließende Umwandlung der Carbamoylgruppe (-CONH₂) der erhaltenen Amidverbindung der Formel (6) in eine Cyangruppe (-CN) umfasst.

13. Verfahren zur Herstellung eines Fluoralkylthioacetonitrils der Formel (9): 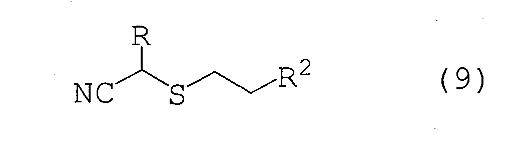
worin R und R² die unten genannte Bedeutung haben, welches die Herstellung eines Fluoralkylthioacetamids der Formel (8) : 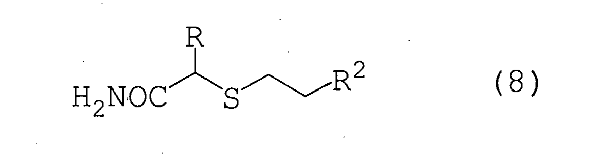
worin R ein Wasserstoffatom oder eine Alkylgruppe mit 1 bis 5 Kohlenstoffatomen bedeutet und R² eine Fluor enthaltende Alkylgruppe mit 1 bis 5 Kohlenstoffatomen bedeutet, durch das Verfahren nach Anspruch 10 und die anschließende Umwandlung der Carbamoylgruppe (-CONH₂) des erhaltenen Fluoralkylthioacetamids der Formel (8) in eine Cyangruppe (-CN) umfasst.

14. Verfahren zur Herstellung einer Nitrilverbindung der Formel (7) : 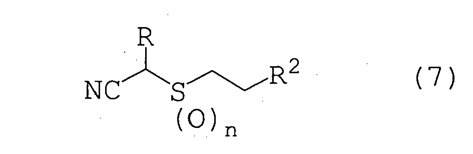
worin R und R² die unten genannte Bedeutung haben und n 1 oder 2 bedeutet, welches die Herstellung eines Fluoralkylthioacetonitrils der Formel (9): 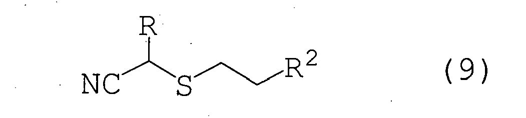
worin R ein Wasserstoffatom oder eine Alkylgruppe mit 1 bis 5 Kohlenstoffatomen bedeutet und R² eine Fluor enthaltende Alkylgruppe mit 1 bis 5 Kohlenstoffatomen bedeutet, durch das Verfahren nach Anspruch 13 und die anschließende Oxidation des erhaltenen Fluoralkylthioacetonitrils der Formel (9) umfasst.

## Revendications

1. Procédé pour produire un ester d'acide (fluoroalkylthio)-acétique représenté par la formule (3) : 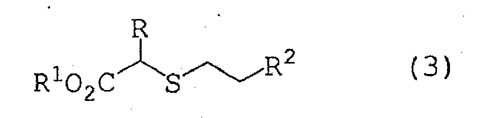
où R, R¹ et R² ont les mêmes significations que celles définies ci-dessous, qui comprend la réaction d'un ester d'acide thioglycolique représenté par la formule (1) : 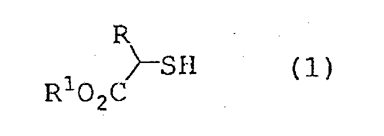
où R représente un atome d'hydrogène ou un groupe alkyle ayant 1 à 5 atomes de carbone, et R¹ représente un groupe alkyle ayant 1 à 4 atomes de carbone, avec une fluorooléfine représentée par la formule (2) : 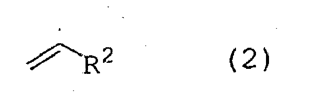
où R² représente un groupe fluoroalkyle ayant 1 à 5 atomes de carbone, en présence d'un générateur de radicaux libres.

2. Procédé selon la revendication 1, où le générateur de radicaux libres est un composé azoïque représenté par la formule (4) : 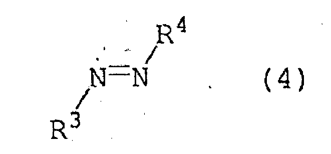
où R³ et R⁴ représentent chacun indépendamment un groupe alkyle capable d'avoir un substituant.

3. Procédé selon la revendication 2, où le composé azoïque représenté par la formule (4) est le 2,2'-azobis(2,4-diméthyl-4-méthoxy-valéronitrile).

4. Procédé selon l'une quelconque des revendications 1 à 3, où R¹ est un groupe méthyle.

5. Procédé selon l'une quelconque des revendications 1 à 4, où R² est un groupe trifluorométhyle.

6. Procédé selon l'une quelconque des revendications 1 à 5, où la réaction est conduite en présence d'acétate d'éthyle.

7. Procédé pour produire un composé ester représenté par la formule (5) : 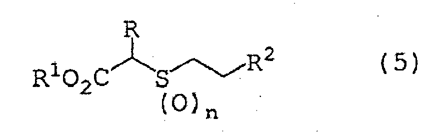
où R, R¹ et R² ont les mêmes significations que celles définies ci-dessous, et n représente 1 ou 2, qui comprend l'obtention d'un ester d'acide (fluoroalkylthio)acétique représenté par la formule (3) : 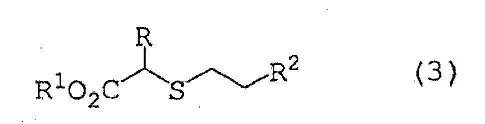
où R représente un atome d'hydrogène ou un groupe alkyle ayant 1 à 5 atomes de carbone, R¹ représente un groupe alkyle ayant 1 à 4 atomes de carbone et R² représente un groupe fluoroalkyle ayant 1 à 5 atomes de carbone, par le procédé selon la revendication 1, puis l'oxydation de l'ester d'acide (fluoroalkylthio)acétique représenté par la formule (3) obtenu.

8. Procédé pour produire un composé amide représenté par la formule (6) : 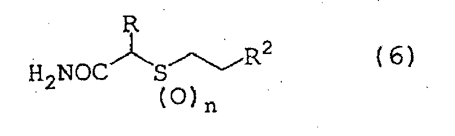
où R, R² et n ont les mêmes significations que celles définies ci-dessous, qui comprend l'obtention d'un composé ester représenté par la formule (5) : 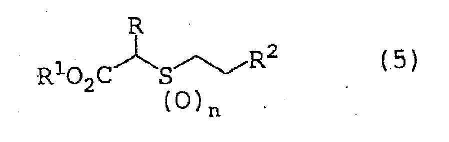
où R représente un atome d'hydrogène ou un groupe alkyle ayant 1 à 5 atomes de carbone, R¹ représente un groupe alkyle ayant 1 à 4 atomes de carbone, R² représente un groupe fluoroalkyle ayant 1 à 5 atomes de carbone et n représente 1 ou 2, par le procédé selon la revendication 7, puis la réaction du composé ester représenté par la formule (5) obtenu avec l'ammoniac.

9. Procédé pour produire un composé nitrile représenté par la formule (7) : 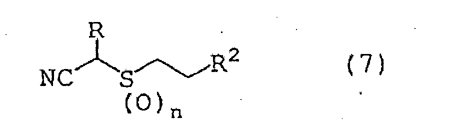
où R, R² et n ont les mêmes significations que celles définies ci-dessous, qui comprend l'obtention d'un composé amide représenté par la formule (6) : 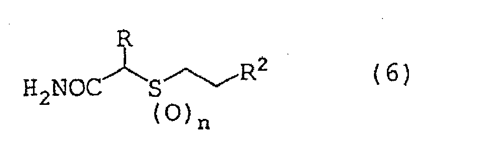
où R représente un atome d'hydrogène ou un groupe alkyle ayant 1 à 5 atomes de carbone, R² représente un groupe fluoroalkyle ayant 1 à 5 atomes de carbone et n représente 1 ou 2, par le procédé selon la revendication 8, puis la conversion d'un groupe carbamoyle (-CONH₂) du composé amide représenté par la formule (6) obtenu en un groupe cyano (-CN).

10. Procédé pour produire un (fluoroalkylthio)acétamide représenté par la formule (8) : 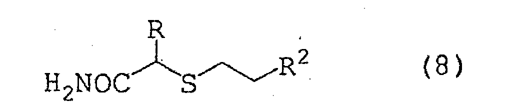
où R et R² ont les mêmes significations que celles définies ci-dessous, qui comprend l'obtention d'un ester d'acide (fluoroalkylthio)acétique représenté par la formule (3) : 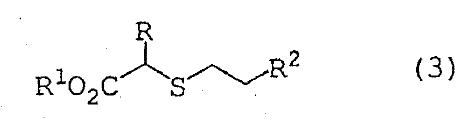
où R représente un atome d'hydrogène ou un groupe alkyle ayant 1 à 5 atomes de carbone, R¹ représente un groupe alkyle ayant 1 à 4 atomes de carbone et R² représente un groupe alkyle contenant du fluor ayant 1 à 5 atomes de carbone, par le procédé selon la revendication 1, puis la réaction de l'ester d'acide (fluoroalkylthio)acétique représenté par la formule (3) obtenu avec l'ammoniac.

11. Procédé pour produire un composé amide représenté par la formule (6) : 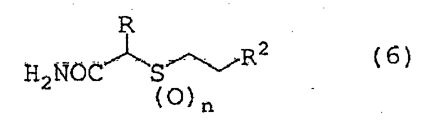
où R et R² ont les mêmes significations que celles définies ci-dessous, et n représente 1 ou 2, qui comprend l'obtention d'un (fluoroalkylthio)acétamide représenté par la formule (8) : 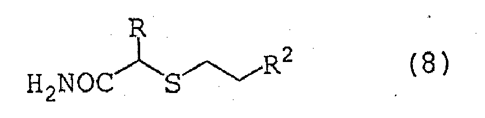
où R représente un atome d'hydrogène ou un groupe alkyle ayant 1 à 5 atomes de carbone et R² représente un groupe alkyle contenant du fluor ayant 1 à 5 atomes de carbone, par le procédé selon la revendication 10, puis l'oxydation du (fluoroalkylthio)acétamide représenté par la formule (8) obtenu.

12. Procédé pour produire un composé nitrile représenté par la formule (7) 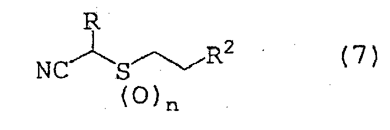
où R, R² et n ont les mêmes significations que celles définies ci-dessous, qui comprend l'obtention d'un composé amide représenté par la formule (6) : 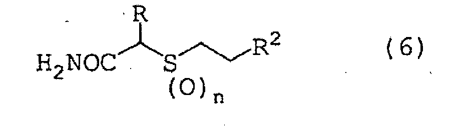
où R représente un atome d'hydrogène ou un groupe alkyle ayant 1 à 5 atomes de carbone, R² représente un groupe alkyle contenant du fluor ayant 1 à 5 atomes de carbone et n représente 1 ou 2, par le procédé selon la revendication 11, puis la conversion d'un groupe carbamoyle (-CONH₂) du composé amide représenté par la formule (6) obtenu en un groupe cyano (-CN).

13. Procédé pour produire un (fluoroalkylthio)acétonitrile représenté par la formule (9) : 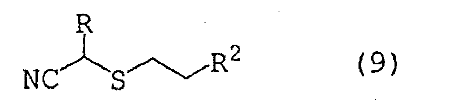
où R et R² ont les mêmes significations que celles définies ci-dessous, qui comprend l'obtention d'un (fluoroalkylthio)acétamide représenté par la formule (8) : 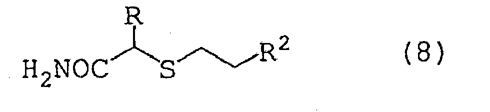
où R représente un atome d'hydrogène ou un groupe alkyle ayant 1 à 5 atomes de carbone et R² représente un groupe alkyle contenant du fluor ayant 1 à 5 atomes de carbone, par le procédé selon la revendication 10, puis la conversion d'un groupe carbamoyle (-CONH₂) du (fluoroalkylthio)-acétamide représenté par la formule (8) obtenu en un groupe cyano (-CN).

14. Procédé pour produire un composé nitrile représenté par la formule (7) : 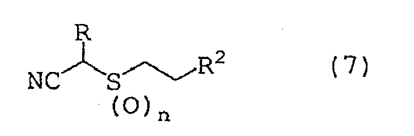
où R et R² ont les mêmes significations que celles définies ci-dessous, et n représente 1 ou 2), qui comprend l'obtention d'un (fluoroalkylthio)acétonitrile représenté par la formule (9) : 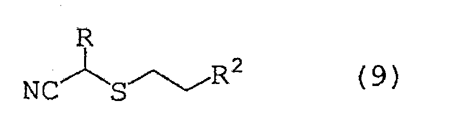
où R représente un atome d'hydrogène ou un groupe alkyle ayant 1 à 5 atomes de carbone et R² représente un groupe alkyle contenant du fluor ayant 1 à 5 atomes de carbone, par le procédé selon la revendication 13, puis l'oxydation du (fluoroalkylthio)acétonitrile représenté par la formule (9) obtenu.
